(19) Europäisches Patentamt
European Patent Office
Office européen des brevets

(11) **EP 4 362 027 A1**

(12) **EUROPEAN PATENT APPLICATION**
published in accordance with Art. 153(4) EPC

(43) Date of publication:
**01.05.2024 Bulletin 2024/18**

(21) Application number: **22828618.3**

(22) Date of filing: **23.05.2022**

(51) International Patent Classification (IPC):
*G16B 15/30* (2019.01)   *G16B 40/00* (2019.01)
*G16B 25/00* (2019.01)   *G16B 45/00* (2019.01)
*G16C 20/30* (2019.01)   *G16C 20/10* (2019.01)
*G16B 35/10* (2019.01)

(52) Cooperative Patent Classification (CPC):
**G16B 15/30; G16B 25/00; G16B 35/10;
G16B 40/00; G16B 45/00; G16C 20/10;
G16C 20/30**

(86) International application number:
**PCT/KR2022/007277**

(87) International publication number:
**WO 2022/270770 (29.12.2022 Gazette 2022/52)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **25.06.2021  KR 20210083446
05.04.2022  KR 20220042378
10.05.2022  KR 20220057185**

(71) Applicant: **Portrai Inc.
Seoul 03136 (KR)**

(72) Inventors:
• **CHOI, Jin Yeong
  Seoul 03129 (KR)**
• **PARK, Jeongbin
  Suwon-si Gyeonggi-do 16229 (KR)**

(74) Representative: **Cabinet Beau de Loménie
158, rue de l'Université
75340 Paris Cedex 07 (FR)**

(54) **EXPLORATION APPARATUS, SYSTEM, AND COMPUTER PROGRAM FOR EXPLORING MOLECULAR MARKER OR PHYSIOLOGICAL ACTIVITY INFORMATION IN TISSUE RELATING TO DISTRIBUTION OR PHYSIOLOGICAL ACTIVITY OF MATERIAL UNDER EXPLORATION**

(57)    The present invention relates to an analysis apparatus, system, and computer program for analyzing a molecular marker or physiological activity information in a tissue relating to distribution or physiological activity of an exploring material.

The present invention discloses an analysis apparatus (100) including: an information receiving unit (110) configured to receive a tissue image of a tissue of interest provided with an exploring material with which a labeling material for analysis is bonded and transcriptome information sharing spatial information of the tissue of interest, a spatial mapping unit (120) configured to generate spatial mapping information by spatially mapping the tissue image by the labeling material of the exploring material with the spatially reserved transcriptome information, a transcriptome information extraction unit (150) configured to extract the transcriptome information in the tissue relating to distribution of the exploring material from the spatial mapping information, and a molecular marker analysis unit (160) configured to analyze a molecular marker relating to the distribution of the exploring material in the tissue of interest through the extraction.

FIG. 1

**Description**

[Technical Field]

The present invention relates to an analysis apparatus, system, and computer program for analyzing a molecular marker or physiological activity information in a tissue relating to distribution or physiological activity of an exploring material.

[Background Art]

In order to estimate distribution or physiological activity-related materials of drugs such as nanomaterials, low molecule compounds, peptides, cells, antibodies or recombinant antibodies, it is essential in a process of developing new drugs to estimate biological distribution or physiological activity of drugs through blood-based simulation or by labeling drugs with labeling materials such as a fluorescent material and estimate effects and kinetics of the drugs.

However, the existing blood-based or in vivo image-based pharmacokinetics estimation technologies may estimate distribution at the level of entire organs, but may not evaluate nonuniform distribution within organs, or the like. This is because normal organs are composed of very heterogeneous cells that perform different functions. In addition, cancer tissues are also composed of very heterogeneous cells due to genetic heterogeneity between cancer cells that appears during cancer cell division and heterogeneity of microenvironment that appears due to blood vessels, immune cells, and the like present in tissues. Due to the heterogeneity of the tissue, drugs are absorbed very non-uniformly in the tissues.

Screening distribution at a microscopic level and molecules related thereto to predict actions of drugs and predict distribution at a molecular target level may be used as an indicator for predicting the final success of new drug development. However, a technology of interpreting where drugs are distributed in tissues according to which cells or molecular profile has not been yet established. Furthermore, information on the distribution of the drugs in the tissues is also related to the physiological action of the drugs.

The recently developed and used spatially resolved transcriptome is capable of acquiring hundreds to tens of thousands of gene expression information at once, and acquiring the gene expression information while preserving tissue location information.

However, analyzing the molecular marker or the physiological activity information relating to the distribution of the drugs in the tissues by using the spatially resolved transcriptome is still at an insufficient level.

[Related Art Document]

[Non-Patent Document]

(1) Nat Rev Drug Discov. 2016 Dec;15(12):817-818.

(2) Sindhwani, S., Syed, A. M., Ngai, J., Kingston, B. R., Maiorino, L., Rothschild, J., ... & Chan, W. C. (2020). The entry of nanoparticles into solid tumours. Nature materials, 19(5), 566-575.

(3) Chen, F., Ma, K., Madajewski, B., Zhuang, L., Zhang, L., Rickert, K., ... & Bradbury, M. S. (2018). Ultrasmall targeted nanoparticles with engineered antibody fragments for imaging detection of HER2-overexpressing breast cancer. Nature communications, 9(1), 1-11.

(4) Bolkestein, M., de Blois, E., Koelewijn, S. J., Eggermont, A. M., Grosveld, F., de Jong, M., & Koning, G. A. (2016). Investigation of factors determining the enhanced permeability and retention effect in subcutaneous xenografts. Journal of nuclear medicine, 57(4), 601-607.

(5) Fick, A. (1855). "V. On liquid diffusion". Phil. Mag. 10 (63): 30-39. doi: 10.1080/14786445508641925

(6) R.B. Bird, W.E. Stewart, E.N. Lightfoot. (2002). Transport Phenomena 2nd edition. Wiley.

(7) Sindhwani, S., Syed, A. M., Ngai, J., Kingston, B. R., Maiorino, L., Rothschild, J., ... & Chan, W. C. (2020). The entry of nanoparticles into solid tumours. Nature materials, 19(5), 566-575.

(8) Bae, S., Choi, H., & Lee, D. S. (2021). Discovery of molecular features underlying the morphological landscape by integrating spatial transcriptomic data with deep features of tissue images. Nucleic acids research, 49(10), e55-e55.

(9) Sebastian, A., Hum, N. R., Martin, K. A., Gilmore, S. F., Peran, I., Byers, S. W., ... & Loots, G. G. (2020). Single-cell Transcriptomic analysis of tumor-derived fibroblasts and Normal tissue-resident fibroblasts reveals fibroblast heterogeneity in breast Cancer. Cancers, 12(5), 1307.

(10) Cable, D. M., Murray, E., Zou, L. S., Goeva, A., Macosko, E. Z., Chen, F., & Irizarry, R. A. (2021). Robust decomposition of cell type mixtures in spatial transcriptomics. Nature Biotechnology, 1-10.

(11) https://www.biorxiv.org/content/10.1101/2021.04.26.441459v1

(12) Zhou, Y., & Luo, G. (2020). Apolipoproteins, as the carrier proteins for lipids, are involved in the development of breast cancer. Clinical and Translational Oncology, 1-11.

(13) Alfarouk, K. O. (2016). Tumor metabolism, cancer cell transporters, and microenvironmental resistance. Journal of enzyme inhibition and medicinal chemistry, 31(6), 859-866.

(14) Wang, Z., Bovik, A. C., Sheikh, H. R., & Simoncelli, E. P. (2004). Image quality assessment: from error visibility to structural similarity. IEEE transactions on image processing, 13(4), 600-612.

(15) Brownlee, W. J., & Seib, F. P. (2018). Impact of the hypoxic phenotype on the uptake and efflux of nanoparticles by human breast cancer cells. Scientific reports, 8(1), 1-11.

(16) Zhang, I., Cui, Y., Amiri, A., Ding, Y., Campbell, R. E., & Maysinger, D. (2016). Pharmacological inhibition of lipid droplet formation enhances the effectiveness of curcumin in glioblastoma. European Journal of Pharmaceutics and Biopharmaceutics, 100, 66-76.

(17) Fujimoto, T., & Parton, R. G. (2011). Not just fat: the structure and function of the lipid droplet. Cold Spring Harbor perspectives in biology, 3(3), a004838.

(18) Cruz, A. L., Barreto, E. D. A., Fazolini, N. P., Viola, J. P., & Bozza, P. T. (2020). Lipid droplets: platforms with multiple functions in cancer hallmarks. Cell death & disease, 11(2), 1-16.

(19) Li, R., Ng, T. S., Wang, S. J., Prytyskach, M., Rodell, C. B., Mikula, H., .. & Miller, M. A. (2021). Therapeutically reprogrammed nutrient signalling enhances nanoparticulate albumin bound drug uptake and efficacy in KRAS-mutant cancer. Nature Nanotechnology, 1-10.

(20) Yokoi K, Kojic M, Milosevic M, Tanei T, Ferrari M, Ziemys A. Capillary-Wall Collagen as a Biophysical Marker of Nanotherapeutic Permeability into the Tumor Microenvironment. 2014, 74(16): 4239-4246.

(21) Moncada R, Barkley D, Wagner F, Chiodin M, Devlin JC, Baron M, et al. Integrating microarray-based spatial transcriptomics and single-cell RNA-seq reveals tissue architecture in pancreatic ductal adenocarcinomas. Nat Biotechnol 2020, 38(3): 333-342.

(22) Bae S, Na KJ, Koh J, Lee DS, Choi H, Kim YT. CellDART: Cell type inference by domain adaptation of single-cell and spatial transcriptomic data. bioRxiv 2021: 2021.2004.2026.441459.

(23) Castellano J, Aledo R, Sendra J, Costales P, Juan-Babot O, Badimon L, et al. Hypoxia stimulates low-density lipoprotein receptor-related protein-1 expression through hypoxia-inducible factor-1$\alpha$ in human vascular smooth muscle cells. Arteriosclerosis, thrombosis, and vascular biology 2011, 31(6): 1411-1420.

(24) Perman JC, Bostrom P, Lindbom M, Lidberg U, StAhlman M, Hagg D, et al. The VLDL receptor promotes lipotoxicity and increases mortality in mice following an acute myocardial infarction. The Journal of clinical investigation 2011, 121(7): 2625-2640

[Disclosure]

[Technical Problem]

The present invention is intended to solve the above-described problems, and is to analyze a molecular marker or physiological activity information in tissues relating to distribution or physiological activity of an exploring material using transcriptome information sharing spatial information.

In addition, the present invention is to provide information on action mechanism, synergistic or blocking action, and the like of drugs from distribution at a microscopic level or physiological activity information in tissues for drugs, and to rediscover values of drugs or provide a platform for scientific or rational drug development.

[Technical Solution]

An aspect of the present invention provides

an analysis apparatus 100 including an information receiving unit 110 configured to receive a tissue image of a tissue of interest provided with an exploring material with which a labeling material for analysis is bonded, and transcriptome information sharing spatial information of the tissue of interest, a spatial mapping unit 120 configured to generate spatial mapping information by spatially mapping the tissue image by the labeling material of the exploring material with the spatially reserved transcriptome information, a transcriptome information extraction unit 150 configured to extract the transcriptome information in the tissue relating to distribution of the exploring material from the spatial mapping information, and a molecular marker analysis unit 160 configured to analyze a molecular marker relating to the distribution of the exploring material in the tissue of interest through the extraction.

Another aspect of the present invention provides

an analysis apparatus 100 including an information receiving unit 110 configured to receive a tissue image of a tissue of interest provided with a first exploring material(s) with which a first labeling material for analysis is bonded and transcriptome information sharing spatial information of the tissue of interest, a spatial mapping unit 120 configured to generate spatial mapping information by spatially mapping the tissue image by the labeling material of the exploring material with the spatially reserved transcriptome information, and a physiological activity information analysis unit 170 configured to analyze physiological activity information in a tissue of interest relating to distribution or action of the first exploring material.

Another aspect of the present invention provides

an analysis apparatus 100 including an information receiving unit 110 configured to receive a tissue image of a tissue of interest provided with an exploring material with which a labeling material for analysis is bonded, and transcriptome information sharing spatial information of the tissue of interest, a spatial mapping unit 120 configured to generate spatial mapping information by spatially mapping the tissue image by the labeling material of the exploring material with the spatially reserved transcriptome information, and a physiological activity information analysis unit 170 configured to analyze physiological activity information of the exploring material in the tissue of interest.

In this case, the analysis apparatus 100 may include a clustering unit 140 configured to partition the tissue image by the labeling material (or including registered image or transformed image thereof) into one or more clusters before or after the spatial mapping.

The spatial mapping information may include spatially mapped information of one or more clusters.

Another aspect of the present invention provides a method of analyzing a molecular marker or physiological activity information in a tissue relating to distribution or physiological activity of an exploring material in the tissue, which may be performed in the analysis apparatus 100.

Another aspect of the present invention provides a system including an analysis apparatus 100 for analyzing a molecular marker or physiological activity information in a tissue relating to distribution or physiological activity of an exploring material in the tissue.

Another aspect of the present invention provides a computer program stored in a computer-readable recording medium for executing a method of analyzing a molecular marker or physiological activity information in a tissue relating to distribution or physiological activity of an exploring material in the tissue.

[Advantageous Effects]

The present invention can provide distribution or physiological activity information in tissues that cannot be found out from existing blood-based or in vivo image-based drug organ distribution or action analysis method.

The present invention can be used for various stages of research and development based on spatially resolved transcriptome analysis methods and analysis algorithms previously available, and can be effectively used for biomarker discovery, new drug development, etc.

The present invention can be used to identify a molecular marker in tissues that inhibits or enhances drug targets, or to optimize targets of developed or known drugs.

The present invention can provide a molecular marker that may be responsible for nonuniform distribution of drugs.

The present invention can be used in a process of developing new drugs, in analyzing existing drug mechanisms to predict or improve effects, or in analyzing effective targets.

[Brief Description of the Drawings]

FIG. 1 is a diagram illustrating an analysis system according to an aspect of the present invention.
FIG. 2 is a block diagram illustrating components that constitute an analysis apparatus of the analysis system of FIG. 1.

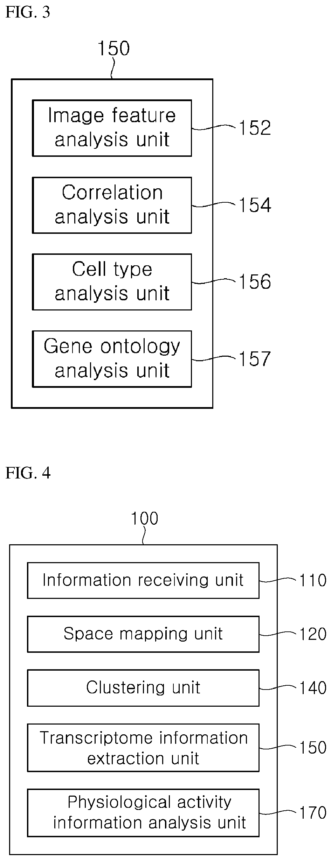

FIG. 3 is a block diagram illustrating components that constitute transcriptome information extraction unit of the analysis apparatus of FIG. 2.
FIG. 4 is a block diagram illustrating components constituting an analysis apparatus of an analysis system according to another aspect of the present invention.
FIG. 5 is a flowchart of a method of analyzing a molecular marker performed in an analysis apparatus according to an aspect of the present invention.
FIG. 6 is a flowchart illustrating a specific example of a method of acquiring a tissue image by a labeling material received in step S10 in an aspect of the present invention of FIG. 5.
FIG. 7 illustrates a method of acquiring transcriptome information sharing spatial information of a tissue of interest received in step S20 in an aspect of the present invention of FIG. 5.
FIG. 8 is a diagram illustrating a specific example of a transcriptome information extraction step (S40) in a tissue in an aspect of the present invention of FIG. 5, in which well-known analysis algorithms or analysis methods such as SPADE, DEG, CellDART, MIA, RCTD, and gene ontology analysis may be used.

FIG. 9 illustrates a specific example of a molecular marker analysis step (S50) in an aspect of the present invention of FIG. 5.

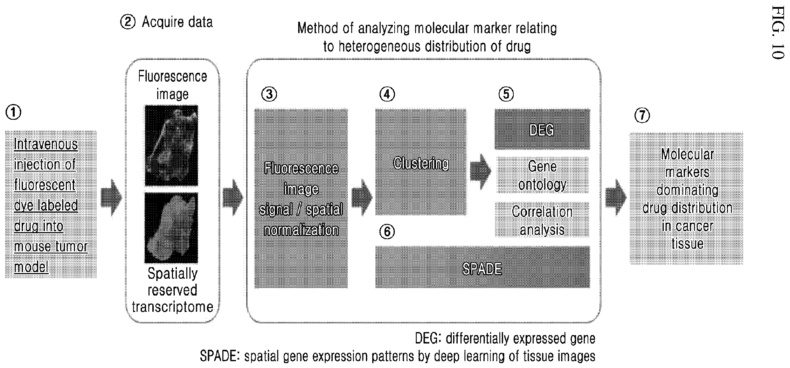

FIG. 10 is a flowchart of a method of analyzing a molecular marker performed in an analysis apparatus according to an embodiment of the present invention.

FIG. 11 is a diagram for describing step by step specific methods and analysis methods that may be applied at each step in the analysis method performed in the analysis apparatus according to the aspects of the present invention.

FIG. 12 illustrates a method of splitting a tissue image into several patches using the SPADE algorithm and extracting principal components (PCs) from these patches by visual geometry group (VGG16) that is an image recognition program using a deep learning-based augmented neural network.

FIG. 13 is a plot of spatial characteristics of a high uptake spot according to a degree of agglomeration, in which the degree of agglomeration is represented as a Euclidean distance, and neighboring spots from any high uptake spot are assigned as the high uptake spot.

FIGS. 14A to 14K illustrate program implementation examples of spatial gene expression pattern analysis using the SPADE algorithm for the tissue image.

FIGS. 15A and 15B illustrate a program implementation example of K-means clustering as clustering means.

FIGS. 16A to 16D illustrate results of a cell type cluster obtained from an RCTD algorithm for clustering 1, in which FIG. 16A is a pie plot illustrating cell type occurrence, FIG. 16B is an RCTD dual distribution stacked bar plot illustrating results for all spots, FIG. 16C is an RCTD double line stacked bar plot for spots in cluster 1, and FIG. 16D is an RCTD double distribution stacked bar plot for spots in cluster 2.

FIG. 17 illustrates an example of setting parameters of the RCTD algorithm.

FIGS. 18A to 18E illustrate a program implementation example required to perform allelic domain adaptive classification with 225 selected feature genes from single cell data by the CellDART algorithm.

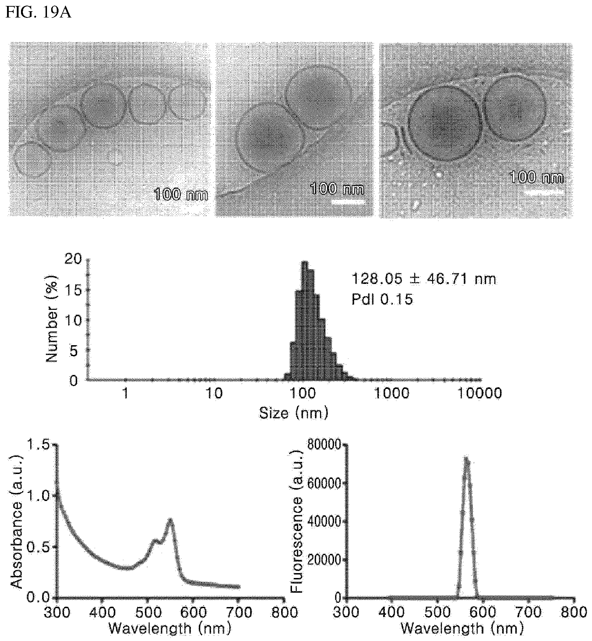

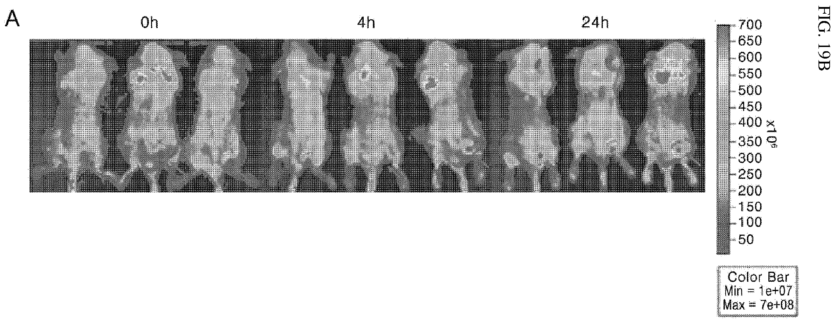

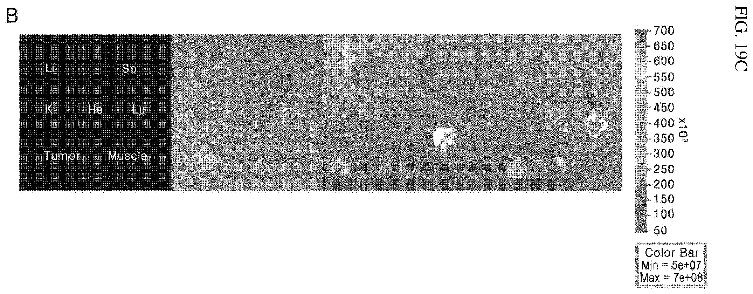

FIG. 19A is a transmission electron micrograph (TEM) (top), a result of a dynamic light scattering (DLS) (middle), and a diagram of a characteristic profile of a microplate reader (bottom) of DiI-loaded liposomes according to an implementation example of the present invention. FIG. 19B is an in vivo fluorescence image after 0 hours, 4 hours, and 24 hours of intravenous injection of DiI-loaded liposomes into a tumor model of a rat, and illustrates that the liposomes were accumulated in each organ. FIG. 19C is an ex vivo fluorescence image of major organs (liver, spleen, kidney, heart, lung, tumor, and muscle) and tumor after 24 hours of the liposome injection.

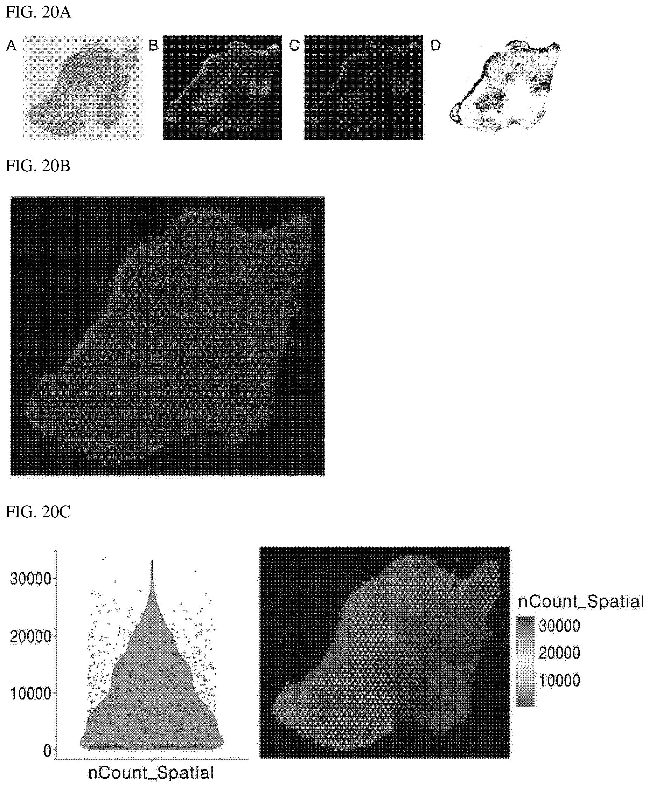

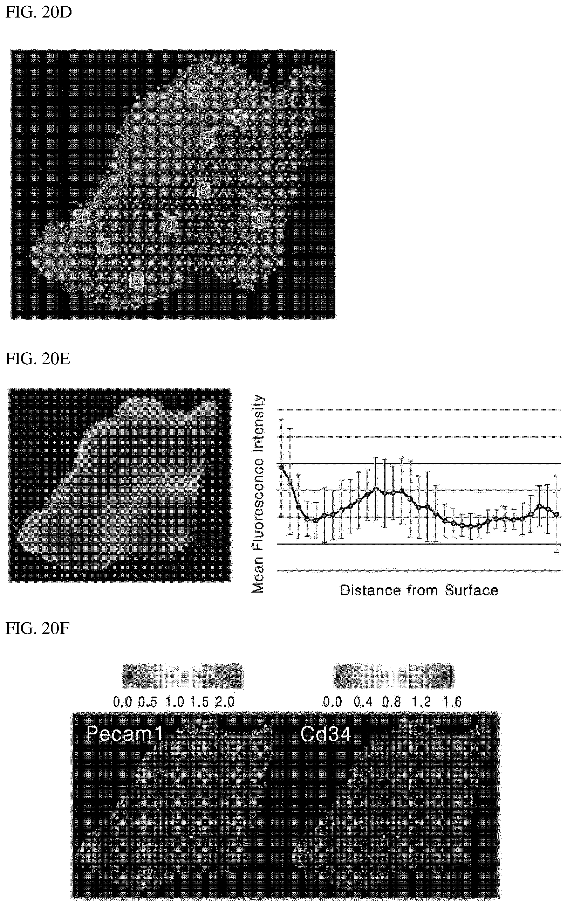

A in FIG. 20A illustrates an H&E staining image showing overall histological features, and B, C, and D in FIG. 20A and FIG. 20B illustrate various steps performed when processing a fluorescence image into a binary map. Specifically, in FIG. 20A, B illustrates fluorescence image normalization, C illustrates a registered image, D illustrates a binary translation image, respectively, and FIG. 20B illustrates a binary map obtained corresponding to a binary translation image. FIG. 20C illustrates the number of RNA transcriptomes plotted by violin plot (left) and spatial mapping (right). FIG. 20D illustrates t-SNE projection of spots according to genetic features as a result of investigating clustering according to a gene expression pattern. FIG. 20E illustrates average fluorescent intensity according to a distance, which is a map colored according to a indicated distance (left) and a graph showing average fluorescent intensity according to a distance from an edge surface on the left (right). FIG. 20F is a spatial feature plot of Pecam1 and Cd34 in overall shape analysis.

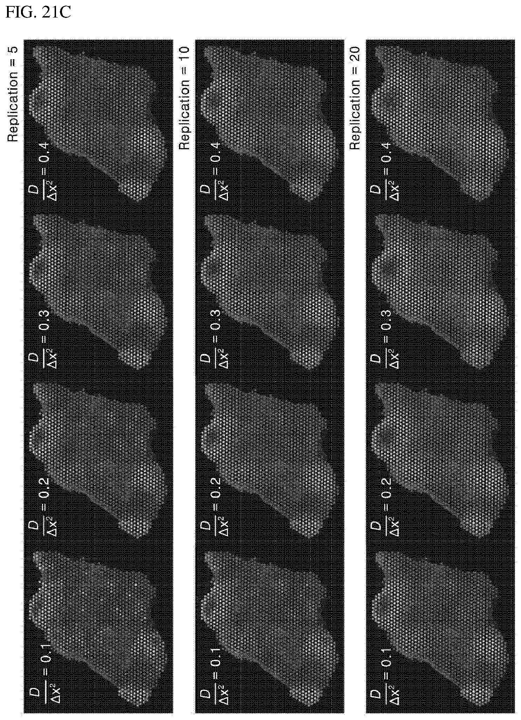

FIG. 21 illustrates, as numerical analysis results according to Fick's law, simulation results of Fick dispersion rep-

$$\frac{D}{\Delta x^2}$$

resented by C/k according to different $\frac{D}{\Delta x^2}$ and repetition numbers.

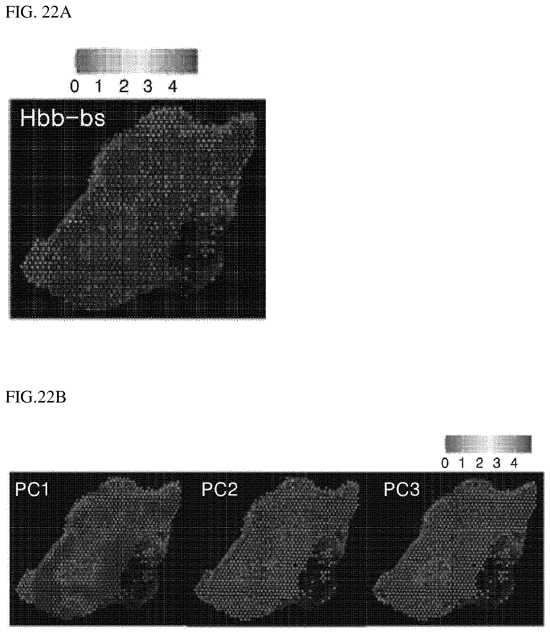

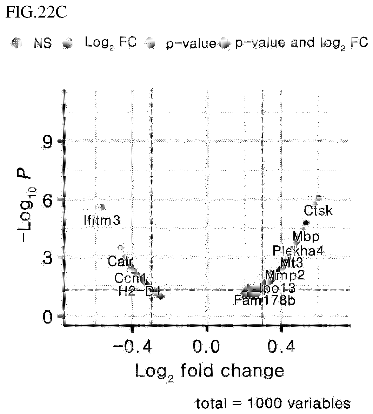

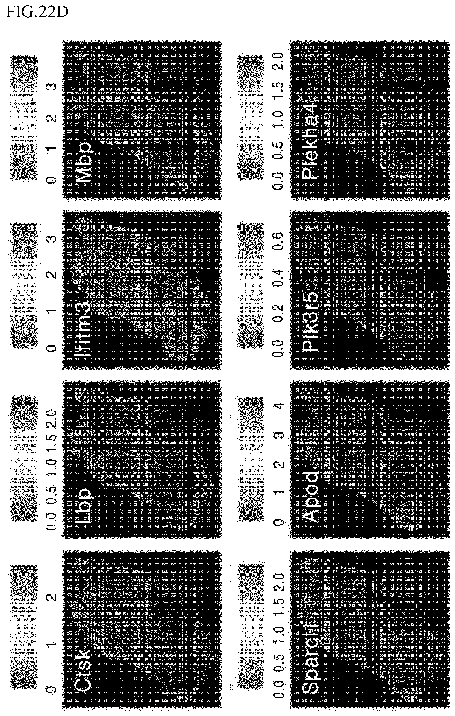

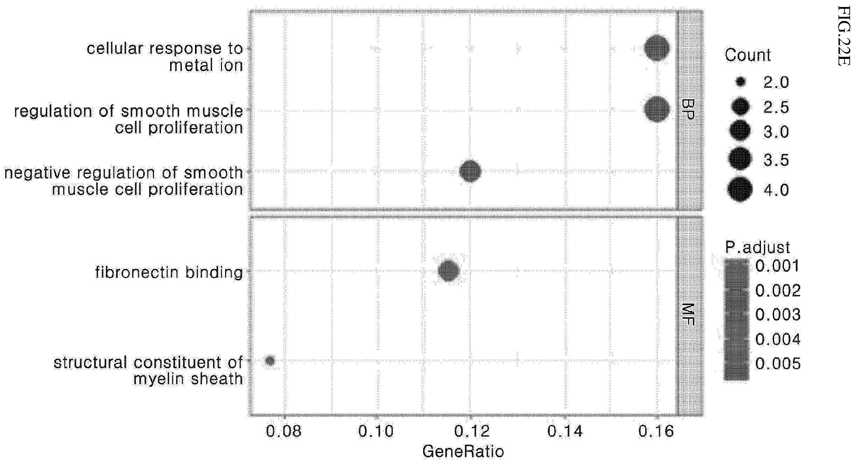

FIGS. 22A to 22E illustrate the total fluorescence analysis results. FIG. 22A is a spatial feature plot of Hbb-bs which is a unique DEG, and FIG. 22B illustrates image latent features PC1, PC2, and PC3 generated by the SPADE algorithm and the PCA algorithm, each of which means principal component 1, principal component 2, and principal component 3. FIG. 22C is an improved volcano plot of the top 1000 variable genes. FIG. 22D is a spatial feature plot of the top 8 SPADE genes having the highest fold change (FC). FIG. 22E illustrates gene ontology (GO) analysis results of SPADE gene in PC1 of the top 30 up-regulated genes according to a biological process (BP), a cellular component (CC), and a molecular function (MF), respectively.

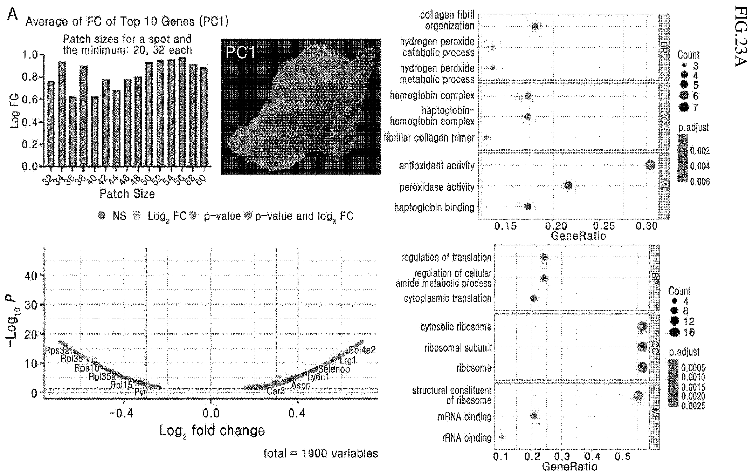

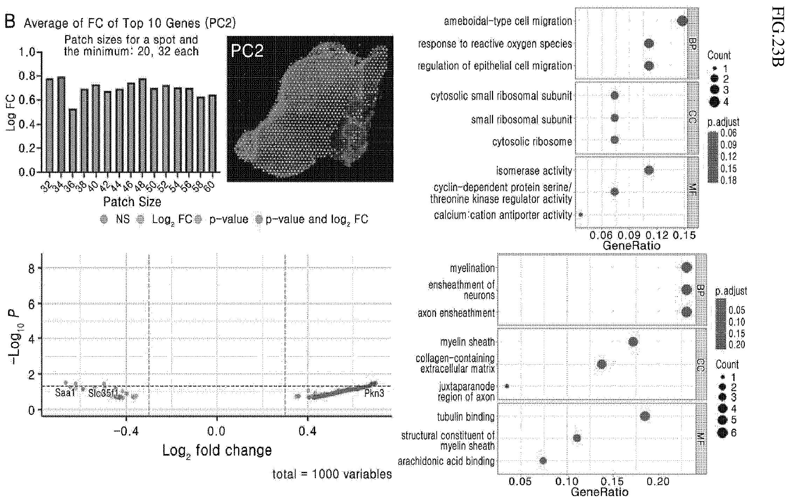

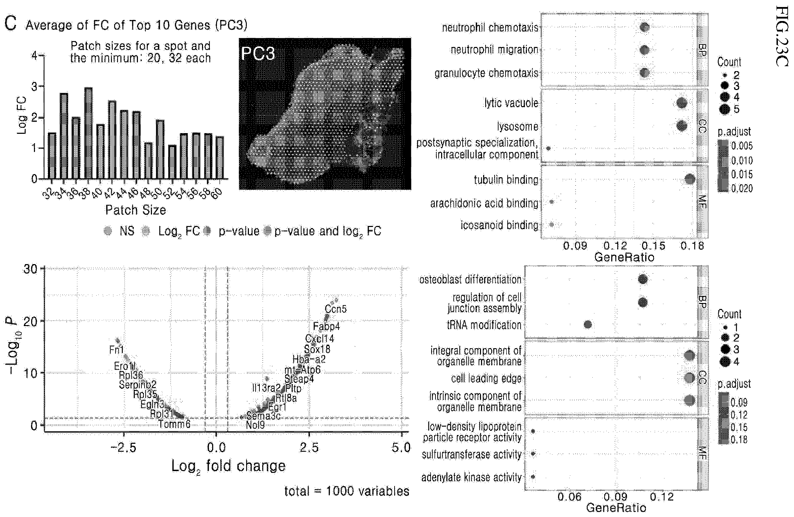

FIG. 23 illustrates the results of the SPADE algorithm from the H&E staining image, and FIGS. 23A to 23C each illustrate improved volcano plots of the top SPADE genes, each GO analysis result, and the top 1000 variable genes in each principal component of the SPADE algorithm, that is, PC1 (FIG. 23A), PC2 (FIG. 23B), and PC3 (FIG. 23C). Here, patch sizes of each PC are determined independently of each other to have the largest average log FC value of the top 10 genes.

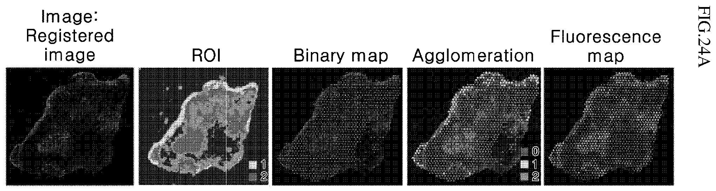

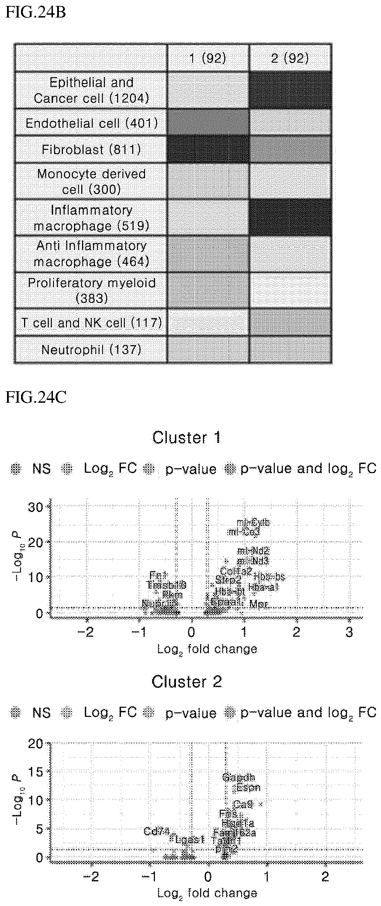

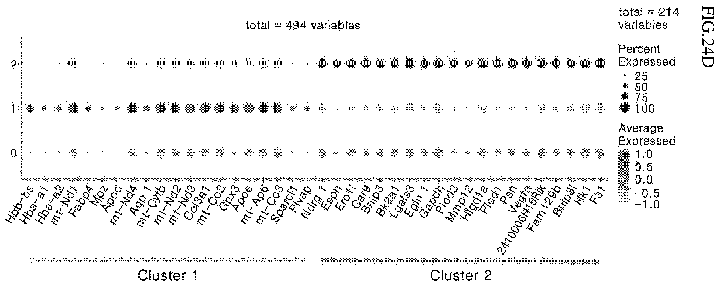

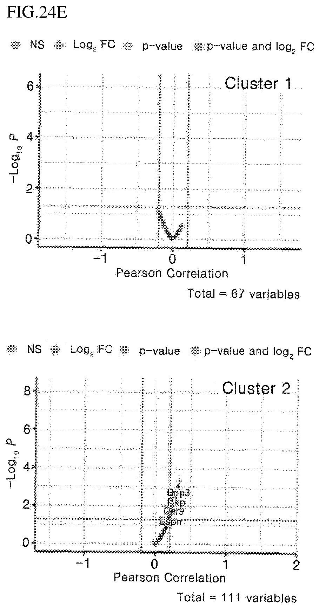

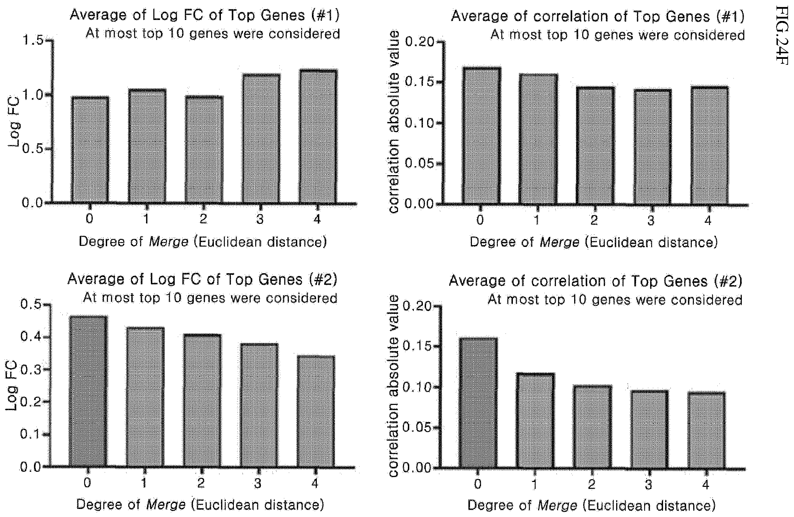

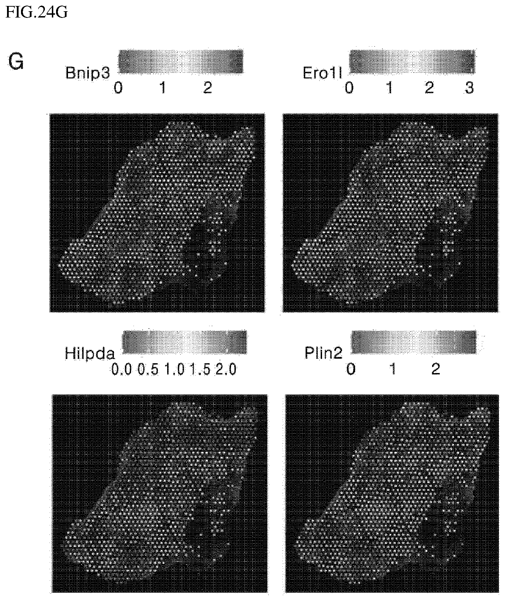

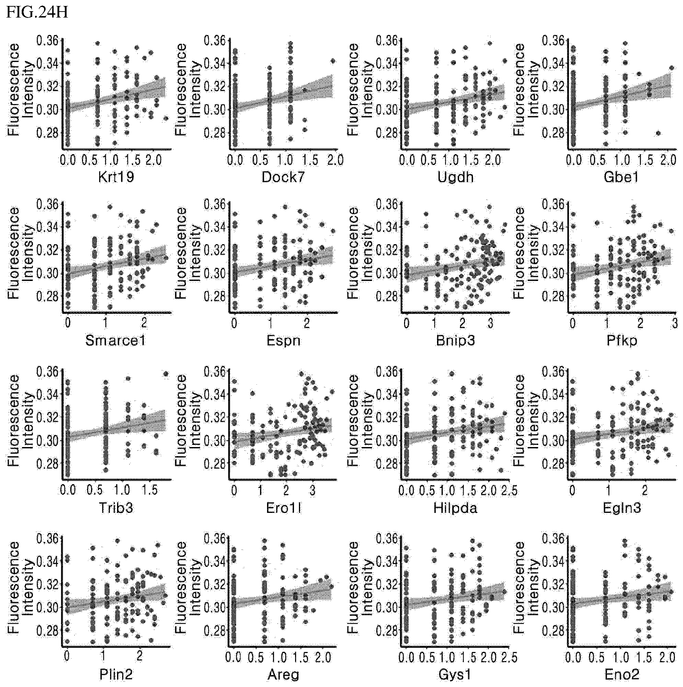

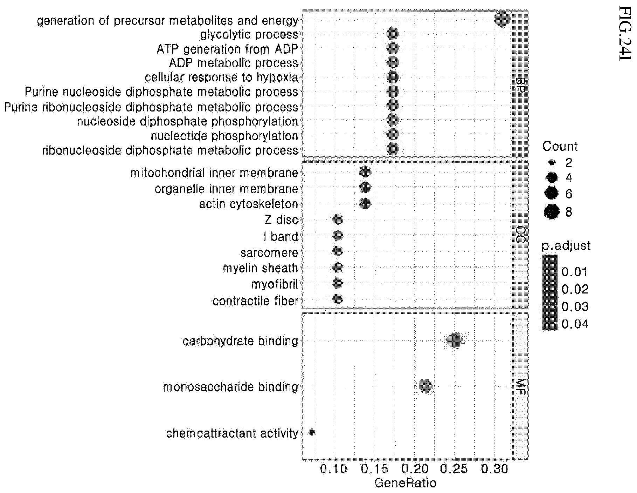

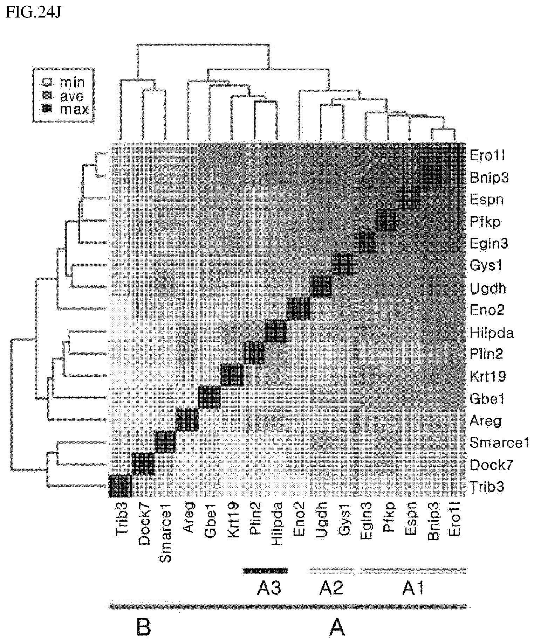

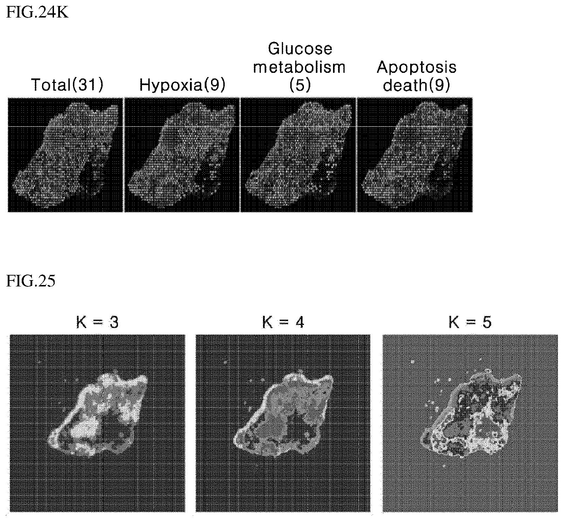

FIG. 24 illustrates fluorescence analysis results of a subgroup, and FIG. 24A illustrates a series of processes of setting a region of interest (ROI) starting from the registered fluorescence image and agglomerating the set ROI with a binary map written from the total fluorescence analysis to obtain a fluorescence map. FIG. 24B illustrates MIA analysis results of clusters 1 and 2, and FIG. 24C illustrates a volcano plot of uptake site-specific genes in clusters 1 (left) and 2 (right). FIG. 24D is a dot plot illustrating the expression of the top 20 DEGs in clusters 1 and 2, and FIG. 24E is a volcano plot illustrating the relationship between a correlation coefficient and a p-value in clusters 1 (top) and 2 (bottom). FIG. 24F illustrates the determination of the degree of agglomeration, FIG. 24G is a feature plot of four exemplary DEGs significantly correlated with cluster 2, that is, Bnip3, Ero1l, Hilpda, and Plin2, and FIG. 24H is a scatter plot of all DEGs having significant correlation in cluster 2. FIG. 24I illustrates GO analysis results according to BP, CC, and MF for DEG having significant correlation in cluster 2, and FIG. 24J illustrates a heatmap derived from correlations between each pair of DEGs having significant correlations with cluster 2. FIG. 24K is a spatial feature plot of scores derived from total genes, hypoxia genes, glucose metabolism genes, and apoptotic death genes, in which the scores were generated with AddModuleScore of the Seurat package.

FIG. 25 illustrates results of KNN average clustering according to parameter K (K=3, 4, or 5), in which all other parameters are set to default in a supplementary code. It may be observed that this algorithm operates in a brightness-driven manner. This algorithm recognized patterns in the image and began to distinguish between a surface and an inside of a tumor only when K = 4.

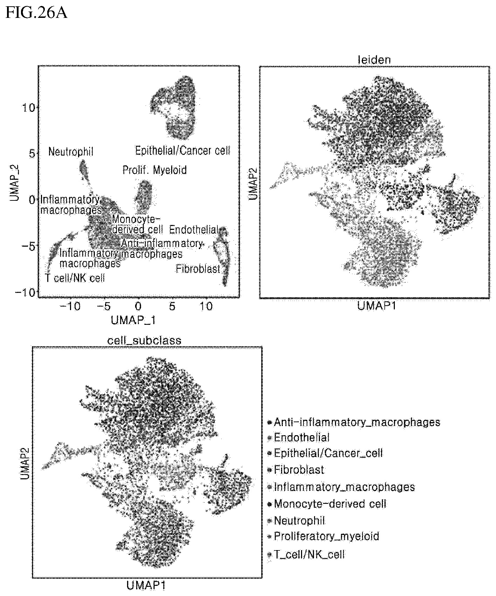

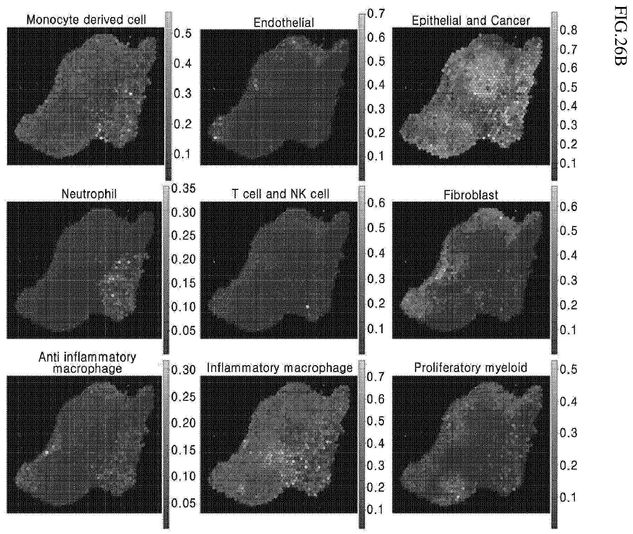

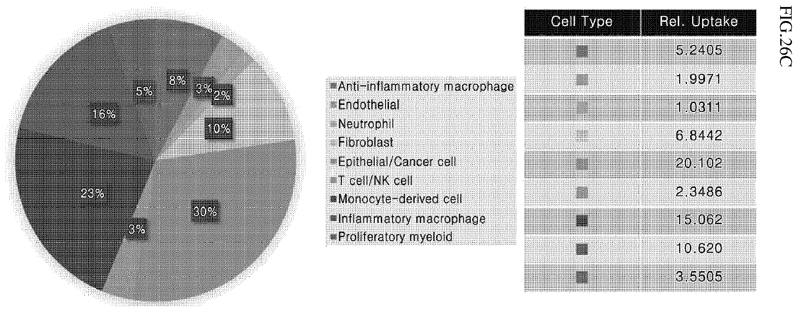

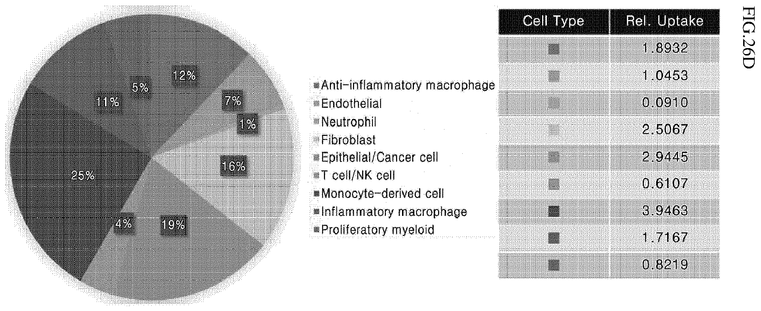

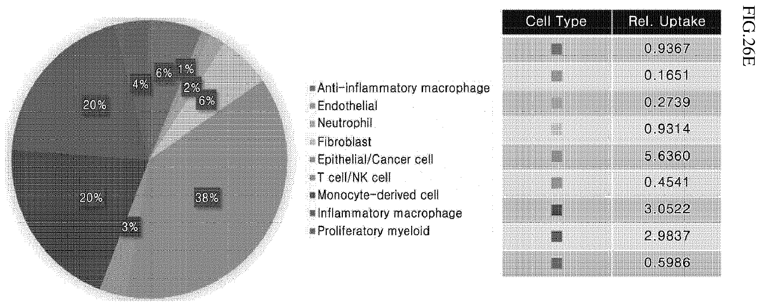

FIG. 26 is a result of the CellDART algorithm. FIG. 26A is a UMAP plot displayed by cell type from single cell RNA sequencing data obtained from 4T1 cells of a solid tumor model. A Leiden algorithm was used for test of cell type classification. FIG. 26B is a spatial feature plot illustrating distribution profiles in tissues for each cell type. FIGS. 26C, 26D, and 26E each are pie charts and tables showing drug distributions for each cell type derived from the CellDART algorithm in all spots (FIG. 26C), cluster 1 (FIG. 26D), and cluster 2 (FIG. 26E) in order.

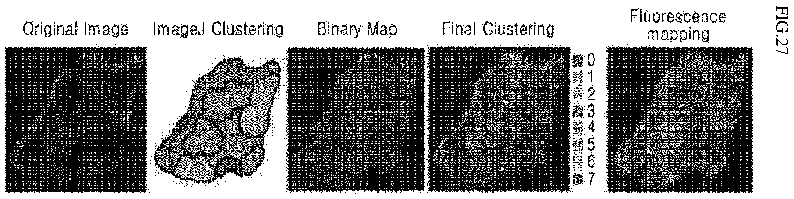

FIG. 27 is a diagram illustrating a series of processes of setting a total of 7 ROIs using an imageJ program, and agglomerating the set ROI with the binary map to be clustered into clusters 0 to 7.

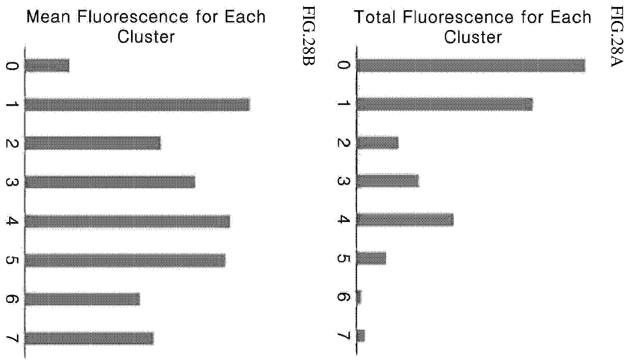

FIG. 28A is a bar graph showing total fluorescent intensity of each cluster in clustering 2, and FIG. 28B is a bar graph showing average fluorescent intensity of each cluster.

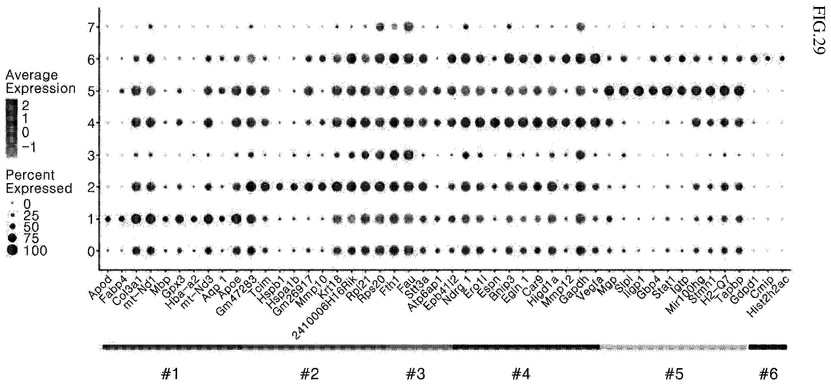

FIG. 29 is a diagram illustrating an expression level of the top 10 DEGs for each cluster through a dot plot in clustering 2.

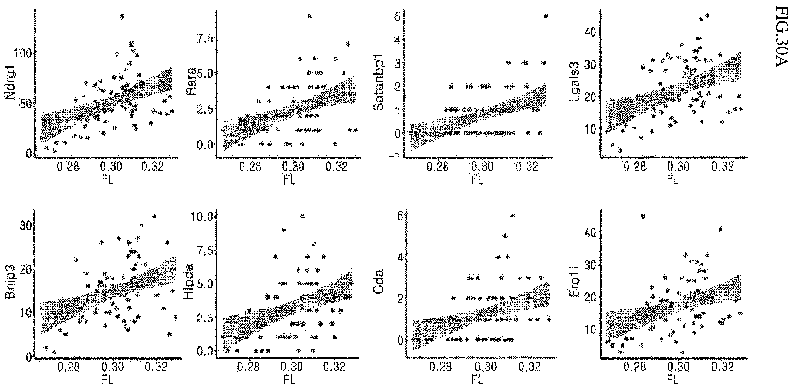

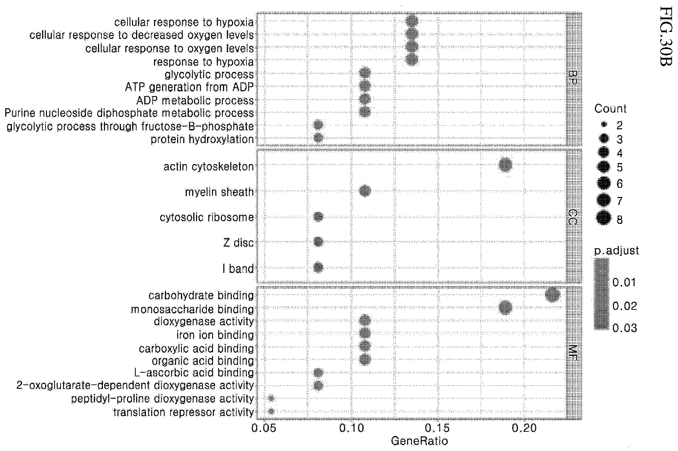

FIG. 30A is a scatter plot of the top 8 DEGs having correlation in cluster 4 of clustering 2, and FIG. 30B is a diagram illustrating GO analysis results of correlated DEGs according to BP, CC, and MF in cluster 4 of clustering 2.

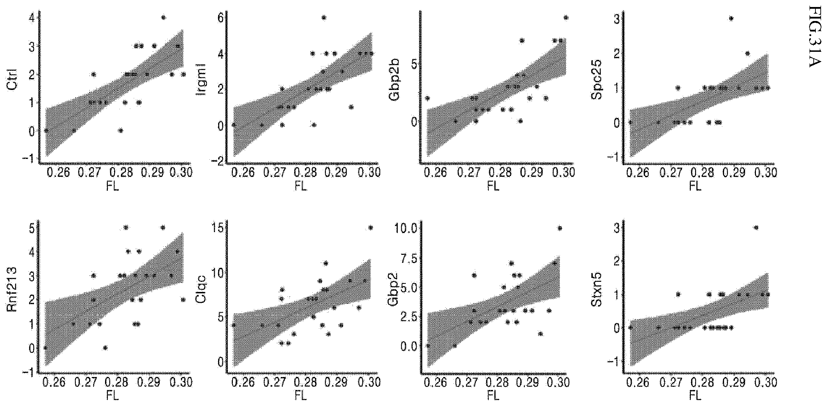

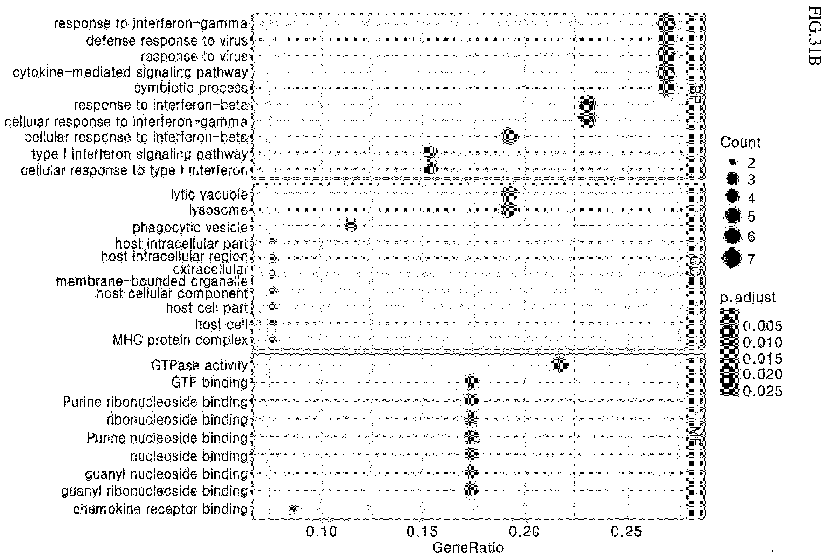

FIG. 31A is a scatter plot of the top 8 DEGs having correlation in cluster 5 of clustering 2, and FIG. 31B is a diagram illustrating GO analysis results of correlated DEGs according to BP, CC, and MF in cluster 5 of clustering 2.

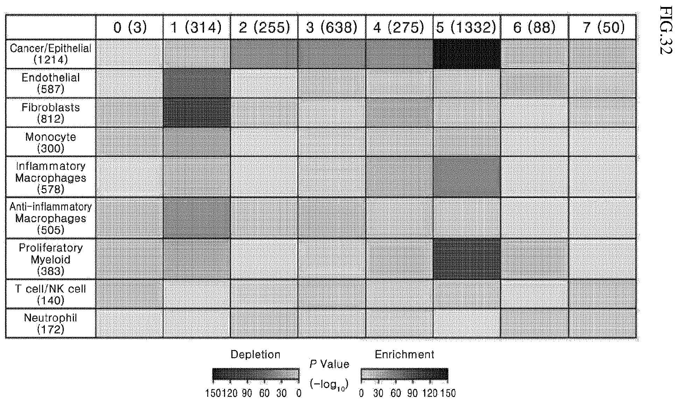

FIG. 32 illustrates MIA analysis results for each cluster in clustering 2.

[Best Mode]

An aspect in the present invention provides
an analysis apparatus 100 including an information receiving unit 110 configured to receive a tissue image of a tissue of interest provided with an exploring material with which a labeling material for analysis is bonded and transcriptome information sharing spatial information of the tissue of interest, a spatial mapping unit 120 configured to generate spatial mapping information by spatially mapping the tissue image by the labeling material of the exploring material with the spatially reserved transcriptome information, a transcriptome information extraction unit 150 configured to extract the transcriptome information in the tissue relating to distribution of the exploring material from the spatial mapping information, and a molecular marker analysis unit 160 configured to analyze a molecular marker relating to the distribution of the exploring material in the tissue of interest through the extraction.

The labeling material for the analysis may be a radioactive material, a fluorescent material, a pigment material, or a luminescent material. The fluorescent material may be a fluorescent dye, a fluorescent protein including GFP, YFP, CFP, and RFP, or nanoparticles emitting fluorescence, but is not limited thereto. Examples of the fluorescent dye may include DiO, DiA, Dil, DiR, DiD, ICG, Alexa Fluor 350, Alexa Fluor 405, Alexa Fluor 430, Alexa Fluor 488, Alexa Fluor 514, Alexa Fluor 532, Alexa Fluor 546, Alexa Fluor 555, Alexa Fluor 568, Alexa Fluor 594, Alexa Fluor 633, Alexa Fluor 635, Alexa Fluor 647, Alexa Fluor 660, Alexa Fluor 680, Alexa Fluor 700, Alexa Fluor 750, Alexa Fluor 790, fluorescein o-acrylate (FA) ($\lambda_{ex}$= 490 nm, $\lambda_{em}$= 514 nm), nile blue acrylamide (NBAM) ($\lambda_{ex}$= 628 nm, $\lambda_{em}$= 667 nm), Indo-1, Ca saturated($\lambda_{ex}$= 331 nm, $\lambda_{em}$= 404 nm), Indo-1, $Ca^{2+}$($\lambda_{ex}$= 346 nm, $\lambda_{em}$= 404 nm), Cascade Blue BSA pH 7.0, Cascade Blue, LysoTracker Blue, LysoSensor Blue pH 5.0, LysoSensor Blue, DyLight 405, DyLight 350, BFP (Blue Fluorescent Protein), 7-Amino-4-methylcoumarin pH 7.0, Amino Coumarin, AMCA conjugate, Coumarin, 7-Hydroxy-4-methylcoumarin, 7-Hydroxy-4-methylcoumarin pH 9.0, 6,8-Difluoro-7-hydroxy-4-methylcoumarin pH 9.0, Hoechst 33342, Pacific Blue, Hoechst 33258, Pacific Blue antibody conjugate pH 8.0, SYTOX Blue-DNA, CFP (Cyan Fluorescent Protein), eCFP (Enhanced Cyan Fluorescent Protein), 1-Anilinonaphthalene-8-sulfonic acid (1,8-ANS), 1,8-ANS (1-Anilinonaphthalene-8-sulfonic acid), evoglow-Pp1, evoglow-Bs1, evoglow-Bs2, Auramine O, LysoSensor Green, eGFP (Enhanced Green Fluorescent Protein), LysoTracker Green, Sapphire, BODIPY FL conjugate, MitoTracker Green, Calcein pH 9.0, FDA, DTAF, CFDA, Rhodamine 110, Acridine Orange, and the like, but is not limited thereto. The radioactive material may include radioactive isotopes such as $^{60}$Cu, $^{61}$Cu, $^{62}$Cu, $^{64}$Cu, $^{67}$Cu, $^{66}$Ga, $^{67}$Ga, $^{68}$Ga, $^{44}$Sc, $^{47}$SC, $^{111}$In, $^{114m}$In, $^{114}$In, $^{86}$Y, $^{90}$Y, $^{212}$Bi, $^{213}$Bi, $^{212}$Pb, $^{225}$Ac, $^{89}$Zr 및 $^{177}$Lu, but is not limited thereto.

The bonding of the labeling material for the analysis with the exploring material may be electrostatic, physical, chemical, or biological bonding.

The exploring material is a material that exhibits physiological activity in the tissue of interest or is distributed in the tissue and is a subject to be explored, and may be selected from the group consisting of nanomaterials (examples: polymeric nanoparticles, lipid-based nanoparticles, polymeric double-layer structure nanoparticles, protein nanoparticles, inorganic nanoparticles, or crystalline nanoparticles), low molecular compounds, high molecular compounds, natural products, aptamers, nanobodies, microorganisms, antibodies, engineering antibodies, antibody-drug conjugates, extracellular vesicles, cells, peptides, nucleic acids, proteins, amino acids, sugar, lipid, biopharmaceuticals or biocandidates (examples: biological agents, genetically recombinant medicines, cell culture medicines, biosimilars, biobetters, advanced biopharmaceuticals, or candidates thereof), synthetic chemical drugs or synthetic chemical candidates, and natural drug products or natural product candidates. The tissue of interest may be skin, intestines such as the small or large intestine, heart, lung, kidney, liver, spleen, muscle, tumor tissue, or the like, but is not limited thereto.

The information receiving unit 110 may additionally receive a tissue image in which the tissue of interest is randomly stained.

The staining method may include alkaline phosphate assay (ALP assay), Sirius red staining, Alcian blue staining, pH map, H&E staining, Trichrome staining, Priodic acid-Schiff (PAS) staining, immunohistochemical staining, etc., but is not limited thereto. The stained tissue image may be used as a complementary or supplementary means to test analysis results of the present invention or to extract useful information during molecular marker analysis. In one example, the tissue staining image is used by itself, or by applying an image feature extraction algorithm using artificial intelligence, for example, spatial gene expression patterns by deep learning of tissue images (SPADE) algorithm, it is possible to extract the image features and obtain SPADE gene information relating to the extracted features.

The exploring material may be provided to the tissue of interest by being directly provided to the exploring material in the tissue of interest or administered to a subject through systemic administration (e.g., intravenous injection) and then distributed to the tissue of interest. The tissue image may be transmitted to a microscope capable of measuring the labeling material, and may be obtained as an entire slide image through a process of tiling the labeling material and transmitted to the information receiving unit 110.

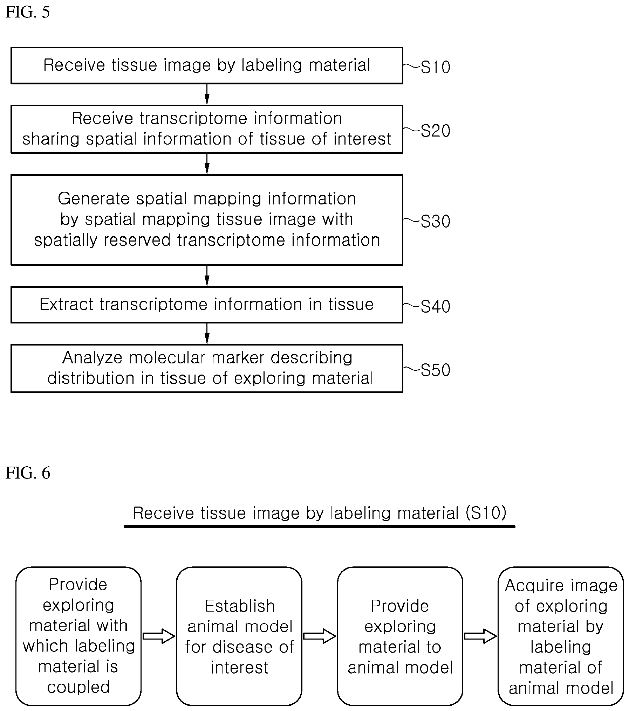

In addition, the information receiving unit 110 may receive a tissue image generated by providing an exploring material to an established animal model having a disease of interest, thereby analyzing the molecular marker that may describe the distribution of the exploring material or the physiological activity information in specific diseases (see FIG. 6).

The diseases of interest are not particularly limited and may include various cancers, a brain disease, a neurological disease, a liver disease, an intestinal disease, an immune disease, a viral disease, a kidney disease, an inflammatory disease, a metabolic disease, a skin disease, a diabetes disease, an infectious disease, a cardiovascular disease, a neurodegenerative disease, etc., and in more specific examples, may be a cancer disease, a brain disease, a diabetic disease, an inflammatory disease, a viral disease, an infectious disease, etc.

The tissue image may be used as it is, or in order to effectively achieve the spatial mapping used, an image transformed from the tissue image using an image registration process of a specific algorithm may be used. To this end, the analysis apparatus 100 may further include an image transformation unit 130 for converting the tissue image using the image registration process of a specific algorithm.

This image registration process may be necessary to match the tissue image with spots of the spatially reserved transcriptome information. An image that is subjected to an algorithm for effective spatial mapping of the tissue image is referred to as a 'registered image' in this specification. The algorithm may be an algorithm that performs translation transformation, rotation transformation, and other topological transformations on an image. Examples of the algorithm include an algorithm provided by a Python-based open-source DiPY package, a method of using a bUnwarpJ module in imageJ, and the like, and the known algorithms may be used without limitation.

In addition, in order to effectively perform the spatial mapping, the transformed images for the tissue images or for the registered images may be used without the registered image into which the tissue image is transformed or the registered image. The transformed image refers to an image that is discontinuously transformed according to image intensity for the tissue image or the registered image. In this case, the image transformation unit 130 may generate the transformed image through the image conversion for the tissue image or the registered image.

For example, the image transformation may be a binary translation that performs splitting into a tissue image spot of high uptake labeling material and a tissue image spot of low uptake labeling material. The binary translation converts the obtained tissue image into data as 0 or 1 based on a specific value for the image intensity. For example, based on the strongest fluorescent intensity value, it may include obtaining an image in which the intensity of about 25% is converted to 0, and obtaining an image in which the intensity of the rest (i.e., intensity more than or equal to 25%) is converted to 1. This binary transformed image may be an example of the 'transformed image' herein.

The present invention may use the tissue image by the labeling material as it is or use the 'registered image' and/or the 'transformed image' in the spatial mapping. Specifically, the registered image may be obtained from the received tissue image, and the transformed image obtained by converting the registered image based on the image intensity may be used.

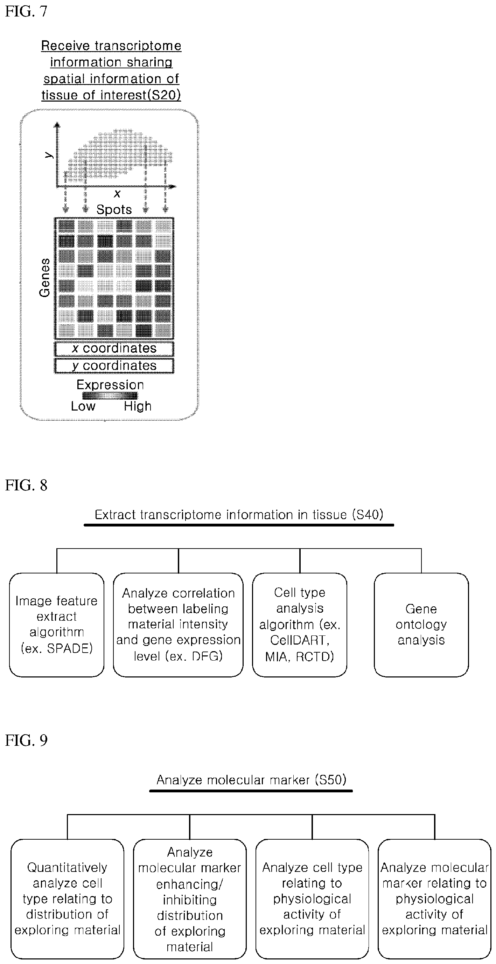

In addition, the information receiving unit 110 may receive the transcriptome information sharing the spatial information of the tissue of interest. The spatially reserved transcriptome information is a technology that provides hundreds to tens of thousands of gene expression information at once and acquires full-length or partial gene expression including spatial information (see FIG. 7). The spatially reserved transcriptome information may be analyzed by fixing a frozen section of the tissue of interest in a mold and performing steps such as permeabilization, cDNA synthesis, and RNA sequencing. The section of the tissue of interest from which the spatially reserved transcriptome information is obtained may be a 'continuous' tissue section with the section for obtaining the tissue image of the exploring material. The spatially reserved transcriptome information may be the number of transcriptome RNA reads and a type of RNA in a spot having spatial coordinates in the tissue of interest. Examples of the types of RNA may refer to information such as whether it is mouse RNA or human RNA, etc., whether to look at gene expression, exons, or introns, whether it includes event-based alternative splicing or isoform-based alternative splicing, whether to look at a mutation burden, and whether it includes noncoding RNA or nontranslated RNA.

In the spatial mapping, the spatially reserved transcriptome information may be information in which a program that visualizes a spot according to a genetic feature is used or a spot with less than the certain number of RNA reads is excluded. For example, to visualize a spot according to the genetic feature, it is possible to use t-distributed stochastic neighbor embedding (t-SNE) using a Seurat package in R. In addition, spots with RNA reads, for example, less than 1000, less than 900, less than 800, less than 700, less than 600, less than 500, less than 400, less than 300, less than 200, or less than 100 may be excluded from the analysis.

The spatial mapping unit 120 may generate spatial mapping information by spatially mapping the tissue image by the labeling material of the exploring material with the spatially reserved transcriptome information.

Herein, the term 'spatial mapping' refers to a process of spatially matching coordinates of the spot of the spatially reserved transcriptome information arranged in a two-dimensional plane with coordinates of a pixel in the tissue image by the labeling material of the exploring material arranged in the corresponding two-dimensional plane for the spatial mapping. The 'spatial mapping' refers to a process of generating one information set by matching the position of the pixel of the registered or transformed image obtained after the registration or conversion with the coordinates of each spatial transcriptome spots.

The spatial mapping unit 120 may spatially match the coordinates of the spot of the spatially reserved transcriptome information arranged in the two-dimensional plane with the coordinates of the pixel in the tissue image by the labeling material of the exploring material arranged in the corresponding two-dimensional plane for the spatial mapping.

In one example, before or after the spatial mapping, the tissue image (or including the registered image or transformed image thereof) by the labeling material may be partitioned into one or more clusters. To this end, the analysis apparatus 100 may further include a clustering unit 140 that partitions the tissue image (or including the registered image or transformed image thereof) by the labeling material into one or more clusters before or after the spatial mapping.

The one or more clusters may be classified using the image intensity by the labeling material, or the image by the labeling material of the exploring material may be split into a plurality of patches, classified by an algorithm that performs the classification based on a similarity of image features for each patch, or classified according to genetic consistency.

The tissue image may be classified into cluster 1, cluster 2, cluster 3, etc., and the total number of clusters is 1 or more, specifically 2, 3, 4, 5, 6, 7, 8, 9, 10, 11, 12, 13, 14, 15, 16, 17, 18, 19, or 20 clusters, and is not limited thereto.

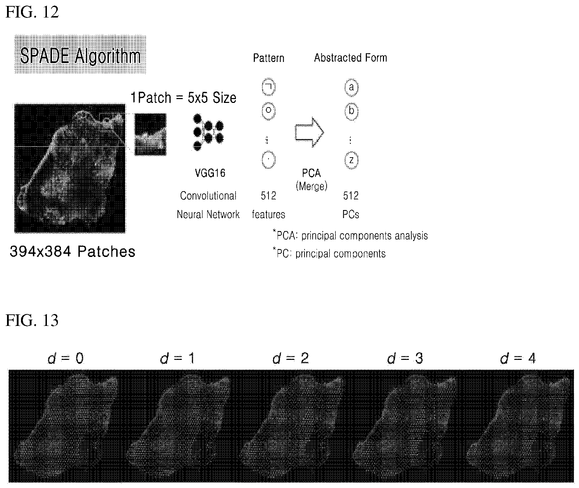

The tissue image by the labeling material may be split into patch tissue images of a preset size, and the features of each patch may be extracted using an image feature extraction model and then classified into one or more clusters according to these features. For example, the tissue image by the labeling material may be split into $394 \times 384$ patches, each patch size may be $5 \times 5$, 512 features may be extracted for each patch, and classified into one or more clusters based on the features (see FIG. 12).

Examples of the algorithm that performs the classification based on the similarity of the image features include K-means clustering, unsupervised hierarchical clustering, stochastic neighbor embedding, agglomerative clustering, spectral clustering, or Gaussian mixture clustering algorithms, and more specifically, the K-means clustering or unsupervised hierarchical clustering algorithms may be used.

The transcriptome information extraction unit 150 may extract the transcriptome information in the tissue relating to the distribution of the exploring material with which the labeling material from the spatial mapping information or the spatial mapping information of each cluster is bonded.

Referring to FIG. 8, for the extraction of the transcriptome information in the tissue (S40), the known algorithms or analysis methods, such as image (or image) feature extraction algorithm using artificial intelligence, correlation analysis between the image intensity and the gene expression level of the labeling material in the tissue, a cell type analysis algorithm, and/or gene ontology analysis, may be used (see FIG. 8).

Specifically, the transcriptome information extraction unit 150 may include at least one of an image feature analysis unit (152) that extracts the transcriptome information using an image (or image) feature extraction algorithm using artificial intelligence, a correlation analysis unit 154 that extracts the transcriptome information by analyzing a correlation between image intensity and a gene expression level of the labeling material in the tissue, a cell type analysis unit 156 that uses a cell type analysis algorithm; and a gene ontology analysis unit 157 that uses gene ontology analysis.

The transcriptome information extraction unit 150 may use the known algorithms or analysis methods in addition to the above-described algorithms or analysis methods.

The image feature analysis unit 152 is configured to extract transcriptome information using an image (or image) feature extraction algorithm using artificial intelligence. As the image feature extraction algorithm using artificial intelligence, the spatial gene expression patterns by deep learning of tissue images (SPADE) algorithm, etc., may be used. The SPADE algorithm is known at **https://doi.org/10.1101/2020.06.15.150698** and is hereby incorporated by reference in its entirety. The image feature extraction algorithm extracts features from the tissue image by the labeling material or each cluster of an image and provides SPADE gene information relating to the extracted features. The SPADE algorithm uses a pre-trained VGG16 model to extract, for example, 512 features per patch around each point, performs principal component analysis (PCA) to reduce the dimensionality of features, and uses a principal component (PC) to identify the SPADE genes.

The correlation analysis unit 154 may perform correlation analysis between the image intensity and the gene expression level of the labeling material in the tissue, and use differentially expressed genes (DEG), correlation analysis by calculation of the correlation coefficient, or an image similarity evaluation algorithm, etc. The analysis method is an analysis method of measuring an expression value of a gene, processing the expression value statistically, and selecting significantly expressed genes based on the image intensity. The correlation coefficient calculation may be a Pearson correlation coefficient, a Spearman correlation coefficient, or a Kendall correlation coefficient calculation, and an example of the image similarity evaluation algorithm may be structured similarity image matching (SSIM). The selected genes may be genes that are correlated with the distribution of the exploring material. The analysis method may select differentially expressed genes between high uptake spots and low uptake spots based on the intensity of the labeling material. For example, the DEG may be classified by a fold change. The fold change indicates a gene expression level that has increased or decreased several times based on a default or reference value. The DEG analysis may be a method of testing site-specific genes and then analyzing uptake-specific genes.

The cell type analysis unit 156 may perform cell type analysis using a cell type analysis algorithm, and the cell type analysis algorithm may be Fisher's exact test, maximum likelihood estimation, domain adaptive classification, logistic regression analysis, or a negative binomial regression algorithm. Specific examples thereof may include multimodal intersection analysis (MIA), a cell type inference by domain adaptation of single-cell and spatial transcriptomic data (CellDART) algorithm, a robust decomposition of cell type mixtures in spatial transcriptomics (RCTD) algorithm, celltypist, cell2location, or the like, but is not limited thereto, and the known cell type analysis tool may be used. The MIA is an analysis method that informs which type of cell is located at any location from the spatially reserved transcriptome information. The method uses a relatively easy statistical technique called hypergeometric test (Fisher's exact test). The CellDART algorithm is an algorithm that classifies cells from the spatial transcriptome information. The RCTD matches cell types using a supervised method such as maximum likelihood estimation, and may also determine cell doublet, which may not be determined by the existing unsupervised methods, which may be called by RCTD analysis.

The gene ontology analysis unit 157 performs analysis using gene ontology analysis, and the gene ontology (GO) analysis is a database that is organized in a model structuring genes (proteins) according to three perspectives, that is, individual genes according to a biological process (BP) to which genes are related, a cellular component (CC), and a molecular function (MF). The names of genes (proteins) are different for each species, but by organizing the genes into common terms, it is useful for comparing functions between different species and provides a function to examine biological functions that are statistically significantly changed. To analyze the function of the gene of interest, gene annotation may be performed against the gene ontology database, and significant results may be obtained through statistical methods.

The transcriptome information extraction unit 150 may perform any one of the image feature extraction algorithm, the correlation analysis between the image intensity and the gene expression level of the cluster, the cell type analysis, and the gene ontology analysis on the entire image or each cluster image by the labeling material, but may perform two or more of these analysis methods and extract commonalities or synthesize the analysis results to extract useful information.

The molecular marker analysis unit 160 may analyze the molecular marker that describes the distribution of the exploring material in the tissue of interest through the extraction.

The molecular marker may be a single molecule derived from DNA, RNA, metabolite, protein, protein fragment, etc., or molecular information based on patterns thereof. The molecular marker may be a material that enhances or inhibits the distribution of the exploring material, blocks or enhances the target of the exploring material, enhances or inhibits the action of the exploring material, or is related to the distribution of the exploring material.

As a concrete example, when the transcriptome government extraction unit 150 extracts the transcriptome information of each cluster, the molecular marker analysis unit 160 may compare the extracted transcriptome information for each cluster, select a cluster that is more related to the distribution of the exploring material, and derive the molecular marker from the transcriptome information of the selected cluster.

Alternatively, the molecular marker analysis unit 160 may compare the transcriptome information extracted for each cluster and derive the molecular markers that may explain the distribution of the exploring material from the comparison.

As an example, as a result of the DEG analysis and SPADE algorithm on the entire tissue image, when in the DEG analysis, Hbb-bs, which is a strong indicator of blood-related actions on a tissue surface, is derived as an important gene and in the SPADE, Lbp, Apod, and Fabp4, which are endothelium-related molecular markers, are derived as the top ranked up-regulated genes on a tissue surface, the molecular marker analysis unit 160 may draw conclusions that genes or proteins related to blood vessels, matrices, surface-related activities, etc., are related to the uptake of the exploring material.

As another example, the molecular marker analysis unit 160 may perform one or more analyses, such as the image feature extraction algorithm, the correlation analysis between the image intensity and the gene expression level of the labeling material in the tissue, the cell type analysis, and the gene ontology analysis, on each cluster such as cluster 1, cluster 2,..., to analyze the molecular markers of each cluster, or integrate the results of the clusters to analyze the molecular markers.

As a concrete example, when blood-related genes such as Hba-a2, Hba-a1, and Hbb-bs in the DEG of cluster 1 and glycolysis regulatory enzymes such as Pfkp, Gapdh, and Hk1 in the DEG of cluster 2 and the gene ontology analysis were commonly found, the molecular marker analysis unit 160 may derive information that cluster 1 has a surface-related tendency, and therefore factors such as hemodynamics affect the distribution and physiological activity of the exploring material and cluster 2 indicates that glucose metabolism may be important in the distribution or physiological activity of the labeling material.

Another aspect of the present invention provides an analysis apparatus 100 including an information receiving unit 110 configured to receive a tissue image of a tissue of interest provided with a first exploring material(s) with which a first labeling material for analysis is bonded and transcriptome information sharing spatial information of the tissue of interest, a spatial mapping unit 120 configured to generate spatial mapping information by spatially mapping the tissue image by the labeling material of the exploring material with the spatially reserved transcriptome information, and a physiological activity information analysis unit 170 configured to analyze physiological activity information in a tissue of interest relating to distribution or action of the first exploring material.

The first exploring material may be provided directly to the tissue of interest, or may be distributed to the tissue of interest after being administered to a subject by systemic administration.

In the above aspect, the definitions of the terms 'labeling material', 'exploring material', 'spatially reserved transcriptome information', 'tissue of interest', 'spatial mapping', and the like are the same as in the above-described aspect, and therefore a description thereof will be omitted.

In the above aspect, the term 'first labeling material' is the same as 'labeling material' in the above-described aspect.

In the present invention, the 'physiological activity information' refers to materials that affect the distribution or a function or physiology of a living body, single molecule derived from DNA, RNA, metabolites, proteins, protein fragments, etc., molecular information based on patterns thereof, or all information on factors that affect the function or physiology of the living body, in relation to the exploring materials. The materials that affect the function or physiology of the living body include nucleic acids, nucleotides, proteins, peptides, amino acids, sugars, lipids, vitamins, compounds, etc., and refer to all materials that affect the function or physiology. The physiological activity information may be the same as or different from the molecular marker. In an example, the materials that affect the function or physiology of the living body may be information on materials that interact with the first exploring material or promotes, induces, blocks, or inhibits the action of the exploring material in the tissue. For example, when glycolysis regulatory enzymes such as Pfkp, Gapdh, and Hk1 are commonly found in the DEG and gene ontology analysis results, nucleic acids, nucleotides, proteins, peptides, amino acids, sugars, lipids, vitamins, compounds, etc., which are related to glucose metabolism or promote, induce, block, or inhibit glucose metabolism, including the regulatory enzymes may be the physiological activity information.

In a concrete example, to analyze physiological activity information of the first exploring material in the tissue of interest, the information receiving unit 110 may additionally receive an image of a tissue of interest by a second labeling material of a second exploring material to which the second labeling material is bonded.

Here, the second labeling material may be the same as or different from the first labeling material. The second exploring material is different from the first exploring material. The analysis apparatus 100 according to the present invention may compare and analyze the spatial mapping information of the first and second exploring materials to analyze the physiological activity information of the first exploring material.

The first exploring material or the second exploring material each may be independently selected from the group consisting of a nanomaterial, a low molecular compound, a high molecular compound, a natural product, aptamer, a nanobody, a microorganism, an antibody, an engineering antibody, an antibody-drug conjugate, an extracellular vesicle, a cell, peptide, nucleic acid, protein, amino acid, sugar, lipid, biopharmaceutical or biocandidate, a synthetic chemical drug or a synthetic chemical candidate, and a natural product medicine or a natural product candidate. In an example, the second exploring material may be the known material with a known pharmacological mechanism or activity.

In one example, when the spatial mapping information obtained from the second exploring material is similar to the spatial mapping information of the first exploring material, the physiological activity information analysis unit 170 may estimate that both exploring materials have similar pharmacological mechanisms or activities.

In one example, when the second exploring material is known to act as an inhibitor of a specific receptor, the physiological activity information analysis unit 170 may estimate that the first exploring material does not act as the inhibitor of the specific receptor when the obtained spatial mapping information does not match the spatial mapping information of the first exploring material. In this regard, for example, in the literature [Reference: Cancer Biotherapy and Radiopharmaceuticals, 32(3), 83-89.], the distribution of the target antigen and the distribution of the antibody that inhibits it are significantly different, and therefore, it has been reported that the intended effect of the antibody is not properly exerted.

Another aspect of the present invention provides an analysis apparatus 100 including an information receiving unit 110 configured to receive a tissue image of a tissue of interest provided with an exploring material with which a labeling material for analysis is bonded and transcriptome information sharing spatial information of the tissue of interest, a spatial mapping unit 120 configured to generate spatial mapping information by spatially mapping the tissue image by the labeling material of the exploring material with the spatially reserved transcriptome information, and a physiological activity information analysis unit 170 configured to analyze physiological activity information of the exploring material in the tissue of interest.

In this case, the analysis apparatus 100 may include a clustering unit 140 configured to partition the tissue image (or including registered image or transformed image thereof) by the labeling material into one or more clusters before or after the spatial mapping.

The splitting of the tissue image into two or more clusters by the clustering unit 140 may be performed before or after the step of obtaining the spatial mapping information. The splitting into the cluster may be performed according to the intensity of the tissue image by the labeling material, and for example, may be the splitting into two clusters based on the maximum image intensity into more than 25% and less than 25% of the image intensity or into three clusters based on 50% and 25%, but is not limited thereto.

Alternatively, the clustering unit 140 may split an image by the labeling material of the exploring material into a plurality of patches and classify the image by an algorithm that performs the classification based on a similarity of image features for each patch. The tissue image by the labeling material may be split into patch tissue images of a preset size, and the features of each patch may be extracted using an image feature extraction model and then classified into one or more clusters according to these features.

For example, the tissue image by the labeling material may be split into $394 \times 384$ patches, each patch size may be $5 \times 5$, 512 features may be extracted for each patch, and classified into one or more clusters based on the features (see FIG. 12).

Examples of the algorithm that performs the classification based on the similarity of the image features include K-means clustering, unsupervised hierarchical clustering, stochastic neighbor embedding, agglomerative clustering, spectral clustering, or Gaussian mixture clustering algorithms, and more specifically, the K-means clustering or unsupervised hierarchical clustering algorithms may be used.

Thereafter, the spatial mapping unit 120 may generate spatially mapped information of each cluster by spatially mapping the transcriptome information with the spatial information of the tissue for each cluster. Here, the spatial mapping information may include spatially mapped information of one or more clusters.

In addition, the analysis apparatus 100 may include a transcriptome information extraction unit 150 that extracts the transcriptome information in the tissue relating to the distribution of the exploring material from the spatial mapping information. The transcriptome extraction unit 150 may extract the transcriptome information of the corresponding cluster from the spatially mapped information of each cluster.

For example, the transcriptome information extraction unit 150 may extract the transcriptome information by using the known algorithms or analysis methods, such as the image feature extraction algorithm, the correlation analysis between the image intensity and gene expression level of the labeling material in the tissue, the cell type analysis, and/or the gene ontology analysis, for cluster 1, and extract the transcriptome information for cluster 2 or 3 in the same way as cluster 1.

The physiological activity information analysis unit 170 may compare and analyze the spatially reserved transcriptome information for each cluster to analyze the physiological activity information in the tissue of the exploring material.

The physiological activity information refers to materials that affect the distribution or a function or physiology of a living body, single molecule derived from DNA, RNA, metabolites, proteins, protein fragments, etc., molecular information based on patterns thereof, or all information on factors that affect the function or physiology of the living body, in relation to the exploring materials.

For example, information on which cell types or molecular markers are significantly characteristic of which cluster, information on the transcriptome significantly correlated with labeling material intensity, information on the physiological function of the transcriptome, and the like may be obtained from the comparison and analysis.

Another aspect of the present invention provides a method of analyzing a molecular marker or physiological activity information in a tissue relating to distribution or physiological activity of an exploring material in the tissue performed in the analysis apparatus 100.

FIG. 5 is a flowchart illustrating an analysis method performed in the analysis apparatus 100 according to the present invention. The analysis method performed in the analysis apparatus 100 according to the present invention may include receiving a tissue image by a labeling material (S10), receiving transcriptome information sharing spatial information of a tissue of interest (S20), calculating spatial mapping information by spatially mapping the tissue image with spatially reserved transcriptome information (S30), extracting the transcriptome information in the tissue (S40), and analyzing a molecular marker or physiological activity information in the tissue relating to distribution of an exploring material in the tissue or physiological activity of the exploring material (S50).

Referring to FIG. 6, the tissue image by the labeling material may be obtained by providing the tissue with the exploring material with which the labeling material is bonded. In addition, by establishing an animal model of a disease of interest and providing the exploring material to the animal model, the image of the exploring material may be obtained by the labeling material of the animal model, and may be transmitted to the information receiving unit 110 of the analysis apparatus 100 according to the present invention.

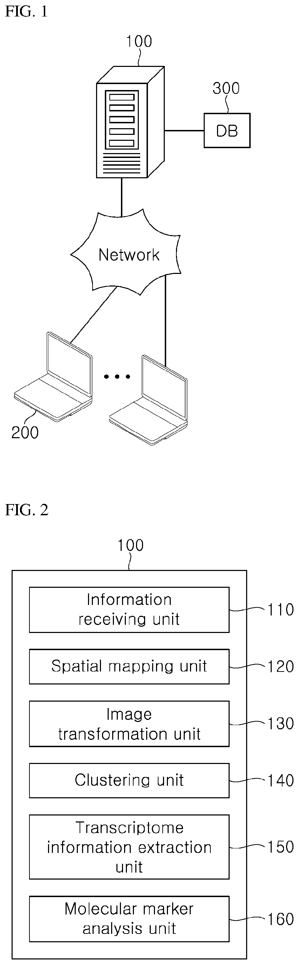

Another aspect of the present invention provides a system including an analysis apparatus 100 for analyzing a molecular marker or physiological activity information in a tissue relating to distribution or physiological activity of an exploring material in the tissue (FIG. 1).

The system may include the analysis apparatus 100 for analyzing a molecular marker or physiological activity information in a tissue relating to distribution of an exploring material or physiological activity of the exploring material, and a user terminal 200 connected to the analysis apparatus 100 through a network.

The analysis apparatus 100 may be a server for analyzing a molecular marker or physiological activity information in a tissue relating to distribution of an exploring material or physiological activity of an exploring material.

The user terminal 200 corresponds to a computing analysis apparatus connected to the analysis apparatus 100 through the network, and may be implemented, for example, as a desktop, a laptop, a tablet PC, or a smartphone, and may include a network interface for network connection to the analysis apparatus 100 and a user input/output interface for user input/output.

For example, the user terminal 200 may correspond to a mobile terminal and may be connected to the analysis apparatus 100 through cellular communication or Wi-Fi communication. As another example, the user terminal 200 may correspond to a desktop and may be connected to the analysis apparatus 100 through the Internet.

In addition, the system may further include a database (DB) 300 that stores various transmission and reception data including the tissue image by the labeling material and the transcriptome information sharing the spatial information of the tissue of interest.

Another aspect of the present invention provides a computer program stored in a computer-readable recording medium for executing a method of analyzing a molecular marker or physiological activity information in a tissue relating to distribution or physiological activity of an exploring material in the tissue.

Hereinafter, the present invention will be described in more detail through Examples and Experimental Examples. However, the following Examples and Experimental Examples are only for illustrating the present invention, and the scope of the present invention is not limited to these only.

**Example**

**Material and Method**

**Material**

1,2-distearoyl-sn-glycero-3-phosphocholine (DSPC), cholesterol, 1,1'-dioctadecyl-3,3,3',3'-tetramethylindocarbocyanine perchlorate was purchased from Sigma-Aldrich, Korea. 1,2-distearoyl-sn-glycero-3-phosphoethanolamine conjugated polyethylene glycol (DSPE-PEG) was purchased from Creative PEGworks. Avanti Mino Extruder was purchased from Avanti Polar Lipids.

**Synthesis and Characterization of DiI Loaded Liposomes**

Liposomes were synthesized by extrusion using an Avanti mini-extruder. The liposomes were composed of DSPC, DSPE-PEG, cholesterol, and DiI fluorescent dye ($\lambda_{ex}$ = 553 nm, $\lambda_{em}$ = 570 nm). Thin film lipids were prepared by vaporizing organic solvents and hydrated with distilled water. The hydrated fluorescent liposome layer was sequentially extruded using 400 nm and 200 nm pore size membrane filters. The DiI-loaded liposome had a uniform and round shape in the TEM image (top of FIG. 19A), which was a typical liposome lipid double-layer structure. The hydrodynamic size of the liposome was 128.05±46.71nm nm in dynamic light scattering (DLS) (middle part of FIG. 19a). At 550 nm excitation, a maximum uptake wavelength and a maximum emission wavelength were 550 nm and 563 nm, respectively (bottom of FIG. 19a).

**Establishment of 4T1 Breast Cancer Model and Fluorescence Imaging**

To prepare a 4T1 allograft tumor model, 4T1 breast cancer cells ($10^6$ cells/0.02 mL) were subcutaneously injected into a right thigh of BALB/c mouse. After ten days, the DiI-loaded liposomes were injected intravenously. In vivo fluorescence imaging was performed at 0, 4, and 24 hours after injection using in vivo imaging system. To confirm the distribution of liposomes in each organ, a mouse was sacrificed after 24 hours of injection. Major organs (heart, lung, kidney, liver, spleen, muscle, and tumor) were collected and observed with the in vivo imaging system for the fluorescence imaging.

**Acquire Spatially Reserved Transcriptome (ST) Library, H&E Staining Image, and Fluorescence Image**

Among the tumor samples, the tumor with the highest signal was selected and used in subsequent experiments. Fresh tumor samples were embedded in a mold with an optimal cutting temperature (OCT) compound (25608-930, VWR, USA) for cryo-sectioning. The ST library was obtained through several steps such as cryo-sectioning, fixation, permeabilization, cDNA synthesis, and RNA sequencing. All methods were performed in the manner recommended by the 10x Genomics visium protocol.

A total of two consecutive tissue slices were obtained. One of the two consecutive tissue slices was used for H&E staining and obtaining the spatially reserved transcriptome library, and the other was used for the fluorescence imaging. Slices were acquired using a thin blade used in a cryotome to be able to thoroughly investigate fluorescence patterns affected by gene expression. The tissue slices for ST were placed on Visium slides (Visium Tissue Optimization Slides, 1000193, 10X Genomics, USA and Visium Spatial Gene Expression Slides, 1000184, 10X Genomics). The fixation was performed under a recommended protocol using cooling methanol. The cDNA library was obtained and sequenced on a NovaSeq 6000 System S1200 (Illumina, USA) at a sequencing depth of less than or equal to 250M read-pairs.

An original FASTQ file and H&E image were processed as samples in Space Ranger v1.1.0 software. The process uses STAR v.2.5.1b (Dobin et al., 2013) for genome alignment with respect to a cell ranger (mouse mm10 reference package). The process was implemented by a 'spaceranger count' comment.

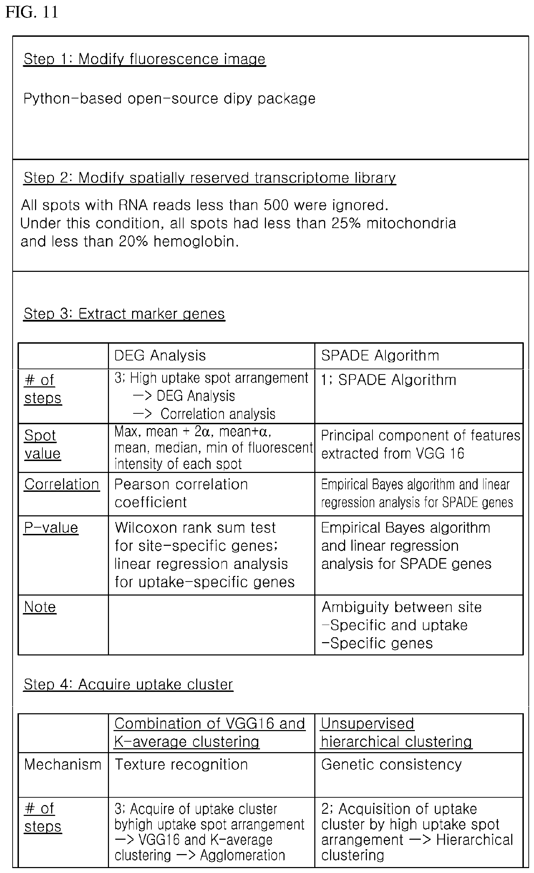

To avoid confusion in terminology, "pixel" was used only in the fluorescence image and "spot" was used only in spatially reserved transcriptome profiles. In addition, all data analysis methods are summarized in several parts below (see FIG. 11).

**Image Registration**

To register the shape of the acquired fluorescence image with the spatially reserved transcriptome spots, an image registration process was implemented using the algorithm provided by the Python-based open source DiPY package. The fluorescence image was transformed into gray scale using an opencv2 package. For registration, the overall centers of both images were matched, and then linear rigid transformation was performed. The rigid and affine transformation processes were optimized using mutual information between two gray scale images. After the linear transformation, a nonlinear warping process based on a symmetric diffeomorphic registration algorithm was performed using the function 'SymmetricDiffeomorphicRegistration' along with 'CCMetric' for optimization. This transformed image was visually evaluated.

When the fluorescence image and the spatial transcriptome spot match well, the image registration may be omitted or replaced by other simplified methods such as image rotation or translation through visual evaluation.

**Distance Annotation of Spatially Reserved Transcriptome Spot**

The distance from the surface of the tumor was calculated and the distance of the spot in the left border area was defined as 0. As the distance was increased one by one, the next layer was immediately displayed. Then, the map was colored with annotated distances. Meanwhile, the fluorescent intensity value for each spot is taken from the registered fluorescence image using the imread function in Python's matplotlib.pyplot to average lengths of patches around each spot so that one length of the patch matches the distance between the spots, thereby representing the fluorescence for each spot. This process was used only to distinguish between site-specific and uptake-specific genes. Finally, a plot that averages the fluorescent intensity values for each distance and shows the relationship between the annotation distance and the average fluorescent intensity was created.

**Mathematical Simulation of Diffusion**

Fick's law is commonly used to describe the dynamics of diffusion (5).

$$\frac{\partial C}{\partial t} + u\frac{\partial C}{\partial x} + v\frac{\partial C}{\partial y} + w\frac{\partial C}{\partial z} = D\left\{\frac{\partial^2 C}{\partial x^2} + \frac{\partial^2 C}{\partial y^2} + \frac{\partial^2 C}{\partial z^2}\right\} + R$$

Here, C denotes a concentration vector, (u,v,w) denotes a velocity vector, D denotes diffusivity, and R denotes a source or sink term. To simulate the results of the above formula, two assumptions were made. First, it was predicted that the number of vascular gaps was proportional to the expression level of the Pecam1 gene. Then, we considered that the tissue sample is approximately the central plane of the entire tumor, allowing us to neglect flow rates and concentration gradients perpendicular to the plane.

(u,v,w) was considered as null vector according to literature (7). Briefly, considering a cylindrical vessel perpendicular to the midplane, the maximum fluid velocity through the vessel may be plotted as the experimentally estimated value:

Vascular diameter = 10μm
Distance between spots = 100μm
Spot height = H μm
Surface area of blood vessel per unit volume of tissue = 0.0034 μm$^{-1}$
Number of gaps per unit area of blood vessel = 500 gaps/mm$^2$
Experimentally estimated flow rate = 0.065μm$^3$/s¡¤gap

$$\therefore \pi \times 10\mu m \times H\mu m \times \text{maximum fluid velocity} =$$

$$\frac{\pi}{4} \times (100 \ \mu m)^2 \times H \ \mu m \frac{0.0034 \ \mu m^2}{1 \ \mu m^3} \times \frac{1 \ mm^2}{10^6 \ \mu m^2} \times 500 \ \text{gaps/mm}^2 \times 0.065 \ \mu m^3/s \cdot gap$$

Therefore, the maximum fluid velocity = 27.625 pm/s was obtained. This means that fluid convection may not even describe the movement of nanoparticles across one point over a 24-hour period.

Therefore, we ignored (u,v,w) and solved the formula using a numerical approach.

$$\frac{C^{t+\Delta t}(x,y) - {}^{\circ}C^t(x,y)}{\Delta t} = D\left\{\frac{C^t(x+\Delta x, {}^{\circ}y) + C^t(x-\Delta x, {}^{\circ}y) - 2C^t(x,y)}{(\Delta x)^2}\right\}$$

$$\cdots\cdots\cdots\cdots\cdots + D\left\{\frac{C^t(x, {}^{\circ}y+\Delta y) + C^t(x, {}^{\circ}y-\Delta y) - 2C^t(x,y)}{(\Delta y)^2}\right\} + k \cdot X \cdot \text{Expression}$$

Here, $\Delta x$ and $\Delta y$ are set equal. Then, various simulation results of C/k annotated Fick diffusion with different

$$\frac{D}{\Delta x^2}$$

values and the number of iterations are explored.

**Confirmation of Delivery of Fluorescent Liposomes to 4T1 Solid Tumor**

We observed with the IVIS fluorescence spectral image apparatus that Dil-loaded liposomes are accumulated in the tumor over time (see FIG. 19b).

In addition, this was confirmed through in vitro fluorescence imaging of normal organs and tumors after 24 hours of injection (FIG. 19c). Higher and heterogeneous uptake of fluorescent nanoparticles was observed in tumors, while weaker uptake was observed in liver and spleen. The fluorescence signal in the kidney may be due to free Dil dye.

**Exploration of H&E Staining, Transcriptome and Fluorescence Image**

Three representative areas stood out on H&E staining: Capsule-like left border, high-density cancer area, and internal necrosis area (A in FIG. 20A). Therefore, H&E image demonstrated that a given sample qualitatively represents the entire tumor microenvironment. The spatial mapping of RNA reads showed that cancer-rich areas showed the highest gene expression and necrosis areas showed the lowest gene expression (FIG. 20C).

Meanwhile, processing a fluorescence image into a binary map goes through several steps (B, C, D in FIGS. 20A to 20B). We also investigated clustering according to the gene expression pattern (FIG. 20D). The average fluorescent intensity over distance was different from a mathematical model of simple passive diffusion, suggesting that the fluorescence pattern within the tumor may be influenced by the complex tumor microenvironment rather than simple physics (FIG. 20E). To predict the passive process of the fluorescent liposome distribution using vascular marker Pecam1, numerical analysis results of Fick's law were obtained. The distribution did not match the actual fluorescent liposome distribution and, in particular, may not describe the uptake of nanoparticles into the tumor (see FIGS. 20F and 21). This was also true for Cd34 which is another representative pan-endothelial marker (see FIGS. 20F and 21).

**Total Fluorescence Analysis**

**Generate Binary Map of Fluorescence Image**

High uptake points were secured using a splitting and agglomerating approach. Initially, the splitting process was performed as follows: Image binarization was performed to connect high-resolution fluorescence image pixels to low-resolution spatially reserved transcriptome spots. By dichotomy, spots with high fluorescence (high uptake spots) and spots with low fluorescence (low uptake spots) were distinguished. When creating the binary image, only pixels with brightness greater than or equal to 25% of the maximum fluorescent intensity were selected as high pixels in imageJ. The fluorescent intensity was measured and analyzed by imageJ (ver 1.8; https://imagej.nih.gov/ij/download.html). Once the binary image was acquired, a binary map was created by searching for the pixel value (i.e., 0 or 1) corresponding to the center of the spot.

Thereafter, the high uptake spots were first agglomerated, and all spots within a certain Euclidean distance from the high uptake spot were assigned as high uptake spots. The distance was determined according to the fold change (FC) or the Pearson correlation coefficient (FIG. 13). It was expected that the parameter dependence occurring in the splitting step is alleviated by the selection process of the optimal distance in the agglomerating step.

**Spatial Mapping of Binary Image and Spatially Reserved Transcriptome Information**

To visualize the spot according to the genetic feature, it is possible to use the t-distributed stochastic neighbor embedding (t-SNE) using a Seurat package (version 4.0.5.) in R. For the quality control, spots with RNA reads less than 500 (i.e., conservative threshold) were excluded from the following analysis.

After the binary image is collected, using .json file (scale information: "spot_diameter_fullres": 56.50370399999998, "tissue_hires_scalef": 0.27979854, "fiducial_diameter_fullres": 91.27521500000002, "tissue lowres_scalef": 0 .08393957) to match the spot to a location of a specific pixel on the image, the spatial mapping was accomplished.

**DEG Analysis**

Perplexity (PPL) of RunTSNE was set to 30. Differentially expressed genes (DEGs) between the high and low uptake spots were explored by FindAllMarkers in the Seurat package in which both min.pct and logfc.threshold are set to 0.25. In addition, only.pos = TRUE was set to make the generated gene site-specific. Finally, DEGs were classified by the fold change (FC).

**SPADE Algorithm Analysis**

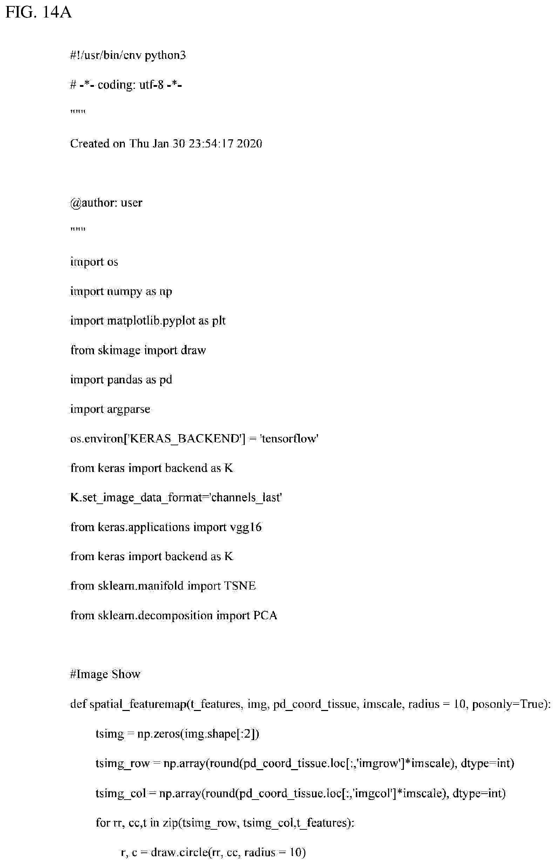

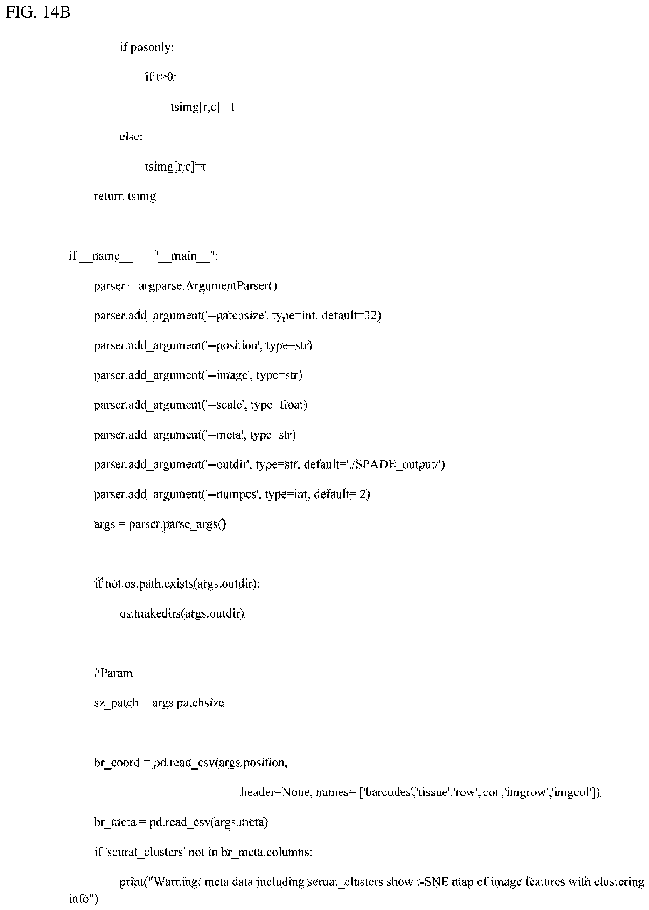

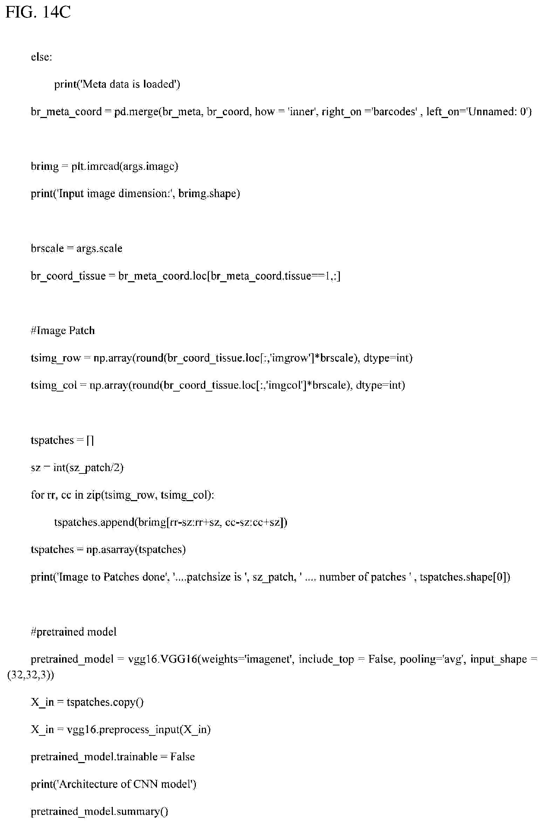

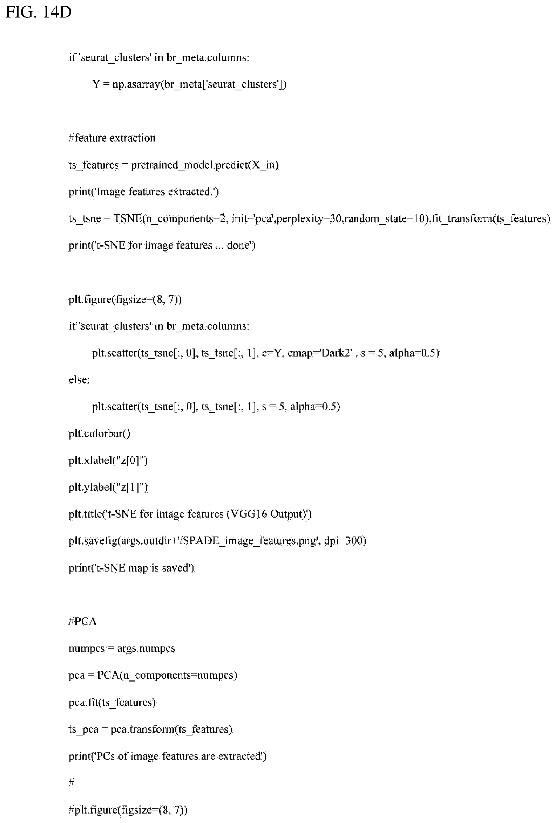

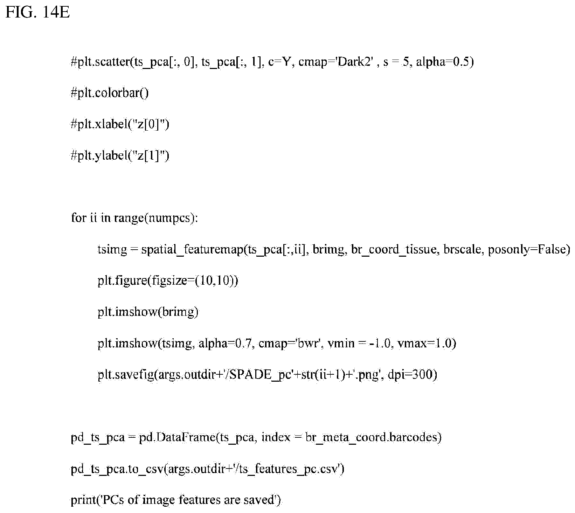

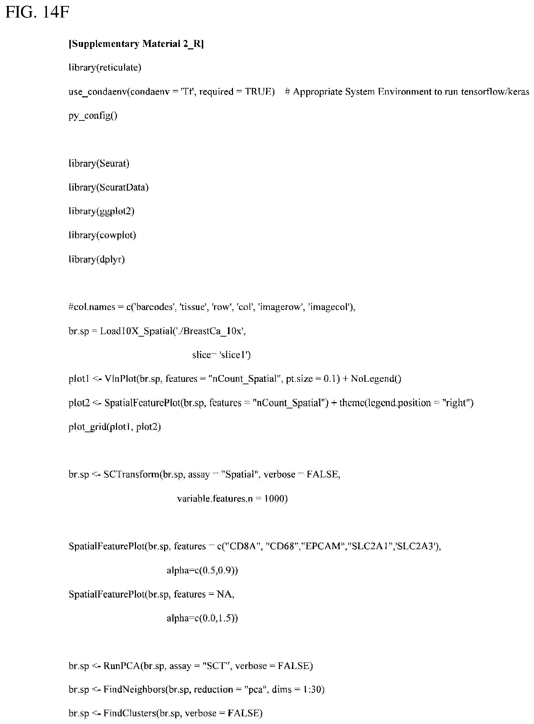

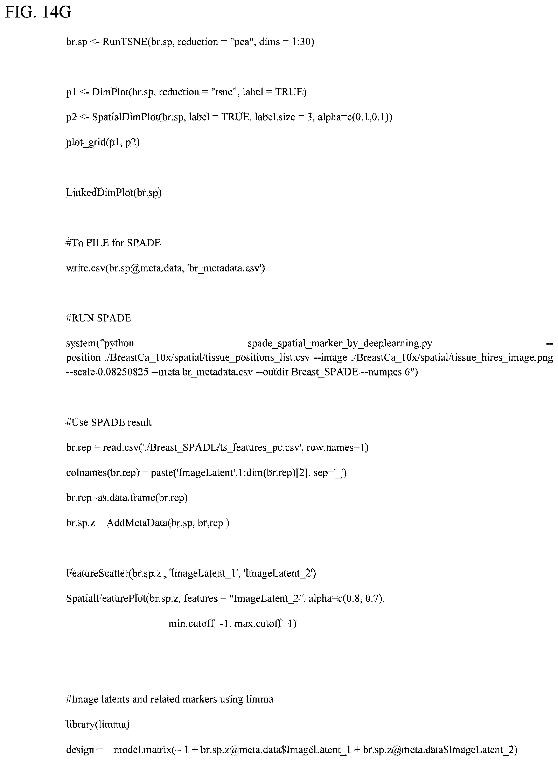

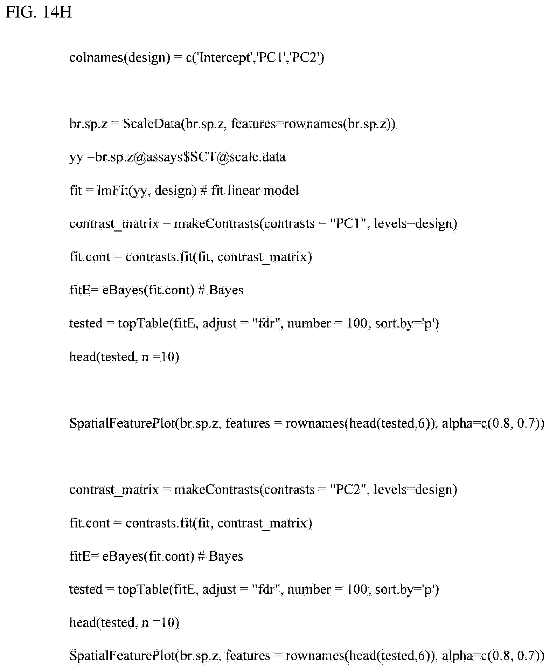

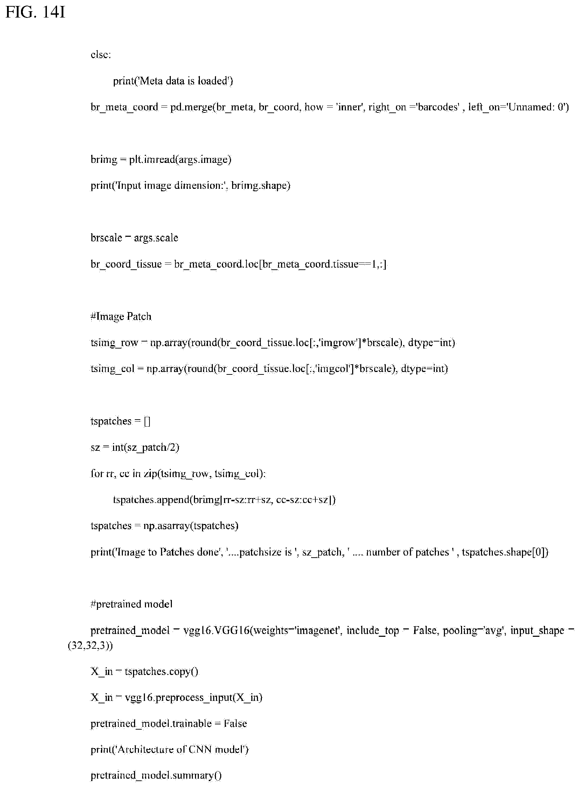

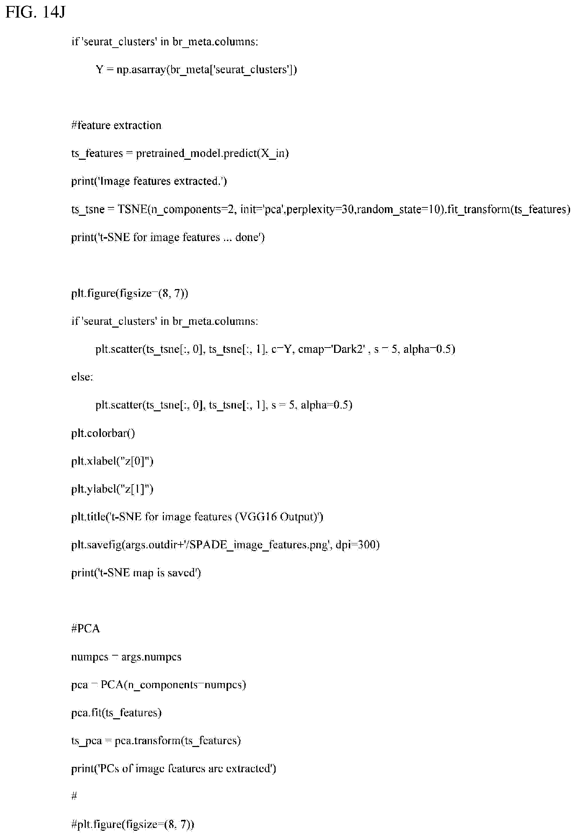

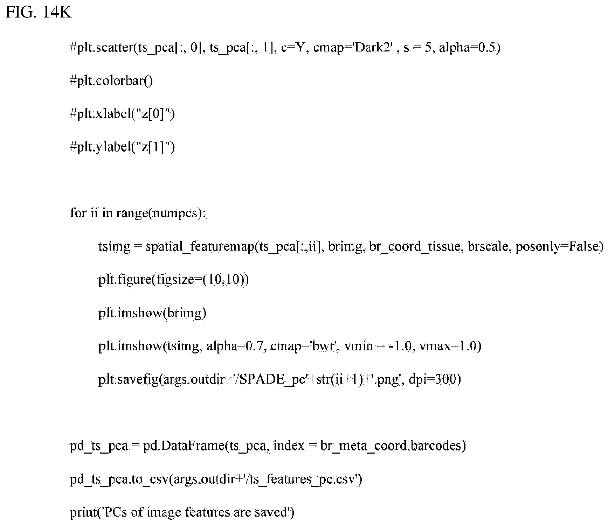

Another approach, which is the spatial gene expression pattern by the deep learning of tissue images (SPADE) algorithm, was used to confirm observations in the all DEG analyses (see FIGS. 14A to 14K). The SPADE algorithm paper (8) published in February 2021 introduces the feature extraction algorithm using VGG16 which won the Image Net Challenge as the CNN deep learning algorithm. The pre-trained VGG16 model extracted 512 features per patch around each spot and performed principal component analysis (PCA) to reduce the dimensions of the features. To identify the SPADE gene, three principal components (PCs) were selected. The patch size was chosen to have the largest average Log FC in PC1. The SPADE genes in each PC were discovered by empirical Bayes algorithm and linear regression analysis. Then, the genes were classified in order of the fold change (FC).

**GO Analysis**

Volcano plot and gene ontology (GO) analysis were performed in R. Briefly, to obtain improved plots, R's EnhancedVolcano function was used along with a pCutoff of 0.05 and FCcutoff of 0.3. The top 1000 genes with FDR less than 0.05 were selected and the spatial feature plot of the top 8 genes with the highest FC was displayed. When using the GO analysis, the enrichGO function in R was used. The GO analysis was performed according to the biological process (BP), the cellular component (CC), and the molecular function (MF) using the top 30 up- or down-regulated genes. When specifying the biological indication, g:Profiler **(https://biit.cs.ut.ee/gprofiler/gost)** was used instead of the GO analysis.

Total fluorescence analysis result: It shows that the drug uptake is influenced by blood circulation

The total fluorescence analysis results are shown in detail in FIGS. 22A to 22E, and the results according to each analysis method are described below.

**DEG Analysis Result**

In all the DEG analyses, there was only one significant gene, referred to as Hbb-bs (FIG. 22A, Table 1).

[Table 1]

| Order | Gene | log2FC | p_val_adj | Order | Gene | log2FC | p_val_adj |
|-------|------|--------|-----------|-------|------|--------|-----------|
| 1 | *Hbb-bs* | 0.755762 | 0.003345 | - | - | - | - |

<List of all DEGs derived by FC from total fluorescence analysis>

Hbb-bs encodes a beta polypeptide chain discovered in hemoglobin of red blood cells and is considered one of red blood cell (RBC) markers (20). It is well known that hemoglobin mRNA remains undegraded as long as red blood cells are alive, and thus, may be used as a powerful indicator of blood-related actions. Accordingly, it was discovered that the expression of several genes related to endothelial cells (e.g., Pecam1, Cd34) and matrix cells (e.g., Fabp4), which are well known to be preferentially distributed near blood vessels, is co-localized with Hbb-bs. This suggests that the overall distribution of fluorescent liposomes is related to blood circulation (FIGS. 22A and 22B). To further investigate the association between the expression of the Hbb-bs and the distribution of the drug, the correlation analysis of the fluorescence signal intensity and the expression level of the Hbb-bs within the high uptake spot was performed. There was no statistically significant correlation between Hbb-bs expression level and fluorescent intensity (r=0.073, p-value=0.188).

**SPADE Analysis Result**

In addition, the SPADE algorithm using a significantly different approach also showed preferential fluorescence patterns in the surface group. It shows three image latent features PC1, PC2, and PC3 of the SPADE algorithm, each of which means principal component 1, principal component 2, and principal component 3. Among these, the latent feature with the largest amount of variance (i.e., PC1) was selected (FIG. 22B). It is noteworthy that in PC1, Lbp, Apod, and Fabp4 were ranked high and up-regulated genes (Table 2). FIG. 22D illustrates a spatial feature plot of the top 8 SPADE genes having the highest fold change (FC).

[Table 2]

| Order | Gene | log2FC | p_vat_adj | Order | Gene | log2FC | p_val_adj |
|---|---|---|---|---|---|---|---|
| 1 | Ctsk | 0.597908 | 8.46E-07 | 11 | Fabp4 | 0.483804 | 0.000151 |
| 2 | Lbp | 0.578055 | 1.81E-06 | 12 | Fosb | 0.482819 | 0.000151 |
| 3 | Mbp | 0.534974 | 1.71E-05 | 13 | Aqpl | 0.478601 | 0.000180 |
| 4 | Sparc11 | 0.532259 | 1.71E-05 | 14 | Rgs5 | 0.468288 | 0.000309 |
| 5 | Apod | 0.529634 | 1.71E-05 | 15 | Igfbp5 | 0.465283 | 0.000332 |
| 6 | Pik3r5 | 0.514425 | 3.94E-05 | 16 | Gas6 | 0.462585 | 0.000359 |
| 7 | Plekha4 | 0.495353 | 9.77E-05 | 17 | Mt3 | 0.459751 | 0.000400 |
| 8 | Nr4a1 | 0.494369 | 9.77E-05 | 18 | Pdlim1 | 0.456149 | 0.000467 |
| 9 | Cilp | 0.494264 | 9.77E-05 | 19 | Scn7a | 0.452321 | 0.000552 |
| 10 | *Selenop* | 0.490979 | 0.000109 | 20 | Mai | 0.442638 | 0.000866 |

<List of top 20 SPADE genes derived by FC from total fluorescence analysis>

Among the SPADE genes, Ctsk, Lbp, Sparcl1, and Apod were the top and up-regulated genes and were enriched in an extracellular matrix (ECM) of the matrix area (FIG. 22D). This discovery is consistent with previous observations that tumor uptake of nanoparticles is related to capillary wall collagen (20). It is well known that Apod is one of the apolipoproteins discovered in the early stages of tumors, which was consistent with the experiment conditions (12). The top 1,000 related genes were acquired by the improved volcano plot. The improved volcano plot preferentially showed the up-regulated genes under the appropriate cutoff (FIG. 22C).

**GO Analysis Result**

In the gene ontology analysis, the SPADE genes had many genes related to fibers and extracellular matrix (ECM) (FIG. 22E). Specifically, FIG. 22E illustrates the gene ontology (GO) analysis results of the top 30 SPADE genes in PC1, up-regulated according to the biological process (BP), the cellular component (CC), and the molecular function (MF), which was mainly associated with the regulation of smooth muscle cell proliferation and fibronectin binding.

**SPADE Algorithm Analysis Result Using H&E Staining Image**

The SPADE algorithm was applied using the H&E staining image. As a result, there were various types of surface families, including an endothelial molecular marker group, a metabolic and signaling molecular marker group, and a lytic activity-related group (FIG. 23). FIGS. 23A to 23C each illustrate improved volcano plots of the top SPADE genes, each GO analysis result, and the top 1000 variable genes in each principal component, that is, PC1 (FIG. 23A), PC2 (FIG. 23B), and PC3 (FIG. 23C) obtained by applying the SPADE algorithm to the H&E staining image. The fluorescence images showed that the molecular markers discovered by the SPADE algorithm were closely related to the first of the three endothelial molecular marker groups.

**Comprehensive Review of Total Fluorescence Analysis Result**

In conclusion, the total fluorescence analysis method including the total DEG analysis and SPADE algorithm may identify genes that determine structural factors of nanodrug uptake, such as blood vessel, matrix, or surface-related activities. The discovery for this approach was supported by several previous papers suggesting the predominance of the passive route. However, this means that the feature maps with the highest-ranked genes were actually far from heterogeneous uptake. This may be due to the heterogeneity between peripheral cells and cells within a tumor.

**Subgroup Fluorescence Analysis**

The Hbb-bs and SPADE genes were found to be related to the fluorescence distribution of nanodrugs in tissues.

However, the gene expression pattern did not match the internal uptake cluster of the tumor (B in FIG. 20A vs. FIGS. 22A and 22D). By speculating that the uptake mechanism of nanodrugs may be different between the surface and internal areas of the tumor, the uptake pattern was analyzed using image feature-based cluster.

**Clustering 1: Classification into Cluster 1 and Cluster 2**

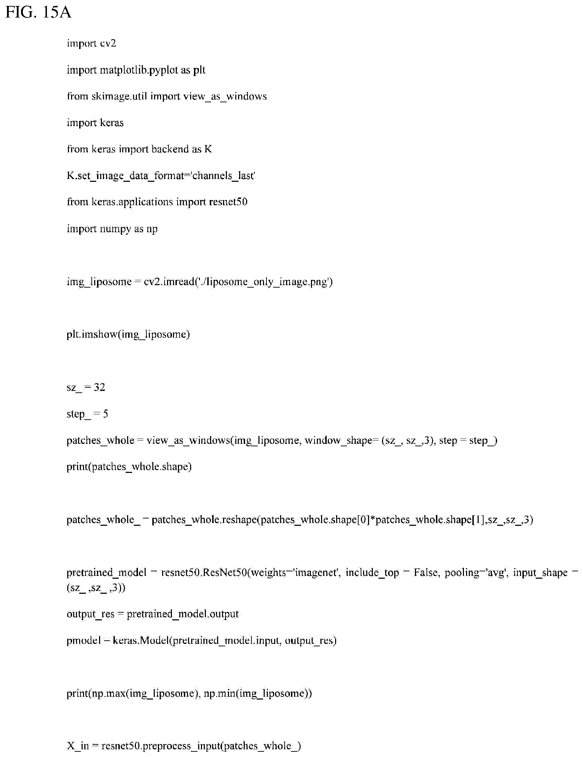

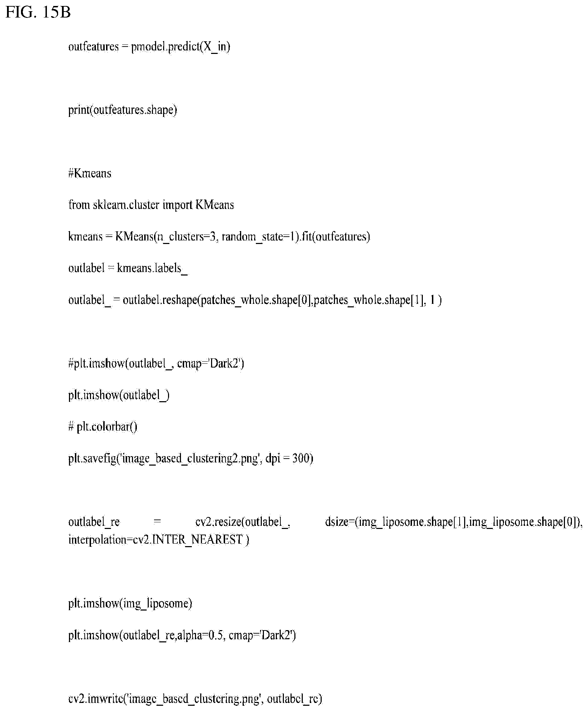

To obtain the uptake clusters, the following approach was used: splitting → clustering → agglomerating. The splitting and agglomerating steps were the same as in the total fluorescence analysis. To perform the clustering of the split spots, two approaches were used: a combination of VGG16 and K-means clustering algorithms and unsupervised hierarchical clustering. By splitting the fluorescence image into 394 × 384 patches with a patch size of 5 × 5 and using the pixels of each patch as the patch input to the VGG16 model, 512 features were extracted for each patch using the VGG16 model (see FIG. 12). Then, the patches were classified by K, which is clustered according to features by setting K to 4 (see FIGS. 15A, 15B, and 25).

As a result, the fluorescence image was separated into four ROIs according to texture. The binary maps described above were agglomerated into two significant ROIs out of four ROIs (see FIG. 24A). As a result, the high uptake spots were split into two clusters and the other spots were assigned to the default value of 0. In conclusion, a total of three clusters were formed. Outliers in each uptake cluster were removed using the unsupervised hierarchical clustering of the spot in clusters 1 and 2. That is, to perform the unsupervised hierarchical clustering, a heatmap was derived from the correlation of each pair of the high uptake spots according to the gene profile or feature extracted from the VGG16 model. It could be found that the high uptake spot was roughly divided in half. Therefore, half of the tumor edge was assigned to cluster 1 and the inner half was assigned to cluster 2. The low uptake spot was assigned to cluster 0. The DEGs representing each uptake cluster were generated compared to the default cluster using FindAllMarkers in R. Therefore, cluster 1 (surface cluster) corresponding to half of the tumor edge and cluster 2 (internal cluster) corresponding to the inner half were obtained based on the high uptake spot among a total of three clusters for subsequent analysis.

**Analysis Method for Cluster 1 and Cluster 2**

**MIA Analysis**

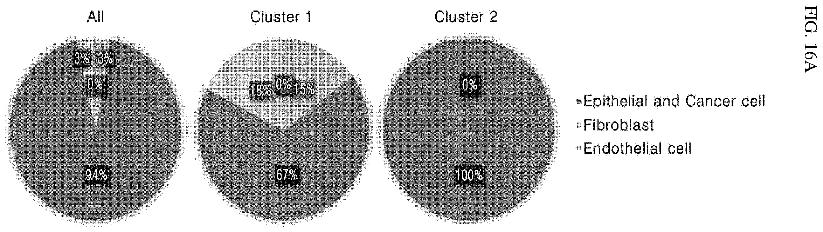

Multimodal intersection analysis (MIA) was performed to understand which cell types were associated with each uptake cluster (21). Single cell RNA sequencing (scRNA-seq) datasets were obtained from the previous study of the 4T1 allograft tumor model (see FIG. 16A) (9). Marker genes for each cell type were determined as an adjusted p-value $<10^{-5}$ in the Wilcoxon rank sum test in FindAllMarkers. In addition, the marker gene for each spatial area was determined similarly to the process for determining the marker gene for each cell type, except that the adjusted p-value $<0.01$ was used instead of the adjusted p-value $<10^{-5}$. Thereafter, each set of common genes in a specific cell type and a specific spatial area was calculated and the abundance or reduction is measured compared to the random output. Finally, all common gene sets were analyzed using the Fisher's exact test to find out which cell types were significantly characterized in the corresponding spatial region.

Due to parameter sensitivity (i.e., p-value dependence) and lack of quantitative criteria for parameter optimization in MIA analysis, other cell type matching algorithms were attempted for further test.

**RCTD Analysis**

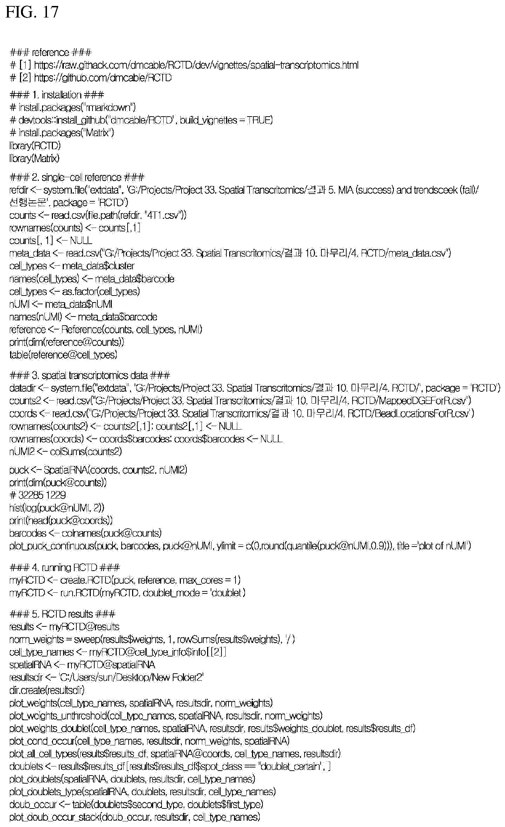

Different cell type matching algorithms were performed for test. The distribution of each cell type was determined through supervised maximum likelihood estimation using robust cell type decomposition (RCTD), a representative alternative method for MIA analysis (10). All parameters were set to default settings. For example, a parameter doublet_mode of run.RCTD was set to 'doublet' (see FIG. 17)

**CellDART Algorithm Analysis**

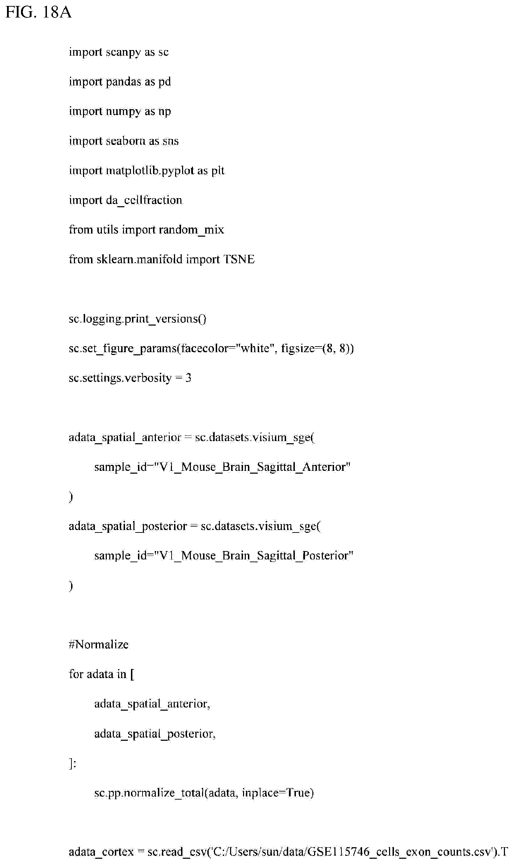

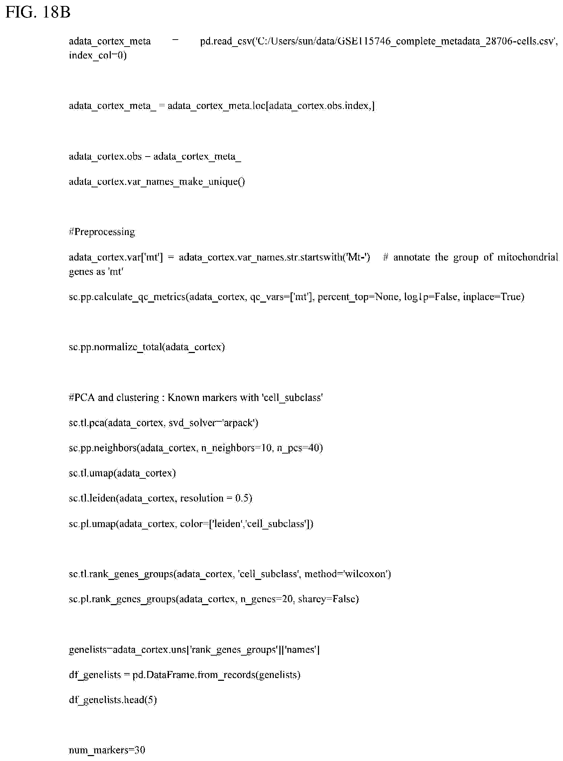

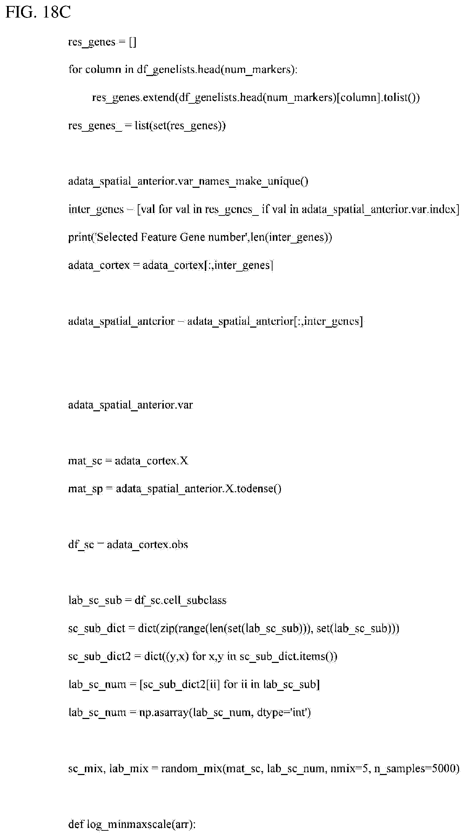

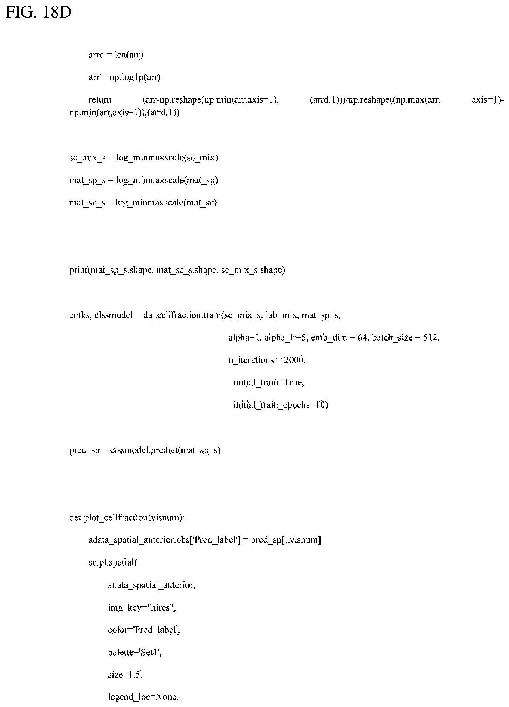

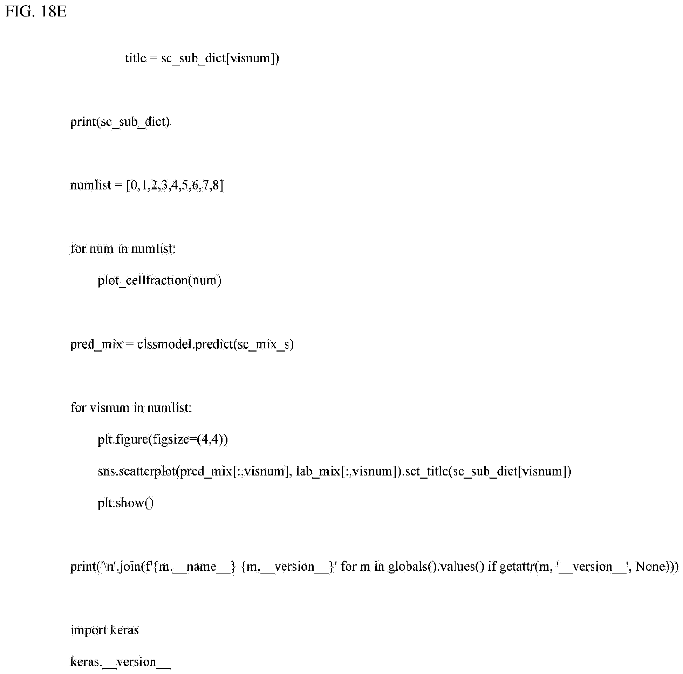

In addition, there is another cell type inference algorithm called domain adaptation of single cell and spatially reserved transcriptome data (CellDART) algorithm (22). This algorithm performed allelic domain adaptive classification with 225 selected feature genes from single cell data with pre-labeled cell types (see FIGS. 18A to 18E) (11). Although the RCTD algorithm may be used to roughly identify overall trends, the CellDART is meaningful in that it provides more sensitive results for small cell type distributions, accurately capturing subtle differences in the surrounding microenvironment and biological context.

**DEG and GO Analysis**

After investigating the characteristics of each cluster, uptake-induced genes were identified. Pearson correlation coefficient was calculated to distinguish between uptake-induced genes and site-specific genes. The fluorescent intensity value was obtained in the method as above using the imread function in Python's matplotlib.pyplot. The DEG analysis of clusters 1 and 2 was performed by comparing cluster 0 vs. cluster 1 and cluster 0 vs. cluster 2. For each DEG in a specific cluster, the fluorescent intensity was associated with the expression of the gene within the cluster. The significance level was searched based on the slope of the regression curve. Only genes with a p value of less than 0.05 were sorted according to the correlation coefficient. The generated uptake-induced genes were subjected to the gene ontology (GO) analysis in the same manner as the total fluorescence DEG analysis. In this way, a two-step approach of first identifying DEGs and then performing correlation analysis was performed. This is because unreliable genes were easily derived when only the correlation analysis was performed.

**Subgroup Fluorescence Analysis Result**

: **The fluorescence pattern of cluster 1 is related to blood-related genes or matrix genes, and the fluorescence pattern of cluster 2 is significantly related to hypoxia-induced genes.**
As described in the clustering, it was classified into cluster 1 and cluster 2, which is a method of obtaining an uptake cluster by specifically performing a series of processes including VGG16 clustering, setting the region of interest (ROI), and agglomerating the binary map into the ROI (FIG. 24A). Therefore, this was combined with the high uptake spot (FIG. 25). Meanwhile, the unsupervised hierarchical clustering according to genetic profile also generated the uptake cluster in a good manner. On the other hand, the unsupervised hierarchical clustering based on features extracted from the VGG16 could hardly distinguish between internal spots and neighboring spots. The unsupervised hierarchical clustering in all spots was able to show similar results to the t-SNE algorithm (FIG. 20D).

**MIA Analysis Result**

Cancer cells were preferentially found in cluster 2, which was consistent with the observation results in the H&E staining image (FIG. 24B). FIG. 24B shows the MIA analysis results of clusters 1 and 2, and from the results, it can be seen that cluster 2 contains many cancer cell genes. In addition, as expected, the intratumoral distribution of inflammatory macrophages and the peripheral distribution of anti-inflammatory macrophages may be observed. Meanwhile, endothelial cells or fibroblasts were predominantly located in cluster 1, which suggests that the capsular edge was related to the matrix area of the tumor (cluster 1 results in FIG. 24B).

**RTCD Result**

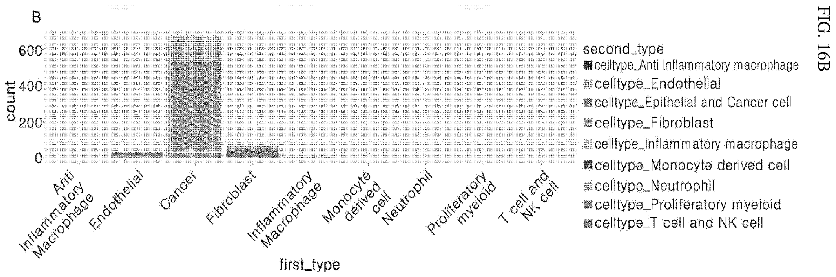

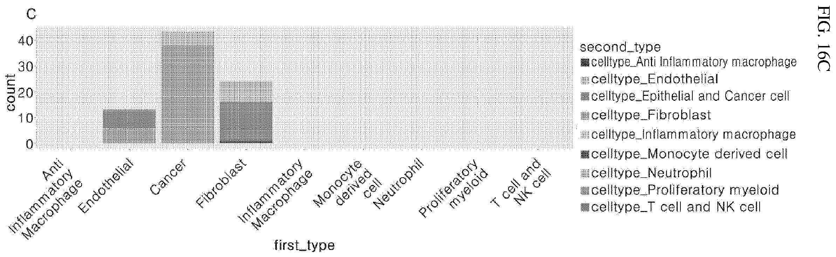

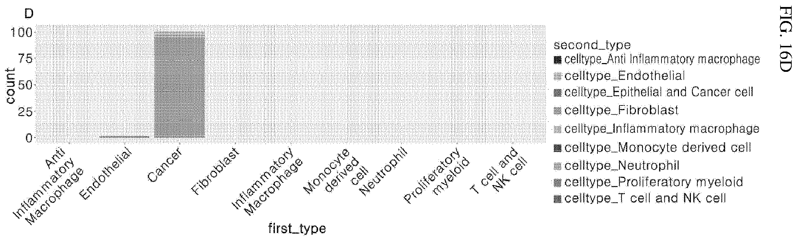

A relatively predominant distribution of endothelial cells and fibroblasts may be confirmed in cluster 1 compared to cluster 2, which was consistent with the MIA analysis (FIG. 16). FIGS. 16B to 16D illustrate results of a cell type cluster obtained from an RCTD algorithm, in which FIG. 16B is an RCTD dual line stacked bar plot illustrating results for all spots, FIG. 16C is an RCTD double distribution stacked bar plot for spots in cluster 1, and FIG. 16D is an RCTD double distribution stacked bar plot for spots in cluster 2. From the results, the relatively predominant distribution of endothelial cells and fibroblasts may be confirmed in cluster 1 compared to cluster 2, and it may be confirmed that cancer cells are predominantly present in cluster 2.

**Results of CellDART**

The results of CellDART proved the observations in the MIA analysis and RCTD (FIG. 26). According to the CellDART results, it was clearly observed that cancer cells were predominant in the tumor tissue, while endothelial cells and fibroblasts were predominant on the tumor surface. It is noted that in the CellDART, the distribution of non-dominant cell types was highlighted and the distribution of inflammatory macrophages was separated from that of cancer cells. The volcano plot of the site-specific genes showed clear differences in genetic profiles between clusters 1 and 2 (FIG. 24C). FIG. 24C is the volcano plot of the site-specific genes in cluster 1 (top) and cluster 2 (bottom), respectively.

**DEG Result**

In addition, a dot plot representing the top 20 genes for each cluster showed uniqueness of clusters 1 and 2 (FIG. 24D).

**DEG Results in Cluster 1**

Among the top 20 DEGs in cluster 1, RBC markers such as Hba-a2, Hba-a1, and Hbb-bs and matrix-related genes such as Aqp1, Col3a1, Gpx3, Apoe, and Sparcl1 may be observed (Table 3).

[Table 3]

| Order | Gene | log2FC | p_val_adj | Order | Gene | log2FC | p_val_adj |
|---|---|---|---|---|---|---|---|
| 1 | *Mpz* | 2.303707 | 1. 75E-06 | 11 | *Cc18* | 1.229044 | 3. 71E-11 |
| 2 | *Apod* | 2.156908 | 2.90E-12 | 12 | *Gpx3* | 1.222476 | 1.83E-13 |
| 3 | *Pmp22* | 1.717571 | 0.021351 | 13 | *Hba-al* | 1.216350 | 1.79E-09 |
| 4 | *Col3a1* | 1.515841 | 4.13E-14 | 14 | *Collal* | 1.211184 | 6.94E-14 |
| 5 | *Fabp4* | 1.330723 | 4.10E-12 | 15 | *Hbb-bs* | 1.204583 | 1.31E-11 |
| 6 | *Apoe* | 1.299234 | 1.40E-15 | 16 | *Pi16* | 1.183888 | 1.25E-10 |
| 7 | *Sparcl1* | 1.296022 | 1.98E-10 | 17 | *mt-Nd4* | 1.170916 | 3.20E-15 |
| 8 | *mt-Nd1* | 1.287372 | 3.58E-18 | 18 | *mt-Cytb* | 1.164417 | 1.28E-18 |
| 9 | *Hba-a2* | 1.279674 | 9.70E-06 | 19 | *Sparc* | 1.163049 | 5.99E-11 |
| 10 | *Aqp1* | 1.263791 | 2.22E-07 | 20 | *mt-Nd2* | 1.151413 | 1.17E-13 |
| **Note:** Stroma: *Mpz, Apod, Pmp22, Col3a1, Fabp4, Apoe, Sparcl1, Col1a1* Blood vessel: *Hba-a2, Hba-a1, Hbb-bs* Tumor associated macrophage: *Cc18, Gpx3* | | | | | | | |

<List of top 20 DEGs classified by FC in cluster 1 in subgroup analysis>

Therefore, it can be seen that cluster 1 is a main cause of surface-related trend formation in the total fluorescence analysis. Cluster 1 had only one gene with a significant intracluster correlation (Table 4).

[Table 4]

| Order | Gene | Correlation Coefficient | P value | Order | Gene | Correlation Coefficient | P value |
|---|---|---|---|---|---|---|---|
| 1 | *Calm1* | 0.223745 | 0.0284 24 | | | | |
| **Note:** Little to do with DEGs of cluster 1 | | | | | | | |

<DEG with significant correlation with fluorescent intensity in cluster 1>

This suggests that other factors including hemodynamics as well as gene expression, may have played a role in the uptake of nanoparticles in cluster 1. This explanation was consistent with the simulation results and observations in all DEG analyses that Hbb-bs, the only significant DEG, did not show a significant correlation between gene expression and fluorescent intensity only at the high uptake spot.

**DEG Results in Cluster 2**

Unlike cluster 1, cluster 2 showed that 16 genes showed a significant correlation, and the number of genes that were up-regulated and positively correlated with the fluorescent intensity of the cluster was 31 in total (FIG. 24E, Table 5). FIG. 24E is a plot illustrating the relationship between the correlation coefficient and p-value of cluster 1 (top) and cluster 2 (bottom).

[Table 5]

| Order | Gene | Correlation Coefficient | P value | Order | Gene | Correlation Coefficient | P value |
|---|---|---|---|---|---|---|---|
| 1 | Krt19 | 0.274451 | 0.001389 | 9 | Trib3 | 0.205230 | 0.017799 |
| 2 | Dock7 | 0.248607 | 0.003908 | 10 | Eroll | 0.200447 | 0.020702 |
| 3 | Ugdh | 0.241876 | 0.005033 | 11 | Hilpda | 0.198512 | 0.021987 |
| 4 | Gbe1 | 0.241849 | 0.005038 | 12 | Egln3 | 0.189723 | 0.028724 |
| 5 | Smarcel | 0.229166 | 0.007970 | 13 | Plin2 | 0.178111 | 0.040256 |
| 6 | Espn | 0.223523 | 0.009702 | 14 | Areg | 0.173992 | 0.045184 |
| 7 | Bnip3 | 0.209294 | 0.015617 | 15 | Gvs1 | 0.173396 | 0.045938 |
| 8 | Pfkp | 0.206807 | 0.016923 | 16 | Eno2 | 0.170759 | 0.049396 |

**Note:**

Hypoxia: *Bnip3, Ero1l, Hilpda, Egln3, Plin2*

Glycolysis: *Ugdh, Gbel1 Pfkp, Gys1, Eno2*

Apoptosis: *Bnip3, Ero1l*

Lipid Metabolism: *Hilpda, Plin2*

<DEG with significant correlation with fluorescent intensity in cluster 2>

Significantly correlated genes in cluster 2 showed three representative physiological functions in the gene ontology (GO): glucose metabolism, apoptosis, and hypoxia (FIG. 24I). Because the high-density cancer cells were mainly in cluster 2, the hypoxia conditions were highly likely to occur. Due to the hypoxia condition, cancer cells may not perform oxidative metabolism of glucose, so glycolysis becomes more active. This phenomenon is called a Warburg effect (13). In addition, previous studies evaluated patients' tumors with in vivo positron emission tomography (PET), so it showed that there is a significant correlation between the degree of hypoxia and glucose metabolism. Therefore, it is not difficult to understand that the glucose metabolism dominates under the hypoxia condition. This observation is consistent with the list of DEGs in cluster 2, where the most important glycolysis mediators such as Pfkp, Gapdh and Hk1 appeared (Table 6). When plotting the scores for the hypoxia, the glucose metabolism, and the apoptosis, the spatial distribution of the scores were all co-localized (FIG. 24K), and this distribution was similar to the internal distribution of the nanoparticles (B in FIG. 20A).

[Table 6]

| Order | Gene | log2FC | p_val_adj | Order | Gene | log2FC | p_val_adj |
|---|---|---|---|---|---|---|---|
| 1 | Ndrg1 | 0.739925 | 0.000106 | 11 | Espn | 0.458356 | 5.89E-06 |
| 2 | Slc2a1 | 0.549974 | 1.06E-06 | 12 | Ugdh | 0.456159 | 3.04E-06 |
| 3 | Rara | 0.517617 | 1.28E-06 | 13 | Id2 | 0.455084 | 0.000730 |
| 4 | Egln1 | 0.510213 | 4.32E-05 | 14 | Ftl1 | 0.453919 | 3.26E-08 |
| 5 | Sbf2 | 0.505446 | 1.47E-05 | 15 | Gapdh | 0.450410 | 5.48E-08 |
| 6 | Pdkl | 0.496558 | 1.36E-05 | 16 | Card | 0.442920 | 6.30E-06 |
| 7 | Areg | 0.488042 | 1.14E-07 | 17 | Hk1 | 0.435197 | 0.000211 |
| 8 | Bnip3 | 0.487212 | 0.000286 | is | Pafahlb3 | 0.433879 | 1.53E-05 |

(continued)

| Order | Gene | log2FC | p_val_adj | Order | Gene | log2FC | p_val_adj |
|---|---|---|---|---|---|---|---|
| 9 | *Ero1l* | 0.486502 | 0.000111 | 19 | *Eno1* | 0.432403 | 0.000202 |
| 10 | *Pfkp* | 0.475245 | 2.13E-08 | 20 | *Ppp4r3b* | 0.427087 | 0.024664 |
| **Note:** Hypoxia: *Ndrg1, Slc2a1, Egln1, Pdk1, Bnip3, Ero1l, Car9* Glycolysis: *Slc2a1, Pdk1, Pfkp, Ugdh, Gapdh, Car9, Hk1, Eno1, Ppp4r3b* Apoptosis: *Bnip3, Ero1l* Lipid Metabolism: *Ndrg1* | | | | | | | |

<List of top 20 DEGs classified by FC in cluster 2 in subgroup analysis>

Similarly, the apoptosis-related activities may be associated with the environment. The starvation-induced apoptosis may be induced due to insufficient energy production in cancer cells due to low efficiency of hypoxic metabolism. Therefore, it is highly likely that, for the group, cancer cells sought alternative energy sources, lipids, and vesicle contents, as nutritional deficiencies are an urgent challenge. Recently, the association between hypoxia and lipid metabolism has also been found. In particular, endocytosis of lipoprotein is enhanced by up-regulation of lipoprotein receptor-related protein (LRP1) (23) and very low density lipoprotein receptor (VLDLR) (24). Therefore, Ndrg1, which participates in lipid metabolism including LDL receptor trafficking, may play an important role in nanodrug uptake. Furthermore, one of the DEGs in cluster 2, Plin2 (FC = 0.311197, adjusted p val = 0.034946, cor = 0.177015, p val for cor = 0.073657), was related to hypoxia-inducible lipid-related protein along with Hif-1$\alpha$, and therefore, may be linked to the above speculation. Accordingly, similar patterns appeared in the heatmap derived from the correlation of each DEG pair with a significant correlation in cluster 2 on high-quality spots (FIG.24J). For example, set A showed overall connectivity with different elements of the set. In addition, subset A1 reflects hypoxic environment and subset A2 is related to glucose metabolic process. Interestingly, even continuity between Hilpda and Plin2 was observed as expected. However, it was observed that sets A and B were less related. Therefore, the heatmap showed that most, but not all genes, show strong connectivity that may be due to a common biological context.

### Clustering 2: Classification into Cluster 0 to Cluster 7

In the same way as clustering 1 above, a seeded region growing plug-in of imageJ program is a tool that allows a user to set an area with the same texture centered on an appropriate seed. Using this tool, a total of 7 ROIs were set and agglomerated with the binary translation map to generate 7 clusters of high fluorescence spots. All spots with low fluorescence were assigned to cluster 0 (see FIG. 27).

### DEG Analysis of Cluster 0 to Cluster 7

Each of 7 clusters consisting of high fluorescence spots was compared with cluster 0, and the same DEG analysis method used previously was performed.

### MIA Analysis of Cluster 0 to Cluster 7

To identify the cell type related to each cluster, the MIA evaluation was performed in the same manner as the MIA analysis method used previously.

### DEG Analysis Results of Cluster 0 to Cluster 7

In the DEG analysis results, most DEG values in clusters 6 and 7 all show values close to 1, showing a clear false positive signal.

Furthermore, the total fluorescence was mainly distributed in the order of clusters 0, 1, and 4. The fluorescence in cluster 0 is fluorescence having a low uptake of less than or equal to 25% of the maximum fluorescence uptake, but tends to be high because the cluster contains many spots. This may be proven by the fact that the average fluorescent intensity was lowest in cluster 0. As the plots of the average fluorescent intensity and the total fluorescent intensity showed opposite trends, it was confirmed that the number of spots within a cluster was predominantly involved in the total fluorescent intensity (FIGS. 28A and 28B, Table 7).

[Table 7]

| Cluster Number | Total Intensity | Average Intensity | Number of Spots |
|---|---|---|---|
| 0 | 26.16079 | 0.03003534 | 871 |
| 1 | 20.16078 | 0.1538991 | 131 |
| 2 | 4.643137 | 0.09286275 | 50 |
| 3 | 6.988235 | 0.1164706 | 60 |
| 4 | 10.97647 | 0.140724 | 78 |
| 5 | 3.301961 | 0.1375817 | 24 |
| 6 | 0.3921569 | 0.07843137 | 5 |
| 7 | 0.8784314 | 0.08784314 | 10 |

<Total fluorescent intensity and average fluorescent intensity of each cluster>

Looking at the average fluorescent intensity, it can be seen that clusters 1, 4, and 5 are involved in liposome uptake in that order.

The expression levels of the top 10 DEGs for each cluster were plotted through a dot plot (FIG. 29). In accordance with the fact that cluster 1 showed the highest positive mean fluorescence intensity, DEGs in cluster 1 were very similar to the results of the total fluorescence analysis. Considering the ranking of Plvap, Pecam1, and several hemoglobin genes, it can be seen that the fluorescence pattern found in cluster 1 is mainly vascular-related uptake.

In addition, as the correlation analysis result, 22 DEGs in cluster 4 and 6 DEGs in cluster 5 were confirmed to have the positive correlation with the fluorescence intensity. From the previous analysis, it can be seen that clusters 1, 4, and 5 are mainly involved in the liposome uptake. Among those, no correlation between the gene expression and the fluorescence intensity was found in cluster 1. These results show that liposome uptake occurs through passive action through blood vessels in cluster 1, but liposome uptake occurs through active action in clusters 4 and 5 inside the tumor. When the correlated genes in cluster 4 were identified, genes related to the hypoxia, the glucose metabolism, and the cell death mechanism were predominant (FIGS. 30A and 30B, Table 8).

[Table 8]

| Order | Gene | Correlation coefficient | P value | Order | Gene | Correlation coefficient | P value |
|---|---|---|---|---|---|---|---|
| 1 | Ndrgl | 0.356 | 0.001400 | 12 | Smox | 0.245 | 0.030669 |
| 2 | Phf14 | 0.339 | 0.002408 | 13 | Bnip3 | 0.243 | 0.031855 |
| 3 | Lgals3 | 0.312 | 0.005398 | 14 | Hmga2 | 0.242 | 0.033155 |
| 4 | Ier3 | 0.307 | 0.006282 | 15 | Osmr | 0.239 | 0.034944 |
| 5 | Dctn3 | 0.305 | 0.006572 | 16 | Ero1l | 0.238 | 0.035657 |
| 6 | Vdac1 | 0.285 | 0.011301 | 17 | Espn | 0.237 | 0.036748 |
| 7 | Hilpda | 0.278 | 0.013765 | 18 | Krt19 | 0.236 | 0.037135 |
| 8 | Plin2 | 0.274 | 0.015262 | 19 | Mt1 | 0.234 | 0.039351 |
| 9 | Aldoa | 0.262 | 0.020272 | 20 | Smtn | 0.231 | 0.042060 |
| 10 | Higdla | 0.245 | 0.030308 | 21 | Rara | 0.226 | 0.046298 |
| 11 | Pfkp | 0.245 | 0.030534 | 22 | Gpi1 | 0.223 | 0.049340 |

<Correlated DEGs in cluster 4>

Through this, it can be inferred that there is the hypoxic condition inside cluster 4, and thus, the anaerobic glucose metabolism process of tumor cells by the Warburg effect occurs, and at the same time, the cell death occurs due to the hypoxic condition. In addition, the fact that the lipid metabolism-related genes, such as Ndrg1 and Hilpda, show a correlation at the high level, may infer the situation within a tumor in which the tumor cells in the hypoxic condition attempt to metabolize using lipids, a component of liposomes, which are an alternative energy source.

On the other hand, in cluster 5, various immune-related genes appeared, similar to the MIA analysis. Although no specific biological mechanism was discovered as in cluster 4, the fact that the correlated genes were not only related to the immune system but also genes related to angiogenesis, endosome, lysosome, phagosome, and the like may be thought about the uptake mechanism of nanoparticles in cluster 5 (FIGS. 31A and 31B, Table 9).

[Table 9]

| Order | Gene | Correlation coefficient | P value | Order | Gene | Correlation coefficient | P value |
|---|---|---|---|---|---|---|---|
| 1 | Rap1gap | 0.577 | 0.003180 | | | | |
| 2 | Cxcl10 | 0.489 | 0.015342 | | | | |
| 3 | Irgm1 | 0.458 | 0.024323 | | | | |
| 4 | Cdk10 | 0.456 | 0.025114 | | | | |
| 5 | Sfxn5 | 0.441 | 0.031065 | | | | |
| 6 | Pigk | 0.408 | 0.048087 | | | | |

<Correlated DEGs in cluster 5>

**MIA Analysis Result of Cluster 0 to Cluster 7**

As a result of MIA, clusters 2, 3, 4, and 5 had a high proportion of cancer cells, which was consistent with the H&E staining image results (FIG. 32). In addition, many endothelial cells and fibroblast cells were found in cluster 1, which suggests that the capsule-shaped edge portion is related to a stroma portion of a tumor. Interestingly, although clusters 2 and 4 showed similar patterns of expression, cluster 5 showed a significantly different pattern. This means that different biological mechanisms are taking place depending on the area within the tumor. No obvious cell types were found in clusters 0, 6, and 7, which suggests the possibility that clusters 6 and 7 may represent false positive signals. Therefore, it can be seen that clustering containing less than or equal to 10 spots should be avoided for analysis.

**Conclusion**

It can be said that genes that determine the intratumoral microenvironment other than the passive pathway and the permeation enhancement and retention effect (EPR) govern the intratumoral uptake of fluorescent nanoparticles. This hypothesis is noteworthy because it is consistent with studies showing enhanced uptake of nanoparticles in the hypoxic environments 15 and consistent with studies showing the sequestration of lipophilic drugs in lipid droplets, which are being reexamined as organelles of active cells 16, 17, and 18.

It is found that even nutrient signaling reprogramming, such as fasting, increases macropinocytosis of drugs (19). Furthermore, it was thought that multiple pathways, including macropinocytosis, are cooperated to alleviate nutritional stress. Statistical variations found by gene expression in acid viscosity may be indicative of various factors related to uptake of drugs, not to mention gene expression itself.

The present invention was implemented by involving all interdisciplinary concepts, including image processing, AI algorithm-based genetic analysis, biological context, and flexible interpretation of complex material transfer to explore factors that determine the uptake of nanodrugs or molecular markers related to the physiological activity of nanodrugs.

The total fluorescence analysis showed that the uptake of drugs may be affected by blood fluid dynamics, and the subgroup fluorescence analysis showed that in clustering 1, the fluorescence pattern in cluster 1 was related to blood-related genes or matrix genes, and the fluorescence pattern in cluster 2 is significantly correlated with the hypoxia-induced genes. It can be seen that, in clustering 2, no obvious cell types were found in clusters 0, 6, and 7, which suggests the possibility that clusters 6 and 7 may represent false positive signals, clusters 1, 4, and 5 are mainly related to liposome uptake, in cluster 1, liposome uptake occurs through passive action through blood vessels, but in clusters 4 and 5, which are inside the tumor, liposome uptake occurs through active action, and in cluster 4, genes related to hypoxia, glucose metabolism, and cell death mechanisms were predominant.

The methods disclosed herein may be utilized to identify new molecular pathways for drugs not only in solid tumors and nano drugs, but also in various tissues and other forms, and to explore strategies for unmet diseases.

**Claims**

1.   An analysis apparatus (100), comprising:

an information receiving unit (110) configured to receive a tissue image of a tissue of interest provided with an exploring material with which a labeling material for analysis is bonded and transcriptome information sharing spatial information of the tissue of interest;
a spatial mapping unit (120) configured to generate spatial mapping information by spatially mapping the tissue image by the labeling material of the exploring material with the spatially reserved transcriptome information; a transcriptome information extraction unit (150) configured to extract the transcriptome information in the tissue relating to distribution of the exploring material from the spatial mapping information; and a molecular marker analysis unit (160) configured to analyze a molecular marker relating to the distribution of the exploring material in the tissue of interest through the extraction.

2. The analysis apparatus (100) of claim 1, wherein the exploring material is provided to the tissue of interest by being directly provided to the tissue of interest or administered to a subject by systemic administration and then distributed to the tissue of interest.

3. The analysis apparatus (100) of claim 1, wherein the transcriptome information extraction unit (150) includes at least one of:

an image feature analysis unit (152) configured to extract the transcriptome information using an image feature extraction algorithm using artificial intelligence; a correlation analysis unit (154) configured to extract the transcriptome information by analyzing a correlation between image intensity and a gene expression level of the labeling material in the tissue; a cell type analysis unit (156) configured to use a cell type analysis algorithm; and a gene ontology analysis unit (157) configured to use gene ontology analysis.

4. The analysis apparatus (100) of claim 3, wherein the image feature extraction algorithm using artificial intelligence is spatial gene expression patterns by deep learning of tissue images (SPADE) algorithm.

5. The analysis apparatus (100) of claim 3, wherein the correlation analysis unit (154) uses differentially expressed genes (DEG), correlation analysis by calculating a correlation coefficient, or an image similarity evaluation algorithm.

6. The analysis apparatus (100) of claim 5, wherein the correlation coefficient calculation is a Pearson correlation coefficient, Spearman correlation coefficient, or Kendall correlation coefficient calculations.

7. The analysis apparatus (100) of claim 3, wherein the cell type analysis unit (156) uses Fisher's exact test, maximum likelihood estimation, domain adaptive classification, logistic regression analysis, or negative binomial regression analysis algorithm.

8. The analysis apparatus (100) of claim 7, wherein the cell type analysis algorithm is MIA, CellDART algorithm, RCTD algorithm, celltypist or cell2location.

9. The analysis apparatus (100) of claim 1, further comprising a clustering unit (140) configured to partition the tissue image by the labeling material into one or more clusters before or after the spatial mapping, wherein the spatially mapped information includes spatially mapped information of one or more clusters.

10. The analysis apparatus (100) of claim 9, wherein the clustering unit 140 classifies the one or more clusters based on the image intensity by the labeling material, or splits the tissue image by the labeling material of the exploring material into a plurality of patches and classifies the tissue image into one or more clusters by an algorithm that performs the classification based on a similarity of image features for each patch.

11. The analysis apparatus (100) of claim 9, wherein the spatially mapped information of the cluster is information obtained using an image feature extraction algorithm of individual clusters, correlation analysis between image intensity and a gene expression level of the cluster, a cell type analysis algorithm, and/or gene ontology analysis.

12. The analysis apparatus (100) of any one of claims 1 to 11, further comprising an image transformation unit (130) configured to apply the tissue image by labeling material to an algorithm, which performs translation transformation, rotation transformation, or topological transformation, in the spatial mapping to generate a registered image.

13. The analysis apparatus (100) of claim 12, wherein the image transformation unit (130) generates the registered image using an algorithm provided by a Python-based open-source DiPY package or a bUnwarpJ module in imageJ.

14. The analysis apparatus (100) of claim 12, wherein the image transformation unit (130) additionally transforms the registered image to generate a transformed image that effectively performs the spatial mapping.

15. The analysis apparatus (100) of claim 14, wherein the transformed image is an image in which an image signal is discontinuously transformed according to the image intensity.

16. The analysis apparatus (100) of any one of claims 1 to 11, wherein the spatially reserved transcriptome information in the spatial mapping is information in which a program that visualizes a spot according to a genetic feature is used or a spot with less than the certain number of RNA reads is excluded.

17. The analysis apparatus (100) of any one of claims 1 to 11, wherein an information receiving unit (1100) additionally receives the tissue image in which the tissue of interest is stained.

18. The analysis apparatus (100) of any one of claims 1 to 11, wherein the spatial mapping unit (120) spatially matches coordinates of a spot of the spatially reserved transcriptome information arranged in a two-dimensional plane with coordinates of a pixel in the tissue image by the labeling material of the exploring material arranged in the corresponding two-dimensional plane for the spatial mapping.

19. The analysis apparatus (100) of any one of claims 1 to 11, wherein the spatially reserved transcriptome information is the number of transcriptome RNA reads and an RNA type in a spot having spatial coordinates in the tissue of interest.

20. The analysis apparatus (100) of any one of claims 1 to 11, wherein the labeling material for the analysis is a radioactive material, a fluorescent material, a pigment material, or a luminescent material.

21. The analysis apparatus (100) of any one of claims 1 to 11, wherein the molecular marker is a single molecule derived from DNA, RNA, metabolite, protein, protein fragment, etc., or molecular information based on patterns thereof.

22. The analysis apparatus (100) of any one of claims 1 to 11, wherein the exploring material is selected from the group consisting of a nanomaterial, a low molecular compound, a high molecular compound, a natural product, aptamer, a nanobody, a microorganism, an antibody, an engineering antibody, an antibody-drug conjugate, an extracellular vesicle, a cell, peptide, nucleic acid, protein, amino acid, sugar, lipid, biopharmaceutical or biocandidate, a synthetic chemical drug or a synthetic chemical candidate, and a natural drug product or a natural product candidate.

23. The analysis apparatus (100) of claim 22, wherein the nanomaterial is a polymer nanoparticle, a lipid-based nanoparticle, a polymer double-layer structure nanoparticle, a protein nanoparticle, an inorganic nanoparticle, or a crystalline nanoparticle, and the biopharmaceutical or biocandidate is a biological agent, a genetically recombinant medicine, a cell culture medicine, a biosimilar, a biobetter, an advanced biopharmaceutical, or a candidate thereof.

24. A method of analyzing a molecular marker in a tissue of interest relating to distribution of an exploring material in a tissue, wherein the method is performed in the analysis apparatus (100) of any one of claims 1 to 11.

25. A computer program stored on a computer-readable recording medium for executing the analysis method of claim 24.

26. An analysis apparatus (100), comprising:

an information receiving unit (110) configured to receive a tissue image of a tissue of interest provided with a first exploring material with which a first labeling material for analysis is bonded and transcriptome information sharing spatial information of the tissue of interest;
a spatial mapping unit (120) configured to generate spatial mapping information by spatially mapping the tissue image by the labeling material of the exploring material with the spatially reserved transcriptome information; and
a physiological activity information analysis unit (170) configured to analyze physiological activity information in a tissue of interest relating to distribution or action of the first exploring material.

27. The analysis apparatus (100) of claim 26, wherein the first exploring material is provided to the tissue of interest by being directly provided to the tissue of interest or administered to a subject by systemic administration and then distributed to the tissue of interest.

28. The analysis apparatus (100) of claim 26, wherein the physiological activity information in the tissue of interest is information on a material that affects a function or physiology of a living body.

29. The analysis apparatus (100) of claim 26, wherein the information receiving unit (110) receives an image of the tissue of interest by a second labeling material of a second exploring material with which the second labeling material is bonded, and
the spatial mapping unit (120) spatially maps the image of the tissue of interest by the labeling material of the second exploring material with which the second labeling material is bonded to spatially reserved transcriptome information of the tissue of interest to generate spatial mapping information.

30. The analysis apparatus (100) of claim 29, wherein the physiological activity information analysis unit (170) analyzes the physiological activity information of the first exploring material by comparing and analyzing the spatial mapping information of the second exploring material.

31. The analysis apparatus (100) of any one of claims 26 to 30, wherein the first exploring material or the second exploring material each is independently selected from the group consisting of a nanomaterial, a low molecular compound, a high molecular compound, a natural product, aptamer, a nanobody, a microorganism, an antibody, an engineering antibody, an antibody-drug conjugate, an extracellular vesicle, a cell, peptide, nucleic acid, protein, amino acid, sugar, lipid, biopharmaceutical or biocandidate, a synthetic chemical drug or a synthetic chemical candidate, and a natural product medicine or a natural product candidate.

32. A method of analyzing physiological activity information in a tissue of interest relating to physiological activity of an exploring material, wherein the method is performed in the analysis apparatus (100) of any one of claims 26 to 30.

33. A computer program stored on a computer-readable recording medium for executing the analysis method of claim 32.

34. An analysis apparatus (100), comprising:

an information receiving unit (110) configured to receive a tissue image of a tissue of interest provided with an exploring material with which a labeling material for analysis is bonded and transcriptome information sharing spatial information of the tissue of interest;
a spatial mapping unit (120) configured to generate spatial mapping information by spatially mapping the tissue image by the labeling material of the exploring material with the spatially reserved transcriptome information;
a transcriptome information extraction unit (150) configured to extract the transcriptome information in the tissue relating to distribution of the exploring material from the spatial mapping information; and
a physiological activity information analysis unit (170) configured to analyze physiological activity information of the exploring material in the tissue of interest,
wherein the spatial mapping information includes spatially mapped information of one or more clusters, and

the transcriptome extraction unit (150) extracts the transcriptome information of the corresponding cluster from the spatially mapped information of each cluster.

35. The analysis apparatus (100) of claim 34, wherein the exploring material is provided to the tissue of interest by being directly provided to the tissue of interest or administered to a subject by systemic administration and then distributed to the tissue of interest.

36. The analysis apparatus (100) of claim 34, wherein the one or more clusters are classified using image intensity by the labeling material, or the image by the labeling material of the exploring material is split into a plurality of patches and is classified by a similarity of image features for each patch.

37. The analysis apparatus (100) of claim 36, wherein the classification by the similarity of the image features for each patch is performed by clustering algorithms of K-means clustering, unsupervised hierarchical clustering, stochastic neighbor embedding, agglomerative clustering, spectral clustering, or Gaussian mixture.

38. The analysis apparatus (100) of any one of claims 34 to 37, wherein the transcriptome information extraction unit (150) extracts the transcriptome information of the corresponding cluster using an image feature extraction algorithm of individual clusters, correlation analysis between image intensity and a gene expression level of the cluster, a cell type analysis algorithm, and/or gene ontology analysis.

39. The analysis apparatus (100) of any one of claims 34 to 37, wherein the physiological activity information in the tissue of interest is molecular information based on a single molecule derived from a compound, DNA, RNA, metabolite, protein, protein fragment, etc., which affect the distribution or action of the exploring material, and a pattern thereof, or information on a material which affect a function or physiology of a living body.

40. A method of analyzing physiological activity information in a tissue of interest relating to physiological activity of an exploring material, wherein the method is performed in the analysis apparatus (100) of any one of claims 34 to 37.

41. A computer program stored on a computer-readable recording medium for executing the analysis method of claim 40.

FIG. 1

FIG. 2

FIG. 3

150

Image feature
analysis unit ～152

Correlation
analysis unit ～154

Cell type
analysis unit ～156

Gene ontology
analysis unit ～157

FIG. 4

100

Information receiving unit ～110

Space mapping unit ～120

Clustering unit ～140

Transcriptome information
extraction unit ～150

Physiological activity
information analysis unit ～170

FIG. 5

```
┌─────────────────────────────────────────────┐
│   Receive tissue image by labeling material  │──S10
└─────────────────────────────────────────────┘
                        │
                        ▼
┌─────────────────────────────────────────────┐
│        Receive transcriptome information     │──S20
│  sharing spatial information of tissue of interest │
└─────────────────────────────────────────────┘
                        │
                        ▼
┌─────────────────────────────────────────────┐
│         Generate spatial mapping information │
│          by spatial mapping tissue image with│──S30
│      spatially reserved transcriptome information │
└─────────────────────────────────────────────┘
                        │
                        ▼
┌─────────────────────────────────────────────┐
│     Extract transcriptome information in tissue │──S40
└─────────────────────────────────────────────┘
                        │
                        ▼
┌─────────────────────────────────────────────┐
│         Analyze molecular marker describing  │──S50
│    distribution in tissue of exploring material │
└─────────────────────────────────────────────┘
```

FIG. 6

Receive tissue image by labeling material (S10)

```
┌──────────────┐     ┌──────────────┐     ┌──────────────┐     ┌──────────────┐
│   Provide    │     │              │     │              │     │Acquire image │
│  exploring   │     │  Establish   │     │   Provide    │     │ of exploring │
│material with │  ⇒  │ animal model │  ⇒  │  exploring   │  ⇒  │ material by  │
│which labeling│     │for disease of│     │ material to  │     │  labeling    │
│ material is  │     │   interest   │     │ animal model │     │ material of  │
│   coupled    │     │              │     │              │     │ animal model │
└──────────────┘     └──────────────┘     └──────────────┘     └──────────────┘
```

FIG. 7

**Receive transcriptome information sharing spatial information of tissue of interest(S20)**

FIG. 8

Extract transcriptome information in tissue (S40)

| Image feature extract algorithm (ex. SPADE) | Analyze correlation between labeling material intensity and gene expression level (ex. DEG) | Cell type analysis algorithm (ex. CellDART, MIA, RCTD) | Gene ontology analysis |
|---|---|---|---|

FIG. 9

Analyze molecular marker (S50)

| Quantitatively analyze cell type relating to distribution of exploring material | Analyze molecular marker enhancing/ inhibiting distribution of exploring material | Analyze cell type relating to physiological activity of exploring material | Analyze molecular marker relating to physiological activity of exploring material |
|---|---|---|---|

FIG. 10

② Acquire data

Method of analyzing molecular marker relating to heterogeneous distribution of drug

① Intravenous injection of fluorescent dye labeled drug into mouse tumor model

Fluorescence image

Spatially reserved transcriptome

③ Fluorescence image signal / spatial normalization

④ Clustering

⑤ DEG

Gene ontology

Correlation analysis

⑥ SPADE

⑦ Molecular markers dominating drug distribution in cancer tissue

DEG: differentially expressed gene
SPADE: spatial gene expression patterns by deep learning of tissue images

EP 4 362 027 A1

33

FIG. 11

<table>
<tr><td colspan="3">Step 1: Modify fluorescence image<br><br>Python-based open-source dipy package<br><br></td></tr>
<tr><td colspan="3">Step 2: Modify spatially reserved transcriptome library<br><br>All spots with RNA reads less than 500 were ignored.<br>Under this condition, all spots had less than 25% mitochondria<br>and less than 20% hemoglobin.<br><br></td></tr>
</table>

Step 3: Extract marker genes

| | DEG Analysis | SPADE Algorithm |
|---|---|---|
| # of steps | 3; High uptake spot arrangement → DEG Analysis → Correlation analysis | 1; SPADE Algorithm |
| Spot value | Max, mean + 2α, mean+α, mean, median, min of fluorescent intensity of each spot | Principal component of features extracted from VGG 16 |
| Correlation | Pearson correlation coefficient | Empirical Bayes algorithm and linear regression analysis for SPADE genes |
| P-value | Wilcoxon rank sum test for site-specific genes; linear regression analysis for uptake-specific genes | Empirical Bayes algorithm and linear regression analysis for SPADE genes |
| Note | | Ambiguity between site -Specific and uptake -Specific genes |

Step 4: Acquire uptake cluster

| | Combination of VGG16 and K-average clustering | Unsupervised hierarchical clustering |
|---|---|---|
| Mechanism | Texture recognition | Genetic consistency |
| # of steps | 3; Acquire of uptake cluster byhigh uptake spot arrangement → VGG16 and K-average clustering → Agglomeration | 2; Acquisition of uptake cluster by high uptake spot arrangement → Hierarchical clustering |

FIG. 12

SPADE Algorithm

1Patch = 5x5 Size

Pattern     Abstracted Form

394x384 Patches

VGG16

Convolutional     512
Neural Network   features

PCA
(Merge)

512
PCs

*PCA: principal components analysis
*PC: principal components

FIG. 13

$d = 0$     $d = 1$     $d = 2$     $d = 3$     $d = 4$

FIG. 14A

```
#!/usr/bin/env python3

# -*- coding: utf-8 -*-

"""

Created on Thu Jan 30 23:54:17 2020


@author: user
"""

import os

import numpy as np

import matplotlib.pyplot as plt

from skimage import draw

import pandas as pd

import argparse

os.environ['KERAS_BACKEND'] = 'tensorflow'

from keras import backend as K

K.set_image_data_format='channels_last'

from keras.applications import vgg16

from keras import backend as K

from sklearn.manifold import TSNE

from sklearn.decomposition import PCA


#Image Show

def spatial_featuremap(t_features, img, pd_coord_tissue, imscale, radius = 10, posonly=True):

    tsimg = np.zeros(img.shape[:2])

    tsimg_row = np.array(round(pd_coord_tissue.loc[:,'imgrow']*imscale), dtype=int)

    tsimg_col = np.array(round(pd_coord_tissue.loc[:,'imgcol']*imscale), dtype=int)

    for rr, cc,t in zip(tsimg_row, tsimg_col,t_features):

        r, c = draw.circle(rr, cc, radius = 10)
```

FIG. 14B

```
        if posonly:
                if t>0:
                        tsimg[r,c]⁻ t
                else:
                        tsimg[r,c]=t
        return tsimg


if __name__ == "__main__":
    parser = argparse.ArgumentParser()
    parser.add_argument('--patchsize', type=int, default=32)
    parser.add_argument('--position', type=str)
    parser.add_argument('--image', type=str)
    parser.add_argument('--scale', type=float)
    parser.add_argument('--meta', type=str)
    parser.add_argument('--outdir', type=str, default='./SPADE_output/')
    parser.add_argument('--numpcs', type=int, default= 2)
    args = parser.parse_args()


    if not os.path.exists(args.outdir):
        os.makedirs(args.outdir)


    #Param
    sz_patch ⁻ args.patchsize


    br_coord ⁻ pd.read_csv(args.position,
                                header=None, names= ['barcodes','tissue','row','col','imgrow','imgcol'])
    br_meta = pd.read_csv(args.meta)
    if 'seurat_clusters' not in br_meta.columns:
        print("Warning: meta data including scruat_clusters show t-SNE map of image features with clustering
info")
```

# FIG. 14C

```
else:

    print('Meta data is loaded')

br_meta_coord = pd.merge(br_meta, br_coord, how = 'inner', right_on ='barcodes' , left_on='Unnamed: 0')


brimg = plt.imread(args.image)

print('Input image dimension:', brimg.shape)


brscale = args.scale

br_coord_tissue = br_meta_coord.loc[br_meta_coord.tissue==1,:]


#Image Patch

tsimg_row = np.array(round(br_coord_tissue.loc[:,'imgrow']*brscale), dtype=int)

tsimg_col = np.array(round(br_coord_tissue.loc[:,'imgcol']*brscale), dtype=int)


tspatches = []

sz = int(sz_patch/2)

for rr, cc in zip(tsimg_row, tsimg_col):

    tspatches.append(brimg[rr-sz:rr+sz, cc-sz:cc+sz])

tspatches = np.asarray(tspatches)

print('Image to Patches done', '....patchsize is ', sz_patch, ' .... number of patches ', tspatches.shape[0])


#pretrained model

pretrained_model = vgg16.VGG16(weights='imagenet', include_top = False, pooling='avg', input_shape = (32,32,3))

X_in = tspatches.copy()

X_in = vgg16.preprocess_input(X_in)

pretrained_model.trainable = False

print('Architecture of CNN model')

pretrained_model.summary()
```

## FIG. 14D

```
if 'seurat_clusters' in br_meta.columns:

    Y = np.asarray(br_meta['seurat_clusters'])


#feature extraction

ts_features = pretrained_model.predict(X_in)

print('Image features extracted.')

ts_tsne = TSNE(n_components=2, init='pca',perplexity=30,random_state=10).fit_transform(ts_features)

print('t-SNE for image features ... done')


plt.figure(figsize=(8, 7))

if 'seurat_clusters' in br_meta.columns:

    plt.scatter(ts_tsne[:, 0], ts_tsne[:, 1], c=Y, cmap='Dark2' , s = 5, alpha=0.5)

else:

    plt.scatter(ts_tsne[:, 0], ts_tsne[:, 1], s = 5, alpha=0.5)

plt.colorbar()

plt.xlabel("z[0]")

plt.ylabel("z[1]")

plt.title('t-SNE for image features (VGG16 Output)')

plt.savefig(args.outdir+'/SPADE_image_features.png', dpi=300)

print('t-SNE map is saved')


#PCA

numpcs = args.numpcs

pca = PCA(n_components=numpcs)

pca.fit(ts_features)

ts_pca = pca.transform(ts_features)

print('PCs of image features are extracted')

#

#plt.figure(figsize=(8, 7))
```

FIG. 14E

```
#plt.scatter(ts_pca[:, 0], ts_pca[:, 1], c=Y, cmap='Dark2' , s = 5, alpha=0.5)

#plt.colorbar()

#plt.xlabel("z[0]")

#plt.ylabel("z[1]")


for ii in range(numpcs):

    tsimg = spatial_featuremap(ts_pca[:,ii], brimg, br_coord_tissue, brscale, posonly=False)

    plt.figure(figsize=(10,10))

    plt.imshow(brimg)

    plt.imshow(tsimg, alpha=0.7, cmap='bwr', vmin = -1.0, vmax=1.0)

    plt.savefig(args.outdir+'/SPADE_pc'+str(ii+1)+'.png', dpi=300)


pd_ts_pca = pd.DataFrame(ts_pca, index = br_meta_coord.barcodes)

pd_ts_pca.to_csv(args.outdir+'/ts_features_pc.csv')

print('PCs of image features are saved')
```

## FIG. 14F

```
[Supplementary Material 2_R]

library(reticulate)

use_condaenv(condaenv = 'Tf', required = TRUE)    # Appropriate System Environment to run tensorflow/keras

py_config()


library(Seurat)

library(SeuratData)

library(ggplot2)

library(cowplot)

library(dplyr)


#col.names = c('barcodes', 'tissue', 'row', 'col', 'imagerow', 'imagecol'),

br.sp = Load10X_Spatial('./BreastCa_10x',

                          slice= 'slice1')

plot1 <- VlnPlot(br.sp, features = "nCount_Spatial", pt.size = 0.1) + NoLegend()

plot2 <- SpatialFeaturePlot(br.sp, features = "nCount_Spatial") + theme(legend.position = "right")

plot_grid(plot1, plot2)


br.sp <- SCTransform(br.sp, assay = "Spatial", verbose = FALSE,

                          variable.features.n = 1000)


SpatialFeaturePlot(br.sp, features = c("CD8A", "CD68","EPCAM","SLC2A1",'SLC2A3'),

                          alpha=c(0.5,0.9))

SpatialFeaturePlot(br.sp, features = NA,

                          alpha=c(0.0,1.5))


br.sp <- RunPCA(br.sp, assay = "SCT", verbose = FALSE)

br.sp <- FindNeighbors(br.sp, reduction = "pca", dims = 1:30)

br.sp <- FindClusters(br.sp, verbose = FALSE)
```

# FIG. 14G

```
br.sp <- RunTSNE(br.sp, reduction = "pca", dims = 1:30)


p1 <- DimPlot(br.sp, reduction = "tsne", label = TRUE)

p2 <- SpatialDimPlot(br.sp, label = TRUE, label.size = 3, alpha=c(0.1,0.1))

plot_grid(p1, p2)


LinkedDimPlot(br.sp)


#To FILE for SPADE

write.csv(br.sp@meta.data, 'br_metadata.csv')


#RUN SPADE

system("python                        spade_spatial_marker_by_deeplearning.py            --
position ./BreastCa_10x/spatial/tissue_positions_list.csv --image ./BreastCa_10x/spatial/tissue_hires_image.png
--scale 0.08250825 --meta br_metadata.csv --outdir Breast_SPADE --numpcs 6")


#Use SPADE result

br.rep = read.csv('./Breast_SPADE/ts_features_pc.csv', row.names=1)

colnames(br.rep) = paste('ImageLatent',1:dim(br.rep)[2], sep='_')

br.rep=as.data.frame(br.rep)

br.sp.z = AddMetaData(br.sp, br.rep )


FeatureScatter(br.sp.z , 'ImageLatent_1', 'ImageLatent_2')

SpatialFeaturePlot(br.sp.z, features = "ImageLatent_2", alpha=c(0.8, 0.7),

                          min.cutoff=-1, max.cutoff=1)


#Image latents and related markers using limma

library(limma)

design =   model.matrix(~ 1 + br.sp.z@meta.data$ImageLatent_1 + br.sp.z@meta.data$ImageLatent_2)
```

FIG. 14H

```
colnames(design) = c('Intercept','PC1','PC2')


br.sp.z = ScaleData(br.sp.z, features=rownames(br.sp.z))

yy =br.sp.z@assays$SCT@scale.data

fit = lmFit(yy, design) # fit linear model

contrast_matrix = makeContrasts(contrasts = "PC1", levels=design)

fit.cont = contrasts.fit(fit, contrast_matrix)

fitE= eBayes(fit.cont) # Bayes

tested = topTable(fitE, adjust = "fdr", number = 100, sort.by='p')

head(tested, n =10)


SpatialFeaturePlot(br.sp.z, features = rownames(head(tested,6)), alpha=c(0.8, 0.7))


contrast_matrix = makeContrasts(contrasts = "PC2", levels=design)

fit.cont = contrasts.fit(fit, contrast_matrix)

fitE= eBayes(fit.cont) # Bayes

tested = topTable(fitE, adjust = "fdr", number = 100, sort.by='p')

head(tested, n =10)

SpatialFeaturePlot(br.sp.z, features = rownames(head(tested,6)), alpha=c(0.8, 0.7))
```

FIG. 14I

```
else:

    print('Meta data is loaded')

br_meta_coord = pd.merge(br_meta, br_coord, how = 'inner', right_on ='barcodes' , left_on='Unnamed: 0')


brimg = plt.imread(args.image)

print('Input image dimension:', brimg.shape)


brscale = args.scale

br_coord_tissue = br_meta_coord.loc[br_meta_coord.tissue==1,:]


#Image Patch

tsimg_row = np.array(round(br_coord_tissue.loc[:,'imgrow']*brscale), dtype=int)

tsimg_col = np.array(round(br_coord_tissue.loc[:,'imgcol']*brscale), dtype=int)


tspatches = []

sz = int(sz_patch/2)

for rr, cc in zip(tsimg_row, tsimg_col):

    tspatches.append(brimg[rr-sz:rr+sz, cc-sz:cc+sz])

tspatches = np.asarray(tspatches)

print('Image to Patches done', '....patchsize is ', sz_patch, ' .... number of patches ' , tspatches.shape[0])


#pretrained model

    pretrained_model = vgg16.VGG16(weights='imagenet', include_top = False, pooling='avg', input_shape =
(32,32,3))

    X_in = tspatches.copy()

    X_in = vgg16.preprocess_input(X_in)

    pretrained_model.trainable = False

    print('Architecture of CNN model')

    pretrained_model.summary()
```

## FIG. 14J

```
if 'seurat_clusters' in br_meta.columns:

    Y = np.asarray(br_meta['seurat_clusters'])


#feature extraction

ts_features = pretrained_model.predict(X_in)

print('Image features extracted.')

ts_tsne = TSNE(n_components=2, init='pca',perplexity=30,random_state=10).fit_transform(ts_features)

print('t-SNE for image features ... done')


plt.figure(figsize=(8, 7))

if 'seurat_clusters' in br_meta.columns:

    plt.scatter(ts_tsne[:, 0], ts_tsne[:, 1], c=Y, cmap='Dark2', s = 5, alpha=0.5)

else:

    plt.scatter(ts_tsne[:, 0], ts_tsne[:, 1], s = 5, alpha=0.5)

plt.colorbar()

plt.xlabel("z[0]")

plt.ylabel("z[1]")

plt.title('t-SNE for image features (VGG16 Output)')

plt.savefig(args.outdir+'/SPADE_image_features.png', dpi=300)

print('t-SNE map is saved')


#PCA

numpcs = args.numpcs

pca = PCA(n_components=numpcs)

pca.fit(ts_features)

ts_pca = pca.transform(ts_features)

print('PCs of image features are extracted')

#

#plt.figure(figsize=(8, 7))
```

FIG. 14K

```
#plt.scatter(ts_pca[:, 0], ts_pca[:, 1], c=Y, cmap='Dark2' , s = 5, alpha=0.5)

#plt.colorbar()

#plt.xlabel("z[0]")

#plt.ylabel("z[1]")


for ii in range(numpcs):

    tsimg = spatial_featuremap(ts_pca[:,ii], brimg, br_coord_tissue, brscale, posonly=False)

    plt.figure(figsize=(10,10))

    plt.imshow(brimg)

    plt.imshow(tsimg, alpha=0.7, cmap='bwr', vmin = -1.0, vmax=1.0)

    plt.savefig(args.outdir+'/SPADE_pc'+str(ii+1)+'.png', dpi=300)


pd_ts_pca = pd.DataFrame(ts_pca, index = br_meta_coord.barcodes)

pd_ts_pca.to_csv(args.outdir+'/ts_features_pc.csv')

print('PCs of image features are saved')
```

FIG. 15A

```
import cv2

import matplotlib.pyplot as plt

from skimage.util import view_as_windows

import keras

from keras import backend as K

K.set_image_data_format='channels_last'

from keras.applications import resnet50

import numpy as np


img_liposome = cv2.imread('./liposome_only_image.png')


plt.imshow(img_liposome)


sz_ = 32

step_ = 5

patches_whole = view_as_windows(img_liposome, window_shape= (sz_, sz_,3), step = step_)

print(patches_whole.shape)


patches_whole_ = patches_whole.reshape(patches_whole.shape[0]*patches_whole.shape[1],sz_,sz_,3)


pretrained_model = resnet50.ResNet50(weights='imagenet', include_top = False, pooling='avg', input_shape =
(sz_,sz_,3))

output_res = pretrained_model.output

pmodel = keras.Model(pretrained_model.input, output_res)


print(np.max(img_liposome), np.min(img_liposome))


X_in = resnet50.preprocess_input(patches_whole_)
```

FIG. 15B

```
outfeatures = pmodel.predict(X_in)


print(outfeatures.shape)


#Kmeans

from sklearn.cluster import KMeans

kmeans = KMeans(n_clusters=3, random_state=1).fit(outfeatures)

outlabel = kmeans.labels_

outlabel_ = outlabel.reshape(patches_whole.shape[0],patches_whole.shape[1], 1 )


#plt.imshow(outlabel_, cmap='Dark2')

plt.imshow(outlabel_)

# plt.colorbar()

plt.savefig('image_based_clustering2.png', dpi = 300)


outlabel_re      =      cv2.resize(outlabel_,      dsize=(img_liposome.shape[1],img_liposome.shape[0]),
interpolation=cv2.INTER_NEAREST )


plt.imshow(img_liposome)

plt.imshow(outlabel_re,alpha=0.5, cmap='Dark2')


cv2.imwrite('image_based_clustering.png', outlabel_re)
```

FIG. 16A

FIG. 16B

FIG. 16C

FIG. 16D

## FIG. 17

```
### reference ###
# [1] https://raw.githack.com/dmcable/RCTD/dev/vignettes/spatial-transcriptomics.html
# [2] https://github.com/dmcable/RCTD

### 1. installation ###
# install.packages("rmarkdown")
# devtools::install_github("dmcable/RCTD", build_vignettes = TRUE)
# install.packages("Matrix")
library(RCTD)
library(Matrix)

### 2. single-cell reference ###
refdir <- system.file("extdata", 'G:/Projects/Project 33. Spatial Transcritomics/결과 5. MIA (success) and trendsceek (fail)/
선행논문', package = 'RCTD')
counts <- read.csv(file.path(refdir. "4T1.csv"))
rownames(counts) <- counts[,1]
counts[, 1] <- NULL
meta_data <- read.csv("G:/Projects/Project 33. Spatial Transcritomics/결과 10. 마무리/4. RCTD/meta_data.csv")
cell_types <- meta_data$cluster
names(cell_types) <- meta_data$barcode
cell_types <- as.factor(cell_types)
nUMI <- meta_data$nUMI
names(nUMI) <- meta_data$barcode
reference <- Reference(counts, cell_types, nUMI)
print(dim(reference@counts))
table(reference@cell_types)

### 3. spatial transcriptomics data ###
datadir <- system.file("extdata", 'G:/Projects/Project 33. Spatial Transcritomics/결과 10. 마무리/4. RCTD/', package = 'RCTD')
counts2 <- read.csv("G:/Projects/Project 33. Spatial Transcritomics/결과 10. 마무리/4. RCTD/MappedDGEForR.csv")
coords <- read.csv("G:/Projects/Project 33. Spatial Transcritomics/결과 10. 마무리/4. RCTD/BeadLocationsForR.csv")
rownames(counts2) <- counts2[,1]; counts2[,1] <- NULL
rownames(coords) <- coords$barcodes; coords$barcodes <- NULL
nUMI2 <- colSums(counts2)

puck <- SpatialRNA(coords. counts2. nUMI2)
print(dim(puck@counts))
# 32285 1229
hist(log(puck@nUMI, 2))
print(head(puck@coords))
barcodes <- colnames(puck@counts)
plot_puck_continuous(puck, barcodes, puck@nUMI, ylimit = c(0,round(quantile(puck@nUMI,0.9))), title ='plot of nUMI')

### 4. running RCTD ###
myRCTD <- create.RCTD(puck, reference, max_cores = 1)
myRCTD <- run.RCTD(myRCTD, doublet_mode = 'doublet')

### 5. RCTD results ###
results <- myRCTD@results
norm_weights = sweep(results$weights, 1, rowSums(results$weights), '/')
cell_type_names <- myRCTD@cell_type_info$info[[2]]
spatialRNA <- myRCTD@spatialRNA
resultsdir <- 'C:/Users/sun/Desktop/New Folder2'
dir.create(resultsdir)
plot_weights(cell_type_names, spatialRNA, resultsdir, norm_weights)
plot_weights_unthreshold(cell_type_names, spatialRNA, resultsdir, norm_weights)
plot_weights_doublet(cell_type_names, spatialRNA, resultsdir, results$weights_doublet, results$results_df)
plot_cond_occur(cell_type_names, resultsdir, norm_weights, spatialRNA)
plot_all_cell_types(results$results_df, spatialRNA@coords, cell_type_names, resultsdir)
doublets <- results$results_df[results$results_df$spot_class == "doublet_certain", ]
plot_doublets(spatialRNA, doublets, resultsdir, cell_type_names)
plot_doublets_type(spatialRNA, doublets, resultsdir, cell_type_names)
doub_occur <- table(doublets$second_type, doublets$first_type)
plot_doub_occur_stack(doub_occur, resultsdir, cell_type_names)
```

FIG. 18A

```
import scanpy as sc

import pandas as pd

import numpy as np

import seaborn as sns

import matplotlib.pyplot as plt

import da_cellfraction

from utils import random_mix

from sklearn.manifold import TSNE


sc.logging.print_versions()

sc.set_figure_params(facecolor="white", figsize=(8, 8))

sc.settings.verbosity = 3


adata_spatial_anterior = sc.datasets.visium_sge(

    sample_id="V1_Mouse_Brain_Sagittal_Anterior"

)

adata_spatial_posterior = sc.datasets.visium_sge(

    sample_id="V1_Mouse_Brain_Sagittal_Posterior"

)


#Normalize

for adata in [

    adata_spatial_anterior,

    adata_spatial_posterior,

]:

    sc.pp.normalize_total(adata, inplace=True)


adata_cortex = sc.read_csv('C:/Users/sun/data/GSE115746_cells_exon_counts.csv').T
```

FIG. 18B

```
adata_cortex_meta      =     pd.read_csv('C:/Users/sun/data/GSE115746_complete_metadata_28706-cells.csv',
index_col=0)


adata_cortex_meta_ = adata_cortex_meta.loc[adata_cortex.obs.index,]


adata_cortex.obs = adata_cortex_meta_

adata_cortex.var_names_make_unique()


#Preprocessing

adata_cortex.var['mt'] = adata_cortex.var_names.str.startswith('Mt-')    # annotate the group of mitochondrial
genes as 'mt'

sc.pp.calculate_qc_metrics(adata_cortex, qc_vars=['mt'], percent_top=None, log1p=False, inplace=True)


sc.pp.normalize_total(adata_cortex)


#PCA and clustering : Known markers with 'cell_subclass'

sc.tl.pca(adata_cortex, svd_solver='arpack')

sc.pp.neighbors(adata_cortex, n_neighbors=10, n_pcs=40)

sc.tl.umap(adata_cortex)

sc.tl.leiden(adata_cortex, resolution = 0.5)

sc.pl.umap(adata_cortex, color=['leiden','cell_subclass'])


sc.tl.rank_genes_groups(adata_cortex, 'cell_subclass', method='wilcoxon')

sc.pl.rank_genes_groups(adata_cortex, n_genes=20, sharey=False)


genelists=adata_cortex.uns['rank_genes_groups']['names']

df_genelists = pd.DataFrame.from_records(genelists)

df_genelists.head(5)


num_markers=30
```

## FIG. 18C

```
res_genes = []

for column in df_genelists.head(num_markers):

        res_genes.extend(df_genelists.head(num_markers)[column].tolist())

res_genes_ = list(set(res_genes))


adata_spatial_anterior.var_names_make_unique()

inter_genes = [val for val in res_genes_ if val in adata_spatial_anterior.var.index]

print('Selected Feature Gene number',len(inter_genes))

adata_cortex = adata_cortex[:,inter_genes]


adata_spatial_anterior = adata_spatial_anterior[:,inter_genes]


adata_spatial_anterior.var


mat_sc = adata_cortex.X

mat_sp = adata_spatial_anterior.X.todense()


df_sc = adata_cortex.obs


lab_sc_sub = df_sc.cell_subclass

sc_sub_dict = dict(zip(range(len(set(lab_sc_sub))), set(lab_sc_sub)))

sc_sub_dict2 = dict((y,x) for x,y in sc_sub_dict.items())

lab_sc_num = [sc_sub_dict2[ii] for ii in lab_sc_sub]

lab_sc_num = np.asarray(lab_sc_num, dtype='int')


sc_mix, lab_mix = random_mix(mat_sc, lab_sc_num, nmix=5, n_samples=5000)


def log_minmaxscale(arr):
```

EP 4 362 027 A1

FIG. 18D

```
arrd = len(arr)

arr = np.log1p(arr)

    return        (arr-np.reshape(np.min(arr,axis=1),        (arrd,1)))/np.reshape((np.max(arr,        axis=1)-
np.min(arr,axis=1)),(arrd,1))


sc_mix_s = log_minmaxscale(sc_mix)

mat_sp_s = log_minmaxscale(mat_sp)

mat_sc_s = log_minmaxscale(mat_sc)


print(mat_sp_s.shape, mat_sc_s.shape, sc_mix_s.shape)


embs, clssmodel = da_cellfraction.train(sc_mix_s, lab_mix, mat_sp_s,

                                alpha=1, alpha_lr=5, emb_dim = 64, batch_size = 512,

                                n_iterations = 2000,

                                  initial_train=True,

                                  initial_train_epochs=10)


pred_sp = clssmodel.predict(mat_sp_s)


def plot_cellfraction(visnum):

    adata_spatial_anterior.obs['Pred_label'] = pred_sp[:,visnum]

    sc.pl.spatial(

        adata_spatial_anterior,

        img_key="hires",

        color='Pred_label',

        palette='Set1',

        size=1.5,

        legend_loc=None,
```

57

FIG. 18E

```
        title = sc_sub_dict[visnum])


print(sc_sub_dict)


numlist = [0,1,2,3,4,5,6,7,8]


for num in numlist:
    plot_cellfraction(num)


pred_mix = clssmodel.predict(sc_mix_s)


for visnum in numlist:
    plt.figure(figsize=(4,4))
    sns.scatterplot(pred_mix[:,visnum], lab_mix[:,visnum]).set_title(sc_sub_dict[visnum])
    plt.show()


print('\n'.join(f'{m.__name__} {m.__version__}' for m in globals().values() if getattr(m, '__version__', None)))


import keras
keras.__version__
```

FIG. 19A

FIG. 19B

FIG. 19C

FIG. 20A

FIG. 20B

FIG. 20C

FIG. 20D

FIG. 20E

Mean Fluorescence Intensity

Distance from Surface

FIG. 20F

0.0 0.5 1.0 1.5 2.0

0.0 0.4 0.8 1.2 1.6

Pecam1

Cd34

FIG. 21C

FIG. 22A

FIG.22B

FIG.22C

FIG.22D

FIG.22E

FIG.23A

FIG.23B

FIG.23C

FIG.24A

FIG.24B

| | 1 (92) | 2 (92) |
|---|---|---|
| Epithelial and Cancer cell (1204) | | |
| Endothelial cell (401) | | |
| Fibroblast (811) | | |
| Monocyte derived cell (300) | | |
| Inflammatory macrophage (519) | | |
| Anti Inflammatory macrophage (464) | | |
| Proliferatory myeloid (383) | | |
| T cell and NK cell (117) | | |
| Neutrophil (137) | | |

FIG.24C

Cluster 1

Cluster 2

FIG.24D

FIG.24E

NS　Log₂ FC　p-value　p-value and log₂ FC

Cluster 1

Total = 67 variables

NS　Log₂ FC　p-value　p-value and log₂ FC

Cluster 2

Total = 111 variables

FIG.24F

FIG.24G

FIG.24H

FIG.24I

FIG.24J

FIG.24K

Total(31)  Hypoxia(9)  Glucose metabolism (5)  Apoptosis death(9)

FIG.25

K = 3  K = 4  K = 5

FIG.26A

FIG.26B

FIG.26C

| Cell Type | Rel. Uptake |
| --- | --- |
|  | 5.2405 |
|  | 1.9971 |
|  | 1.0311 |
|  | 6.8442 |
|  | 20.102 |
|  | 2.3486 |
|  | 15.062 |
|  | 10.620 |
|  | 3.5505 |

Anti-inflammatory macrophage
Endothelial
Neutrophil
Fibroblast
Epithelial/Cancer cell
T cell/NK cell
Monocyte-derived cell
Inflammatory macrophage
Proliferatory myeloid

FIG.26D

| Cell Type | Rel. Uptake |
|---|---|
| | 1.8932 |
| | 1.0453 |
| | 0.0910 |
| | 2.5067 |
| | 2.9445 |
| | 0.6107 |
| | 3.9463 |
| | 1.7167 |
| | 0.8219 |

- Anti-inflammatory macrophage
- Endothelial
- Neutrophil
- Fibroblast
- Epithelial/Cancer cell
- T cell/NK cell
- Monocyte-derived cell
- Inflammatory macrophage
- Proliferatory myeloid

FIG.26E

| Cell Type | Rel. Uptake |
|---|---|
|  | 0.9367 |
|  | 0.1651 |
|  | 0.2739 |
|  | 0.9314 |
|  | 5.6360 |
|  | 0.4541 |
|  | 3.0522 |
|  | 2.9837 |
|  | 0.5986 |

- Anti-inflammatory macrophage
- Endothelial
- Neutrophil
- Fibroblast
- Epithelial/Cancer cell
- T cell/NK cell
- Monocyte-derived cell
- Inflammatory macrophage
- Proliferatory myeloid

FIG.27

FIG.28A

FIG.28B

FIG.29

FIG.30A

FIG.30B

FIG.31A

FIG.31B

| | 0 (3) | 1 (314) | 2 (255) | 3 (638) | 4 (275) | 5 (1332) | 6 (88) | 7 (50) |
|---|---|---|---|---|---|---|---|---|
| Cancer/Epithelial (1214) | | | | | | | | |
| Endothelial (587) | | | | | | | | |
| Fibroblasts (812) | | | | | | | | |
| Monocyte (300) | | | | | | | | |
| Inflammatory Macrophages (578) | | | | | | | | |
| Anti-inflammatory Macrophages (505) | | | | | | | | |
| Proliferatory Myeloid (383) | | | | | | | | |
| T cell/NK cell (140) | | | | | | | | |
| Neutrophil (172) | | | | | | | | |

Depletion   P Value   Enrichment
(−log₁₀)
150 120 90 60 30 0    0 30 60 90 120 150

FIG.32

## INTERNATIONAL SEARCH REPORT

| International application No. |
| --- |
| **PCT/KR2022/007277** |

**A.    CLASSIFICATION OF SUBJECT MATTER**

**G16B 15/30**(2019.01)i; **G16B 40/00**(2019.01)i; **G16B 25/00**(2019.01)i; **G16B 45/00**(2019.01)i; **G16C 20/30**(2019.01)i; **G16C 20/10**(2019.01)i; **G16B 35/10**(2019.01)i

According to International Patent Classification (IPC) or to both national classification and IPC

**B.    FIELDS SEARCHED**

Minimum documentation searched (classification system followed by classification symbols)

G16B 15/30(2019.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 공간 전사체 기술(spatially transcriptomic technique), SPADE(spatial gene expression patterns by deep learning of tissue images), 클러스터(cluster), 맵핑(mapping), 조직(tissue), 생리활성 정보 (bioactive information)

**C.    DOCUMENTS CONSIDERED TO BE RELEVANT**

| Category* | Citation of document, with indication, where appropriate, of the relevant passages | Relevant to claim No. |
| --- | --- | --- |
| Y | BAE, Sungwoo et al. Discovery of molecular features underlying the morphological landscape by integrating spatial transcriptomic data with deep features of tissue images. Nucleic Acids Research. 22 February 2021 (online publication date), vol. 49, no. 10, article no. e55, inner pp. 1-12.<br>    See inner pages 2-4 and 6; and figures 1-3. | 1-41 |
| Y | VU, Tam et al. Spatial transcriptomics using combinatorial fluorescence spectral and lifetime encoding, imaging and analysis. BioRxiv. 23 June 2021 (publication date). Retrieved from Doi: https://doi.org/10.11 01/2021.06.22.449468. inner pp. 1-28.<br>    See abstract; and figure 1. | 1-41 |
| Y | HE, Bryan et al. Integrating spatial gene expression and breast tumour morphology via deep learning. Nature Biomedical Engineering. 22 June 2020 (online publication date), vol. 4, pp. 827-834.<br>    See abstract; and page 827. | 7,8 |
| A | FERREIRA, Ricardo Melo et al. Integration of spatial and single-cell transcriptomics localizes epithelial cell-immune cross-talk in kidney injury. JCI Insight. 22 June 2021 (publication date), vol. 6, no. 12, article no. e147703, inner pp. 1-21.<br>    See inner pages 1-19. | 1-41 |

☑ Further documents are listed in the continuation of Box C.      ☐ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
| --- | --- |
| **26 August 2022** | **26 August 2022** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

International application No.

**PCT/KR2022/007277**

**C. DOCUMENTS CONSIDERED TO BE RELEVANT**

| Category* | Citation of document, with indication, where appropriate, of the relevant passages | Relevant to claim No. |
|---|---|---|
| A | BERGENSTRAHLE, Joseph et al. Seamless integration of image and molecular analysis for spatial transcriptomics workflows. BMC Genomics. 14 July 2020 (online publication date), vol. 21, article no. 482, inner pp. 1-7.<br>  See inner pages 1-6. | 1-41 |
| PX | PARK, Jeong-Bin et al. Spatial transcriptomics-based identification of molecular markers for nanomedicine distribution in tumor tissue. BioRxiv. 04 March 2022 (publication date). Retrieved from Doi : https://doi.org/10.1101/2022.03.02.482584. inner pp. 1-35.<br>  See inner pages 3-30. | 1-41 |

Form PCT/ISA/210 (second sheet) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *Nat Rev Drug Discov.,* December 2016, vol. 15 (12), 817-818
- **SINDHWANI, S ; SYED, A. M. ; NGAI, J. ; KINGSTON, B. R. ; MAIORINO, L. ; ROTHSCHILD, J. ; CHAN, W. C.** The entry of nanoparticles into solid tumours. *Nature materials,* 2020, vol. 19 (5), 566-575
- **CHEN, F. ; MA, K. ; MADAJEWSKI, B. ; ZHUANG, L. ; ZHANG, L. ; RICKERT, K. ; BRADBURY, M. S.** Ultrasmall targeted nanoparticles with engineered antibody fragments for imaging detection of HER2-overexpressing breast cancer. *Nature communications,* 2018, vol. 9 (1), 1-11
- **BOLKESTEIN, M. ; DE BLOIS, E. ; KOELEWIJN, S. J. ; EGGERMONT, A. M. ; GROSVELD, F. ; DE JONG, M. ; KONING, G. A.** Investigation of factors determining the enhanced permeability and retention effect in subcutaneous xenografts. *Journal of nuclear medicine,* 2016, vol. 57 (4), 601-607
- **FICK, A.** V. On liquid diffusion. *Phil. Mag.,* vol. 10 (63), 30-39
- **R.B. BIRD ; W.E. STEWART ; E.N. LIGHTFOOT.** Transport Phenomena. Wiley, 2002
- **SINDHWANI, S. ; SYED, A. M. ; NGAI, J. ; KINGSTON, B. R. ; MAIORINO, L. ; ROTHSCHILD, J. ; CHAN, W. C.** The entry of nanoparticles into solid tumours. *Nature materials,* 2020, vol. 19 (5), 566-575
- **BAE, S. ; CHOI, H. ; LEE, D. S.** Discovery of molecular features underlying the morphological landscape by integrating spatial transcriptomic data with deep features of tissue images. *Nucleic acids research,* 2021, vol. 49 (10), e55-e55
- **SEBASTIAN, A. ; HUM, N. R. ; MARTIN, K. A. ; GILMORE, S. F. ; PERAN, I ; BYERS, S. W. ; LOOTS, G. G.** Single-cell Transcriptomic analysis of tumor-derived fibroblasts and Normal tissue-resident fibroblasts reveals fibroblast heterogeneity in breast Cancer. *Cancers,* 2020, vol. 12 (5), 1307
- **CABLE, D. M. ; MURRAY, E. ; ZOU, L. S. ; GOEVA, A. ; MACOSKO, E. Z. ; CHEN, F. ; IRIZARRY, R. A.** Robust decomposition of cell type mixtures in spatial transcriptomics. *Nature Biotechnology,* 2021, 1-10
- **ZHOU, Y. ; LUO, G.** Apolipoproteins, as the carrier proteins for lipids, are involved in the development of breast cancer. *Clinical and Translational Oncology,* 2020, 1-11

- **ALFAROUK, K. O.** Tumor metabolism, cancer cell transporters, and microenvironmental resistance. *Journal of enzyme inhibition and medicinal chemistry,* 2016, vol. 31 (6), 859-866
- **WANG, Z. ; BOVIK, A. C. ; SHEIKH, H. R. ; SIMONCELLI, E. P.** Image quality assessment: from error visibility to structural similarity. *IEEE transactions on image processing,* 2004, vol. 13 (4), 600-612
- **BROWNLEE, W. J. ; SEIB, F. P.** Impact of the hypoxic phenotype on the uptake and efflux of nanoparticles by human breast cancer cells. *Scientific reports,* 2018, vol. 8 (1), 1-11
- **ZHANG, I ; CUI, Y. ; AMIRI, A. ; DING, Y. ; CAMPBELL, R. E. ; MAYSINGER, D.** Pharmacological inhibition of lipid droplet formation enhances the effectiveness of curcumin in glioblastoma. *European Journal of Pharmaceutics and Biopharmaceutics,* 2016, vol. 100, 66-76
- **FUJIMOTO, T. ; PARTON, R. G.** Not just fat: the structure and function of the lipid droplet. *Cold Spring Harbor perspectives in biology,* 2011, vol. 3 (3), a004838
- **CRUZ, A. L. ; BARRETO, E. D. A. ; FAZOLINI, N. P. ; VIOLA, J. P ; BOZZA, P. T.** Lipid droplets: platforms with multiple functions in cancer hallmarks. *Cell death & disease,* 2020, vol. 11 (2), 1-16
- **LI, R. ; NG, T. S. ; WANG, S. J. ; PRYTYSKACH, M ; RODELL, C. B. ; MIKULA, H. ; MILLER, M. A.** Therapeutically reprogrammed nutrient signalling enhances nanoparticulate albumin bound drug uptake and efficacy in KRAS-mutant cancer. *Nature Nanotechnology,* 2021, 1-10
- **YOKOI K ; KOJIC M ; MILOSEVIC M ; TANEI T ; FERRARI M ; ZIEMYS A.** Capillary-Wall Collagen as a Biophysical Marker of Nanotherapeutic Permeability. *Tumor Microenvironment,* 2014, vol. 74 (16), 4239-4246
- **MONCADA R ; BARKLEY D ; WAGNER F ; CHIODIN M ; DEVLIN JC ; BARON M et al.** Integrating microarray-based spatial transcriptomics and single-cell RNA-seq reveals tissue architecture in pancreatic ductal adenocarcinomas. *Nat Biotechnol,* 2020, vol. 38 (3), 333-342
- **BAE S ; NA KJ ; KOH J ; LEE DS ; CHOI H ; KIM YT.** CellDART: Cell type inference by domain adaptation of single-cell and spatial transcriptomic data. *bioRxiv 2021: 2021.2004.2026.441459*

- **CASTELLANO J ; ALEDO R ; SENDRA J ; COS-TALES P ; JUAN-BABOT O ; BADIMON L et al.** Hypoxia stimulates low-density lipoprotein receptor-related protein-1 expression through hypoxia-inducible factor-1α in human vascular smooth muscle cells. *Arteriosclerosis, thrombosis, and vascular biology,* 2011, vol. 31 (6), 1411-1420

- **PERMAN JC ; BOSTROM P ; LINDBOM M ; LID-BERG U ; STAHLMAN M ; HAGG D et al.** The VLDL receptor promotes lipotoxicity and increases mortality in mice following an acute myocardial infarction. *The Journal of clinical investigation,* 2011, vol. 121 (7), 2625-2640

- *Cancer Biotherapy and Radiopharmaceuticals,* vol. 32 (3), 83-89